# EUROPEAN PATENT APPLICATION

(11) **EP 4 663 198 A1**
(43) Date of publication of application: **17.12.2025**
(21) Application number: 24792718.9
(22) Date of filing: 18.04.2024
(51) Int. Cl.: A61K 38/12, A61K 47/12, A61K 47/18, A61K 47/20, A61P 43/00, C07K 7/54

(54) **COMPOSITION CONTAINING CYCLIC PEPTIDE COMPOUND AND SURFACTANT**

(30) Priority: 19.04.2023 JP 2023068232
(71) Applicant: CHUGAI SEIYAKU KABUSHIKI KAISHA, Tokyo 115-8543 (JP)
(72) Inventor: NAKAE Shinichi, Yokohama City, Kanagawa 244-8602 (JP); KURAMOTO Shino, Yokohama City, Kanagawa 244-8602 (JP); KIMURA Ryosuke, Yokohama City, Kanagawa 244-8602 (JP); SAKAGUCHI Mana, Yokohama City, Kanagawa 244-8602 (JP)
(74) Representative: Vossius & Partner Patentanwälte Rechtsanwälte mbB
(86) International application number: PCT/JP2024/015377
(87) International publication number: WO 2024/219446

(57) **Abstract**

The present invention relates to a composition comprising a compound represented by general formula (1), a pharmaceutically acceptable salt thereof, or a pharmaceutically acceptable solvate thereof, and a surfactant. 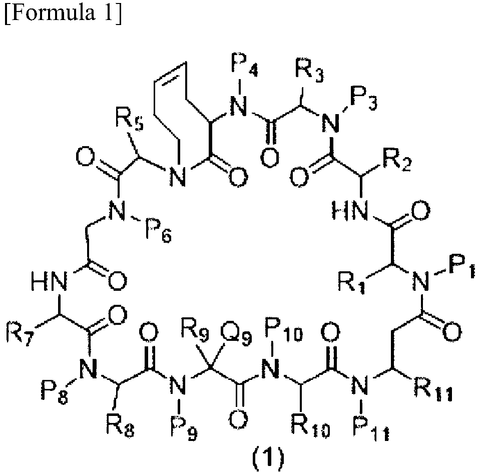 [wherein R₁, P₁, R₂, P₃, P₄, R₅, P₆, R₁₀, P₁₀ and P₁₁ are C₁ to C₆ alkyl or the like; R₃ and P₉ are hydrogen or the like; R₇ is phenethyl optionally substituted by halogen or the like; R₈ forms a 4-to 7-membered saturated heterocyclic ring together with a carbon atom bonded to P₈ and R₈ and a nitrogen atom bonded to P₈; R₉ forms a 3- to 8-membered alicyclic ring together with Q₉ and a carbon atom bonded to R₉ and Q₉; and R₁₁ is di-C₁ to C₆ alkylaminocarbonyl or the like].

## Description

### Technical Field

The present invention relates to a composition containing a cyclic peptide compound and a surfactant, particularly a cyclic peptide compound having selective KRAS inhibitory action on HRAS and NRAS and a surfactant.

### Background Art

RAS is a protein belonging to the small GTPase family, of which KRAS, NRAS, and HRAS are known. RAS is defined as activated or inactivated depending on its binding state to GDP or GTP, and it is activated by the exchange reaction of GDP to GTP by GEF (guanine nucleotide exchange factor) and inactivated by the hydrolysis reaction of GTP by GAP (GTPase-activating proteins) (Non Patent Literature 1). Activated RAS induces cell proliferation, survival, and differentiation by activating various downstream signals such as the MAPK pathway, PI3K/Akt pathway, and RAL pathway, and constitutive activation of RAS plays an important role in the development and progression of cancer. In cancer, the RAS-RAF-MEK-ERK pathway is known to be activated due to the activation of RAS upstream signaling, constitutive activation of RAS, and/or activating mutations of RAS (Non Patent Literature 2). These activating mutations of RAS have been found in numerous types of cancer. G12, G13, and Q61 are known as RAS mutation hotspots, and mutations are frequently found at G12 in KRAS and at Q61 in NRAS. These mutations are also known to be associated with patient prognosis (Non Patent Literature 3).

Meanwhile, in access to tough targets, such as the inhibition of protein-protein interactions, medium molecular compounds (having molecular weights of 500 to 2000 g/mol) may be superior to small molecular compounds. Medium molecular compounds may also be superior to antibodies in that they can migrate into the cells. Among the biologically active medium molecular compounds, peptide pharmaceuticals are high valuable molecular species and 40 or more kinds of them are already on the market (Non Patent Literature 4). Representative examples of these peptide pharmaceuticals include cyclosporine A and polymyxin B. These are peptides containing several non-natural amino acids. Non-natural amino acids are amino acids that are not naturally encoded on mRNA, and it is very interesting that non-natural amino acids are included in the naturally-derived cyclosporine A and polymyxin B.

Ever since naturally-derived peptides were found to be useful as medicaments, peptides that have pharmacological activity and can be absorbed by living organisms have gained attention, and those with a molecular weight of about 500 to 2000 g/mol have been actively studied (Non Patent Literature 5).

It is generally believed that compounds having a molecular weight of 500 g/mol or more have low membrane permeability and problems in BA. The same holds for the peptide compound, which is an example of the medium molecular compounds. As strategies to improve the membrane permeability of peptide compounds, a peptide compound containing an N-substituted amino acid residue (e.g., an N-methylamino acid residue) as a component, a cyclic peptide compound containing a cyclic structure, or the like, have been created (e.g., Patent Literature 1). In addition, a combination of a peptide compound with a surfactant has been attempted to improve BA. For example, Patent Literature 2 discloses a finished medicament adapted for oral delivery, containing a physiologically active peptide agent, at least one pharmaceutically acceptable pH-lowering agent, and at least one absorption-promoting agent effective to enhance the bioavailability of the active agent.

In this context, cyclic peptide compounds having selectivity in RAS, specifically a selective KRAS inhibitory action on HRAS and NRAS, have been reported (Patent Literature 3).

### Citation List

### Patent Literature

Patent Literature 1: International Publication No. WO2013/100132
Patent Literature 2: Japanese Translation of PCT International Application Publication No. JP2009-518437
Patent Literature 3: International Publication No. WO2022/234853

### Non Patent Literature

Non Patent Literature 1: Nat. Rev. Drug Discov., 2014, Vol. 13, No. 11, pp. 828-851
Non Patent Literature 2: Nat. Rev. Drug Discov., 2014, Vol. 13, No. 12, pp. 928-942
Non Patent Literature 3: Nat. Rev. Drug Discov., 2016, Vol. 15, No. 11, pp. 771-785
Non Patent Literature 4: Future Med. Chem., 2009, Vol. 1, pp. 1289-1310
Non Patent Literature 5: Current Topics in Medicinal Chemistry, 2013, Vol. 13, No. 7, pp. 821-836

### Summary of Invention

### Technical Problem

As far as the present inventor knows, there are no reports of cyclic peptide compounds having selective KRAS inhibitory action on HRAS and NRAS being combined with a surfactant to enhance the BA of the cyclic peptide compounds.

It is an object of the present invention to provide a composition containing a cyclic peptide compound and a surfactant, having enhanced membrane permeability and/or BA of the cyclic peptide compound. Another object is to provide a composition containing a cyclic peptide compound for use in combination with a surfactant. Yet another object is to provide a method for using a surfactant to improve the membrane permeability and/or BA of the cyclic peptide compound.

### Solution to Problem

The present invention relates to, for example, each of the following inventions.
[A1] A composition comprising a compound represented by general formula (1), a pharmaceutically acceptable salt thereof, or a pharmaceutically acceptable solvate thereof, and a surfactant: 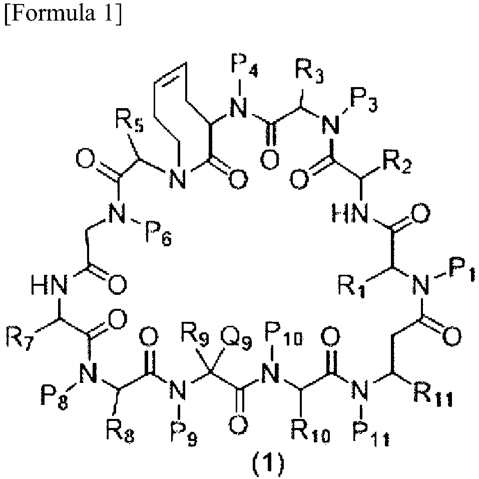 [wherein R₁ is C₁ to C₆ alkyl or C₃ to C₈ cycloalkyl C₁ to C₆ alkyl;
   P₁ is C₁ to C₆ alkyl;
   R₂ is C₁ to C₆ alkyl;
   R₃ is hydrogen, or forms a 4- to 7-membered saturated heterocyclic ring together with a carbon atom bonded to P₃ and R₃ and a nitrogen atom bonded to P₃;
   P₃ is C₁ to C₆ alkyl or C₃ to C₈ cycloalkyl except when R₃ and P₃ form a 4- to 7-membered saturated heterocyclic ring;
   P₄ is C₁ to C₆ alkyl;
   R₅ is benzyl optionally substituted by one or more groups selected from the group consisting of C₁ to C₆ alkyls, C₁ to C₆ haloalkyls, and C₃ to C₈ cycloalkyls;
   P₆ is C₁ to C₆ alkyl;
   R₇ is phenethyl optionally substituted by one or more groups selected from the group consisting of halogens, C₁ to C₆ haloalkyls, and C₁ to C₆ alkoxys;
   R₈ forms a 4- to 7-membered saturated heterocyclic ring together with a carbon atom bonded to P₈ and R₈ and a nitrogen atom bonded to P₈, the 4- to 7-membered saturated heterocyclic ring being optionally substituted by a C₁ to C₆ alkoxy;
   R₉ forms a 3- to 8-membered alicyclic ring together with Q₉ and a carbon atom bonded to R₉ and Q₉, the 3- to 8-membered alicyclic ring being optionally substituted by one or more C₁ to C₆ alkyls;
   P₉ is hydrogen or C₁ to C₆ alkyl;
   R₁₀ is C₁ to C₆ alkyl or C₃ to C₈ cycloalkyl;
   P₁₀ is C₁ to C₆ alkyl;
   R₁₁ is di-C₁ to C₆ alkylaminocarbonyl or 4- to 8-membered cyclic aminocarbonyl; and
   P₁₁ is C₁ to C₆ alkyl].
[A2] A composition comprising a compound represented by general formula (1), a pharmaceutically acceptable salt thereof, or a pharmaceutically acceptable solvate thereof, to be used in combination with a surfactant: 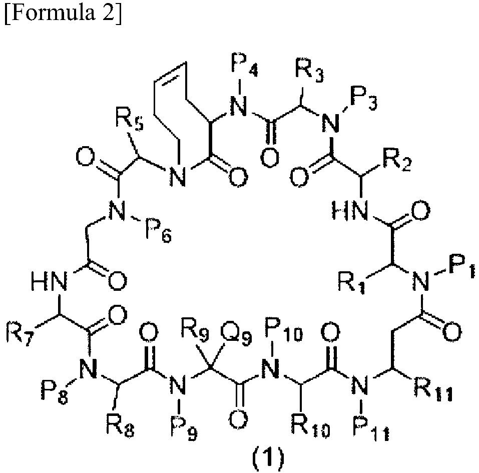 [wherein R₁ is C₁ to C₆ alkyl or C₃ to C₈ cycloalkyl C₁ to C₆ alkyl;
   P₁ is C₁ to C₆ alkyl;
   R₂ is C₁ to C₆ alkyl;
   R₃ is hydrogen, or forms a 4- to 7-membered saturated heterocyclic ring together with a carbon atom bonded to P₃ and R₃ and a nitrogen atom bonded to P₃;
   P₃ is C₁ to C₆ alkyl or C₃ to C₈ cycloalkyl except when R₃ and P₃ form a 4- to 7-membered saturated heterocyclic ring;
   P₄ is C₁ to C₆ alkyl;
   R₅ is benzyl optionally substituted by one or more groups selected from the group consisting of C₁ to C₆ alkyls, C₁ to C₆ haloalkyls, and C₃ to C₈ cycloalkyls;
   P₆ is C₁ to C₆ alkyl;
   R₇ is phenethyl optionally substituted by one or more groups selected from the group consisting of halogens, C₁ to C₆ haloalkyls, and C₁ to C₆ alkoxys;
   R₈ forms a 4- to 7-membered saturated heterocyclic ring together with a carbon atom bonded to P₈ and R₈ and a nitrogen atom bonded to P₈, the 4- to 7-membered saturated heterocyclic ring being optionally substituted by a C₁ to C₆ alkoxy;
   R₉ forms a 3- to 8-membered alicyclic ring together with Q₉ and a carbon atom bonded to R₉ and Q₉, the 3- to 8-membered alicyclic ring being optionally substituted by one or more C₁ to C₆ alkyls;
   P₉ is hydrogen or C₁ to C₆ alkyl;
   R₁₀ is C₁ to C₆ alkyl or C₃ to C₈ cycloalkyl;
   P₁₀ is C₁ to C₆ alkyl;
   R₁₁ is di-C₁ to C₆ alkylaminocarbonyl or 4- to 8-membered cyclic aminocarbonyl; and
   P₁₁ is C₁ to C₆ alkyl].
[A3] The composition according to [A1] or [A2], wherein the surfactant is represented by any one of the following general formulae (a1) to (a3): 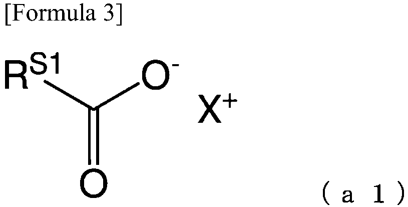 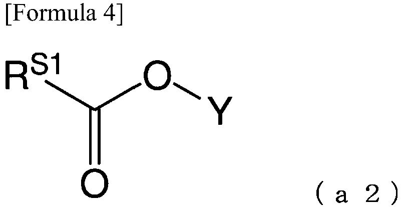 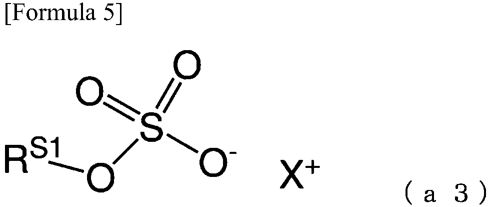 [wherein R^{S1} represents a saturated or unsaturated, linear hydrocarbon group having 5 or more and 13 or less carbon atoms which may have a substituent, X represents sodium or potassium, and Y represents a group represented by the following formula (a4) or a stereoisomer thereof.] 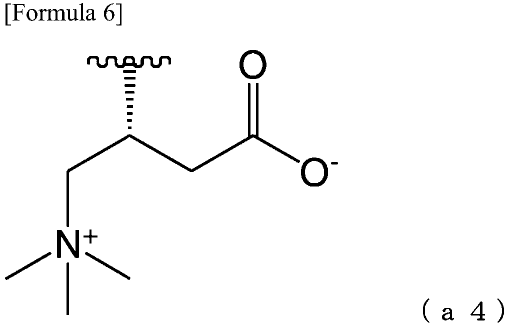 [wherein 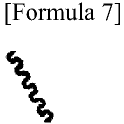 represents a bond.]
[A4] The composition according to [A3], wherein the R^{S1} is a linear alkyl group having 7 or more and 13 or less carbon atoms.
[A5] The composition according to [A3], wherein the R^{S1} is an unsubstituted linear alkyl group.
[A6] The composition according to [A3], wherein the R^{S1} is a linear alkyl group having 8 or more and 12 or less carbon atoms.
[A7] The composition according to [A3], wherein the R^{S1} is a linear alkyl group having 10 or more and 12 or less carbon atoms.
[A8] The composition according to any one of [A1] to [A7], wherein the surfactant is a medium-chain fatty acid ester, a sodium salt of medium-chain fatty acid, or a potassium salt of medium-chain fatty acid.
[A9] The composition according to any one of [A1] to [A8], wherein the surfactant is acylcarnitine.
[A10] The composition according to [A9], wherein the acylcamitine is lauroyl-L-carnitine.
[A11] The composition according to any one of [A1] to [A8], wherein the surfactant is an alkyl carboxylate.
[A12] The composition according to [A11], wherein the surfactant is a caprylate.
[A13] The composition according to [A12], wherein the surfactant is sodium caprylate.
[A14] The composition according to any one of [A1] to [A8], wherein the surfactant is an alkyl sulfate.
[A15] The composition according to [A14], wherein the surfactant is a lauryl sulfate.
[A16] The composition according to [A15], wherein the surfactant is sodium lauryl sulfate.
[A17] The composition according to [A3], wherein the substituent is an N-(2-hydroxybenzoyl)amino group.
[A18] The composition according to [A17], wherein the surfactant is an N-(8-[2-hydroxybenzoyl]amino)caprylate.
[A19] The composition according to [A18], wherein the surfactant is sodium N-(8-[2-hydroxybenzoyl]amino)caprylate.
[A20] A composition comprising a compound represented by general formula (1), a pharmaceutically acceptable salt thereof, or a pharmaceutically acceptable solvate thereof, and a surfactant selected from the group consisting of lauroyl-L-carnitine, sodium caprylate, sodium lauryl sulfate, and sodium N-(8-[2-hydroxybenzoyl]amino)caprylate: 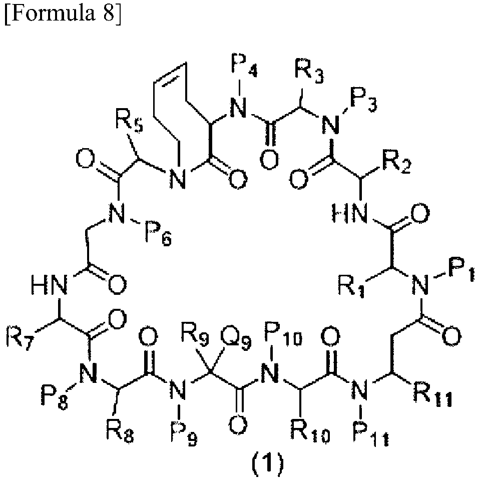 [wherein R₁ is C₁ to C₆ alkyl or C₃ to C₈ cycloalkyl C₁ to C₆ alkyl;
   P₁ is C₁ to C₆ alkyl;
   R₂ is C₁ to C₆ alkyl;
   R₃ is hydrogen, or forms a 4- to 7-membered saturated heterocyclic ring together with a carbon atom bonded to P₃ and R₃ and a nitrogen atom bonded to P₃;
   P₃ is C₁ to C₆ alkyl or C₃ to C₈ cycloalkyl except when R₃ and P₃ form a 4- to 7-membered saturated heterocyclic ring;
   P₄ is C₁ to C₆ alkyl;
   R₅ is benzyl optionally substituted by one or more groups selected from the group consisting of C₁ to C₆ alkyls, C₁ to C₆ haloalkyls, and C₃ to C₈ cycloalkyls;
   P₆ is C₁ to C₆ alkyl;
   R₇ is phenethyl optionally substituted by one or more groups selected from the group consisting of halogens, C₁ to C₆ haloalkyls, and C₁ to C₆ alkoxys;
   R₈ forms a 4- to 7-membered saturated heterocyclic ring together with a carbon atom bonded to P₈ and R₈ and a nitrogen atom bonded to P₈, the 4- to 7-membered saturated heterocyclic ring being optionally substituted by a C₁ to C₆ alkoxy;
   R₉ forms a 3- to 8-membered alicyclic ring together with Q₉ and a carbon atom bonded to R₉ and Q₉, the 3- to 8-membered alicyclic ring being optionally substituted by one or more C₁ to C₆ alkyls;
   P₉ is hydrogen or C₁ to C₆ alkyl;
   R₁₀ is C₁ to C₆ alkyl or C₃ to C₈ cycloalkyl;
   P₁₀ is C₁ to C₆ alkyl;
   R₁₁ is di-C₁ to C₆ alkylaminocarbonyl or 4- to 8-membered cyclic aminocarbonyl; and
   P₁₁ is C₁ to C₆ alkyl].
[A21] The composition according to any one of [A1] to [A20], wherein R₁ is n-propyl.
[A22] The composition according to any one of [A1] to [A20], wherein R₁ is 2-methylpropyl.
[A23] The composition according to any one of [A1] to [A20], wherein R₁ is cyclopentylmethyl.
[A24] The composition according to any one of [A1] to [A23], wherein P₁ is methyl.
[A25] The composition according to any one of [A1] to [A24], wherein R₂ is 1-methylpropyl.
[A26] The composition according to any one of [A1] to [A25], wherein R₃ is hydrogen.
[A27] The composition according to any one of [A1] to [A25], wherein R₃ forms a 5-membered saturated heterocyclic ring together with a carbon atom bonded to P₃ and R₃ and a nitrogen atom bonded to P₃.
[A28] The composition according to any one of [A1] to [A27], wherein P₃ is methyl.
[A29] The composition according to any one of [A1] to [A27], wherein P₃ forms a 5-membered saturated heterocyclic ring together with a carbon atom bonded to R₃ and R₃ and a nitrogen atom bonded to P₃.
[A30] The composition according to any one of [A1] to [A27], wherein P₃ is cyclopropyl.
[A31] The composition according to any one of [A1] to [A30], wherein P₄ is methyl.
[A32] The composition according to any one of [A1] to [A31], wherein R₅ is 4-trifluoromethylbenzyl.
[A33] The composition according to any one of [A1] to [A31], wherein R₅ is 4-methylbenzyl.
[A34] The composition according to any one of [A1] to [A33], wherein P₆ is methyl.
[A35] The composition according to any one of [A1] to [A34], wherein R₇ is 3-methoxy-4-trifluoromethylphenethyl.
[A36] The composition according to any one of [A1] to [A34], wherein R₇ is 3,5-difluoro-4-trifluoromethylphenethyl.
[A37] The composition according to any one of [A1] to [A36], wherein R₈ forms a 5-membered saturated heterocyclic ring together with a carbon atom bonded to P₈ and R₈ and a nitrogen atom bonded to P₈, the 5-membered saturated heterocyclic ring being substituted by an ethoxy.
[A38] The composition according to any one of [A1] to [A37], wherein R₉ forms a 4- to 5-membered alicyclic ring together with Q₉ and a carbon atom bonded to R₉ and Q₉.
[A39] The composition according to any one of [A1] to [A38], wherein R₉ forms a 4-membered alicyclic ring together with Q₉ and a carbon atom bonded to R₉ and Q₉, the 4-membered alicyclic ring being substituted by two methyls.
[A40] The composition according to any one of [A1] to [A39], wherein P₉ is hydrogen or methyl.
[A41] The composition according to any one of [A1] to [A40], wherein R₁₀ is cyclopentyl.
[A42] The composition according to any one of [A1] to [A41], wherein P₁₀ is methyl.
[A43] The composition according to any one of [A1] to [A42], wherein R₁₁ is dimethylaminocarbonyl.
[A44] The composition according to any one of [A1] to [A43], wherein P₁₁ is methyl.
[A45] The composition according to any one of [A1] to [A20], wherein the compound represented by general formula (1) is a compound represented by formula (1a). 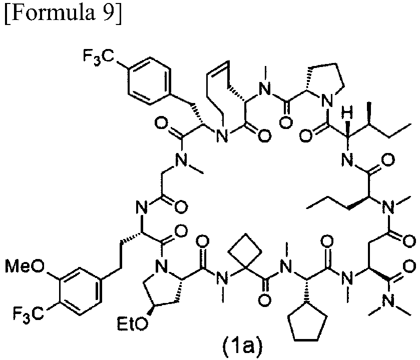
[A46] The composition according to any one of [A1] to [A20], wherein the compound represented by general formula (1) is a compound represented by formula (1b). 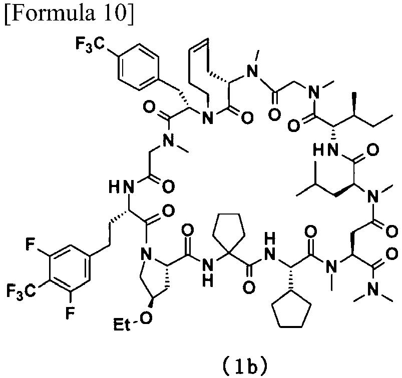
[A47] The composition according to any one of [A1] to [A20], wherein the compound represented by general formula (1) is a compound represented by formula (1c). 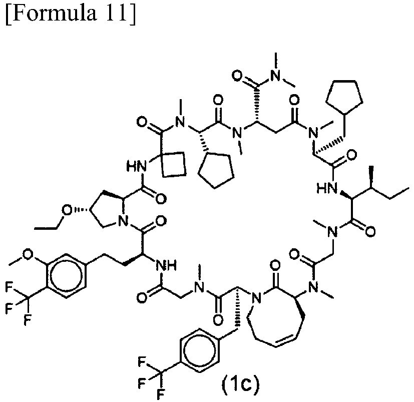
[A48] The composition according to any one of [A1] to [A20], wherein the compound represented by general formula (1) is a compound represented by formula (1d). 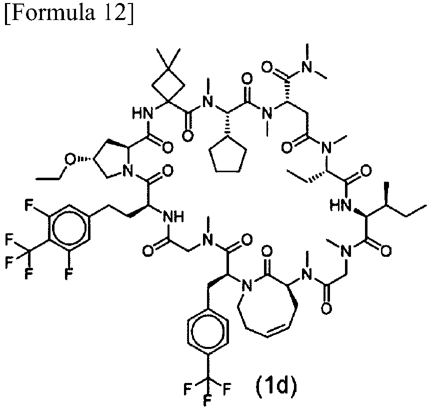
[A49] The composition according to any one of [A1] to [A20], wherein the compound represented by general formula (1) is a compound represented by formula (1e). 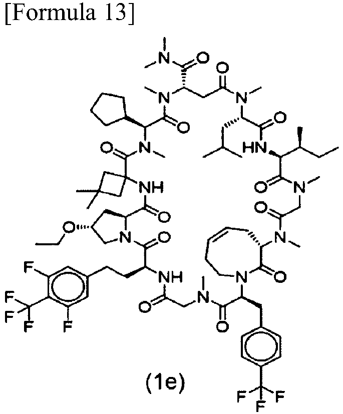
[A50] The composition according to any one of [A1] to [A20], wherein the compound represented by general formula (1) is a compound represented by formula (1 f). 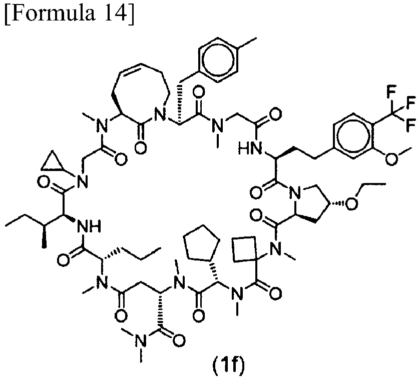
[A51] The composition according to any one of [A1] to [A50], comprising a pharmaceutically acceptable excipient.
[A52] The composition according to any one of [A1] to [A51], in the form of a capsule or a tablet.
[A53] The composition according to any one of [A1] to [A52], which is a pharmaceutical composition.
[A54] The composition according to any one of [A1] to [A53], wherein a value of the bioavailability (BA) of the compound as measured in a system in which the surfactant is present is 1.1-fold or more the value as measured in a system in which the surfactant is not present.
[A55] The composition according to any one of [A1] to [A54], wherein a value of the bioavailability (BA) of the compound as measured in a system in which the surfactant is present is 1.2-fold or more, 1.4-fold or more, 1.6-fold or more, 1.8-fold or more, or 2.0-fold or more the value as measured in a system in which the surfactant is not present.
[A56] The composition according to any one of [A1] to [A55], wherein a value of bioavailability (BA) of the compound as measured in a system in which the surfactant is present is 2.0-fold or more the value as measured in a system in which the surfactant is not present.
[A57] The composition according to any one of [A1] to [A56], wherein a value of Caco-2 Papp (cm/sec) of the compound as measured in a system in which the surfactant is present is 1.1-fold or more the value as measured in a system in which the surfactant is not present.
[A58] The composition according to any one of [A1] to [A57], wherein a value of Caco-2 Papp (cm/sec) of the compound as measured in a system in which the surfactant is present is 1.2-fold or more, 1.5-fold or more, 2-fold or more, 3-fold or more, or 5-fold or more the value as measured in a system in which the surfactant is not present.
[A59] The composition according to any one of [A1] to [A58], wherein a value of Caco-2 Papp (cm/sec) of the compound as measured in a system in which the surfactant is present is 2-fold or more the value as measured in a system in which the surfactant is not present.
[A60] The composition according to any one of [A1] to [A59], further comprising a solubility improver.
[A61] The composition according to [A60], wherein a content of the solubility improver is 0.05% by volume or more and 50% by volume or less based on 100% by volume of a liquid component contained in the composition.
[A62] The composition according to [A60] or [A61], wherein a content of the solubility improver is 0.3% by volume or more and 30% by volume or less, preferably 0.5% by volume or more and 15% by volume or less, more preferably 0.8% by volume or more and 10% by volume or less based on 100% by volume of a liquid component contained in the composition.
[A63] The composition according to any one of [A60] to [A62], wherein the solubility improver comprises a polyoxyethylene structure.
[A64] The composition according to [A63], wherein an average number of moles of ethylene oxide added in the solubility improver is 2 or more and 100 or less.
[A65] The composition according to any one of [A60] to [A64], wherein the solubility improver is polyoxyethylene castor oil or polyoxyethylene sorbitan fatty acid ester.
[A66] The composition according to any one of [A60] to [A62], wherein the solubility improver comprises a polymer that forms a solid dispersion with the compound.
[A67] The composition according to [A66], wherein the polymer that forms a solid dispersion is selected from the group consisting of polyethylene glycol, polyvinylpyrrolidone, copovidone, polyvinyl alcohol, cellulose-based polymers, and methacrylic acid methacrylic acid copolymers.
[A68] The composition according to [A66], wherein the polymer that forms a solid dispersion is selected from the group consisting of polyvinyl alcohol, polyvinyl alcohol polyvinyl acetate copolymers, polyvinyl pyrrolidone, copovidone, copolymers of acrylate and methacrylic acid, polyethylene polyvinyl alcohol copolymers, polyoxyethylene-polyoxypropylene block copolymers (also called as poloxamer), polyethylene glycol, hydroxypropyl methyl cellulose acetate (HPMCA), hydroxypropyl methyl cellulose (HPMC), hydroxypropyl cellulose (HPC), methylcellulose, hydroxyethyl methyl cellulose, hydroxyethyl cellulose, hydroxyethyl cellulose acetate, hydroxyethyl ethyl cellulose, cellulose acetate phthalate, cellulose acetate trimellitate, cellulose acetate succinate, methyl cellulose phthalate, hydroxymethyl cellulose ethyl phthalate, hydroxypropyl methyl cellulose phthalate, hydroxypropyl methyl cellulose acetate succinate (HPMCAS), hydroxypropyl methyl acetate maleate, hydroxypropyl methyl trimellitate, carboxymethyl ethyl cellulose, polyvinyl butylate phthalate, polyvinyl alcohol acetate phthalate, methacrylic acid/ethyl acrylate copolymers (preferably in a mass ratio of 1 : 99 to 99 : 1), methacrylic acid/methyl methacrylate copolymers (preferably in a mass ratio of 1 : 99 to 99 : 1), methacrylic acid copolymers, aminoalkyl methacrylic acid copolymer E, and polyvinyl acetal diethyl aminoacetate.
[A69] The composition according to any one of [A1] to [A68], wherein the composition comprises 0.05 parts by mass or more and 300 parts by mass or less of the surfactant based on 1 part by mass of the compound.
[A70] The composition according to any one of [A1] to [A69], wherein the composition comprises 0.075 parts by mass or more and 80 parts by mass or less, preferably 0.1 parts by mass or more and 60 parts by mass or less, more preferably 0.2 parts by mass or more and 40 parts by mass or less, and further preferably 0.3 parts by mass or more and 30 parts by mass or less of the surfactant based on 1 part by mass of the compound.
[A71] The composition according to any one of [A1] to [A70], which is a composition for administration.
[A72] The composition according to any one of [A1] to [A71], which is a composition for oral administration.
[A73] The composition according to any one of [A1] to [A72], which is a composition for promoting absorption of the compound.
[A74] The composition according to [A2], wherein the amount of surfactant used in combination is 0.05 parts by mass or more and 300 parts by mass or less based on 1 part by mass of the compound.
[A75] The composition according to [A2] or [A74], wherein the amount of surfactant used in combination is 0.075 parts by mass or more and 80 parts by mass or less, preferably 0.1 parts by mass or more and 60 parts by mass or less, more preferably 0.2 parts by mass or more and 40 parts by mass or less, and further preferably 0.3 parts by mass or more and 30 parts by mass or less based on 1 part by mass of the compound.
[A76] The composition according to any one of [A1] to [A75], which is a pharmaceutical composition for treating or preventing cancer.
[A77] A method for improving the absorption of a compound represented by general formula (1),
   the method comprising incorporating, in a same composition, a compound represented by general formula (1), a pharmaceutically acceptable salt thereof, or a pharmaceutically acceptable solvate thereof, and at least one surfactant selected from the group consisting of lauroyl-L-carnitine, sodium caprylate, sodium lauryl sulfate, and sodium N-(8-[2-hydroxybenzoyl]amino)caprylate: 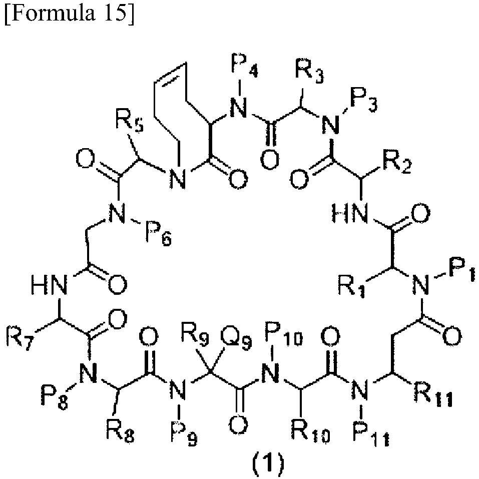 [wherein R₁ is C₁ to C₆ alkyl or C₃ to C₈ cycloalkyl C₁ to C₆ alkyl;
   P₁ is C₁ to C₆ alkyl;
   R₂ is C₁ to C₆ alkyl;
   R₃ is hydrogen, or forms a 4- to 7-membered saturated heterocyclic ring together with a carbon atom bonded to P₃ and R₃ and a nitrogen atom bonded to P₃;
   P₃ is C₁ to C₆ alkyl or C₃ to C₈ cycloalkyl except when R₃ and P₃ form a 4- to 7-membered saturated heterocyclic ring;
   P₄ is C₁ to C₆ alkyl;
   R₅ is benzyl optionally substituted by one or more groups selected from the group consisting of C₁ to C₆ alkyls, C₁ to C₆ haloalkyls, and C₃ to C₈ cycloalkyls;
   P₆ is C₁ to C₆ alkyl;
   R₇ is phenethyl optionally substituted by one or more groups selected from the group consisting of halogens, C₁ to C₆ haloalkyls, and C₁ to C₆ alkoxys;
   R₈ forms a 4- to 7-membered saturated heterocyclic ring together with a carbon atom bonded to P₈ and R₈ and a nitrogen atom bonded to P₈, the 4- to 7-membered saturated heterocyclic ring being optionally substituted by a C₁ to C₆ alkoxy;
   R₉ forms a 3- to 8-membered alicyclic ring together with Q₉ and a carbon atom bonded to R₉ and Q₉, the 3- to 8-membered alicyclic ring being optionally substituted by one or more C₁ to C₆ alkyls;
   P₉ is hydrogen or C₁ to C₆ alkyl;
   R₁₀ is C₁ to C₆ alkyl or C₃ to C₈ cycloalkyl;
   P₁₀ is C₁ to C₆ alkyl;
   R₁₁ is di-C₁ to C₆ alkylaminocarbonyl or 4- to 8-membered cyclic aminocarbonyl; and
   P₁₁ is C₁ to C₆ alkyl].
[A78] A method for improving the absorption of a compound represented by general formula (1),
   the method comprising using a compound represented by general formula (1), a pharmaceutically acceptable salt thereof, or a pharmaceutically acceptable solvate thereof, in combination with at least one surfactant selected from the group consisting of lauroyl-L-carnitine, sodium caprylate, sodium lauryl sulfate, and sodium N-(8-[2-hydroxybenzoyl]amino)caprylate: 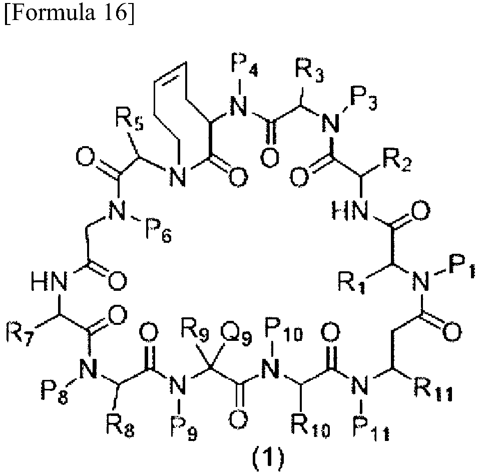 [wherein R₁ is C₁ to C₆ alkyl or C₃ to C₈ cycloalkyl C₁ to C₆ alkyl;
   P₁ is C₁ to C₆ alkyl;
   R₂ is C₁ to C₆ alkyl;
   R₃ is hydrogen, or forms a 4- to 7-membered saturated heterocyclic ring together with a carbon atom bonded to P₃ and R₃ and a nitrogen atom bonded to P₃;
   P₃ is C₁ to C₆ alkyl or C₃ to C₈ cycloalkyl except when R₃ and P₃ form a 4- to 7-membered saturated heterocyclic ring;
   P₄ is C₁ to C₆ alkyl;
   R₅ is benzyl optionally substituted by one or more groups selected from the group consisting of C₁ to C₆ alkyls, C₁ to C₆ haloalkyls, and C₃ to C₈ cycloalkyls;
   P₆ is C₁ to C₆ alkyl;
   R₇ is phenethyl optionally substituted by one or more groups selected from the group consisting of halogens, C₁ to C₆ haloalkyls, and C₁ to C₆ alkoxys;
   R₈ forms a 4- to 7-membered saturated heterocyclic ring together with a carbon atom bonded to P₈ and R₈ and a nitrogen atom bonded to P₈, the 4- to 7-membered saturated heterocyclic ring being optionally substituted by a C₁ to C₆ alkoxy;
   R₉ forms a 3- to 8-membered alicyclic ring together with Q₉ and a carbon atom bonded to R₉ and Q₉, the 3- to 8-membered alicyclic ring being optionally substituted by one or more C₁ to C₆ alkyls;
   P₉ is hydrogen or C₁ to C₆ alkyl;
   R₁₀ is C₁ to C₆ alkyl or C₃ to C₈ cycloalkyl;
   P₁₀ is C₁ to C₆ alkyl;
   R₁₁ is di-C₁ to C₆ alkylaminocarbonyl or 4- to 8-membered cyclic aminocarbonyl; and
   P₁₁ is C₁ to C₆ alkyl].
[A79] A method for improving the absorption of a compound represented by general formula (1),
   the method comprising using a composition comprising a compound represented by general formula (1), a pharmaceutically acceptable salt thereof, or a pharmaceutically acceptable solvate thereof, in combination with a composition comprising at least one surfactant selected from the group consisting of lauroyl-L-carnitine, sodium caprylate, sodium lauryl sulfate, and sodium N-(8-[2-hydroxybenzoyl]amino)caprylate: 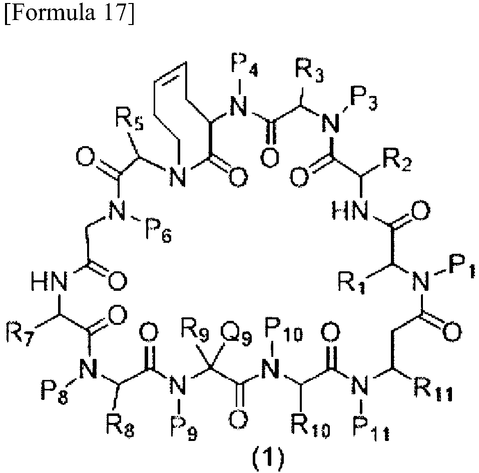 [wherein R₁ is C₁ to C₆ alkyl or C₃ to C₈ cycloalkyl C₁ to C₆ alkyl;
   P₁ is C₁ to C₆ alkyl;
   R₂ is C₁ to C₆ alkyl;
   R₃ is hydrogen, or forms a 4- to 7-membered saturated heterocyclic ring together with a carbon atom bonded to P₃ and R₃ and a nitrogen atom bonded to P₃;
   P₃ is C₁ to C₆ alkyl or C₃ to C₈ cycloalkyl except when R₃ and P₃ form a 4- to 7-membered saturated heterocyclic ring;
   P₄ is C₁ to C₆ alkyl;
   R₅ is benzyl optionally substituted by one or more groups selected from the group consisting of C₁ to C₆ alkyls, C₁ to C₆ haloalkyls, and C₃ to C₈ cycloalkyls;
   P₆ is C₁ to C₆ alkyl;
   R₇ is phenethyl optionally substituted by one or more groups selected from the group consisting of halogens, C₁ to C₆ haloalkyls, and C₁ to C₆ alkoxys;
   R₈ forms a 4- to 7-membered saturated heterocyclic ring together with a carbon atom bonded to P₈ and R₈ and a nitrogen atom bonded to P₈, the 4- to 7-membered saturated heterocyclic ring being optionally substituted by a C₁ to C₆ alkoxy;
   R₉ forms a 3- to 8-membered alicyclic ring together with Q₉ and a carbon atom bonded to R₉ and Q₉, the 3- to 8-membered alicyclic ring being optionally substituted by one or more C₁ to C₆ alkyls;
   P₉ is hydrogen or C₁ to C₆ alkyl;
   R₁₀ is C₁ to C₆ alkyl or C₃ to C₈ cycloalkyl;
   P₁₀ is C₁ to C₆ alkyl;
   R₁₁ is di-C₁ to C₆ alkylaminocarbonyl or 4- to 8-membered cyclic aminocarbonyl; and
   P₁₁ is C₁ to C₆ alkyl].
[A80] A method for producing the composition according to any one of [A1] to [A76], comprising the following steps (a) and (b):
   (a) providing the compound represented by general formula (1), a pharmaceutically acceptable salt thereof, or a pharmaceutically acceptable solvate thereof, and
   (b) mixing the surfactant as an isolated component with the compound represented by general formula (1), a pharmaceutically acceptable salt thereof, or a pharmaceutically acceptable solvate thereof.
[A81] The production method according to [A80], further comprising the following step (c):
   (c) mixing a solubility improver with the compound represented by general formula (1), a pharmaceutically acceptable salt thereof, or a pharmaceutically acceptable solvate thereof.
[A82] A composition comprising a compound represented by formula (1a), a pharmaceutically acceptable salt thereof, or a pharmaceutically acceptable solvate thereof, and lauroyl-L-carnitine. 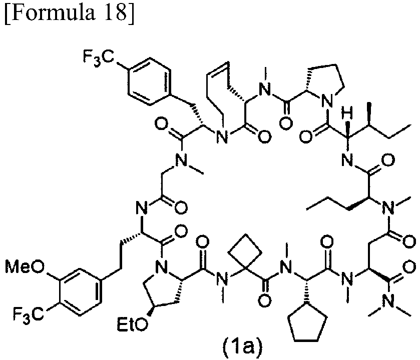
[A83] A composition comprising a compound represented by formula (1a), a pharmaceutically acceptable salt thereof, or a pharmaceutically acceptable solvate thereof, and sodium lauryl sulfate. 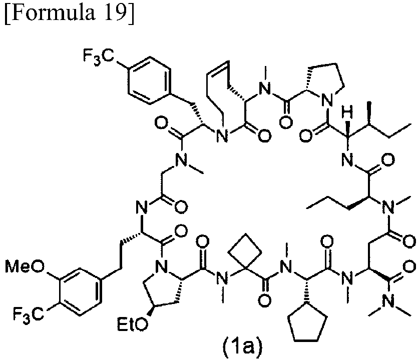
[A84] A composition comprising a compound represented by formula (1a), a pharmaceutically acceptable salt thereof, or a pharmaceutically acceptable solvate thereof, and sodium N-(8-[2-hydroxybenzoyl]amino)caprylate. 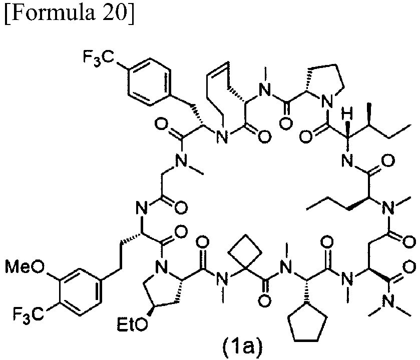
[A85] A composition comprising a compound represented by formula (1b), a pharmaceutically acceptable salt thereof, or a pharmaceutically acceptable solvate thereof, and lauroyl-L-carnitine. 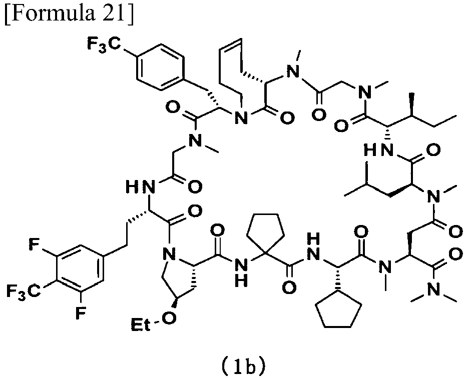
[A86] A composition comprising a compound represented by formula (1b), a pharmaceutically acceptable salt thereof, or a pharmaceutically acceptable solvate thereof, and sodium lauryl sulfate. 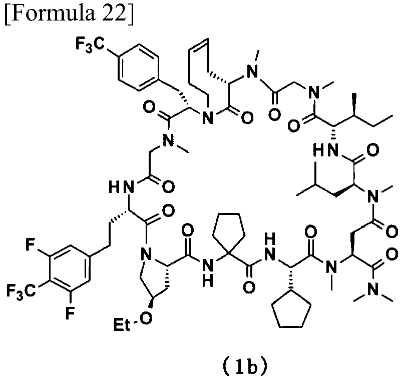
[A87] A composition comprising a compound represented by formula (1b), a pharmaceutically acceptable salt thereof, or a pharmaceutically acceptable solvate thereof, and sodium N-(8-[2-hydroxybenzoyl]amino)caprylate. 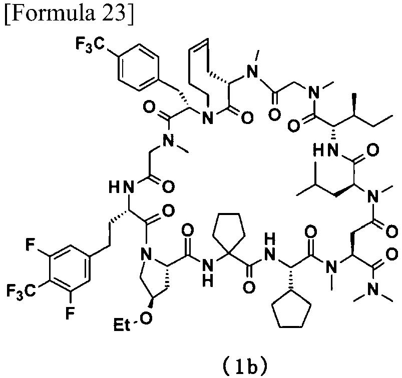
[A88] A composition comprising a compound represented by formula (1c), a pharmaceutically acceptable salt thereof, or a pharmaceutically acceptable solvate thereof, and lauroyl-L-carnitine. 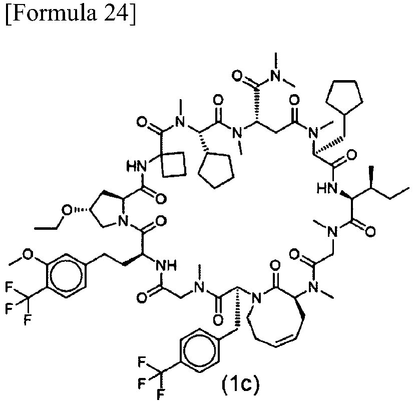
[A89] A composition comprising a compound represented by formula (1c), a pharmaceutically acceptable salt thereof, or a pharmaceutically acceptable solvate thereof, and sodium lauryl sulfate. 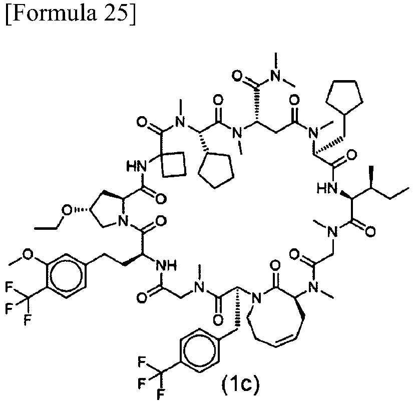
[A90] A composition comprising a compound represented by formula (1c), a pharmaceutically acceptable salt thereof, or a pharmaceutically acceptable solvate thereof, and sodium N-(8-[2-hydroxybenzoyl]amino)caprylate. 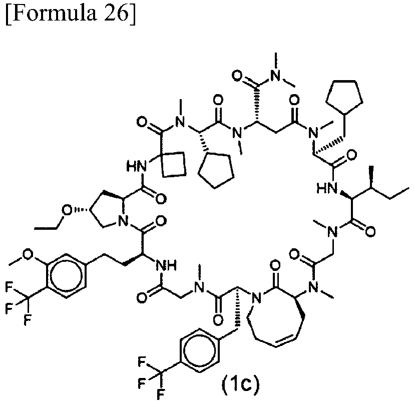
[A91] A composition comprising a compound represented by formula (1d), a pharmaceutically acceptable salt thereof, or a pharmaceutically acceptable solvate thereof, and lauroyl-L-carnitine. 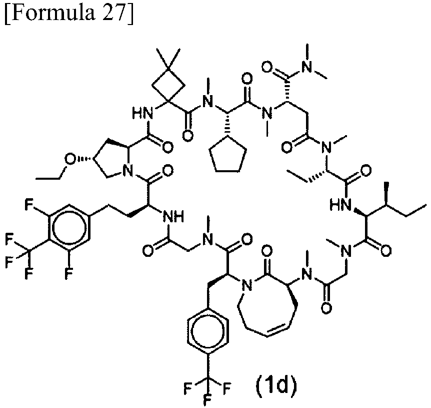
[A92] A composition comprising a compound represented by formula (1d), a pharmaceutically acceptable salt thereof, or a pharmaceutically acceptable solvate thereof, and sodium lauryl sulfate. 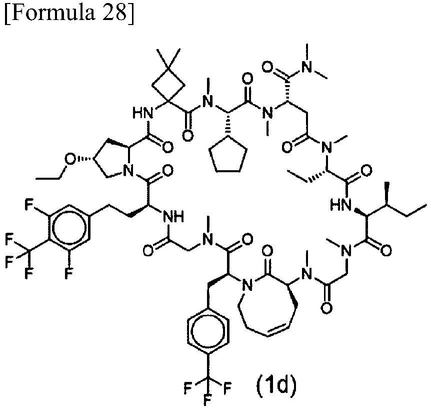
[A93] A composition comprising a compound represented by formula (1d), a pharmaceutically acceptable salt thereof, or a pharmaceutically acceptable solvate thereof, and sodium N-(8-[2-hydroxybenzoyl]amino)caprylate. 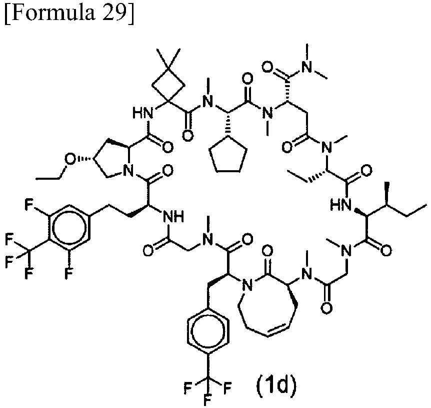
[A94] A composition comprising a compound represented by formula (1e), a pharmaceutically acceptable salt thereof, or a pharmaceutically acceptable solvate thereof, and lauroyl-L-carnitine. 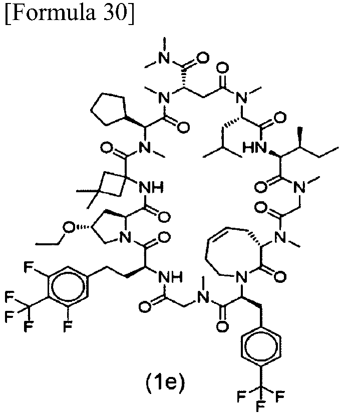
[A95] A composition comprising a compound represented by formula (1e), a pharmaceutically acceptable salt thereof, or a pharmaceutically acceptable solvate thereof, and sodium lauryl sulfate. 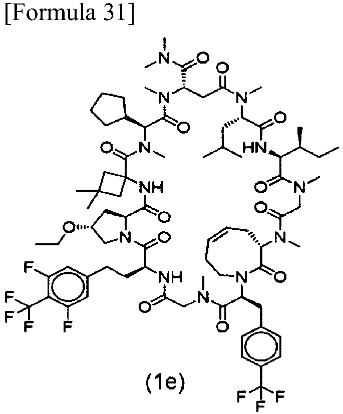
[A96] A composition comprising a compound represented by formula (1e), a pharmaceutically acceptable salt thereof, or a pharmaceutically acceptable solvate thereof, and sodium N-(8-[2-hydroxybenzoyl]amino)caprylate. 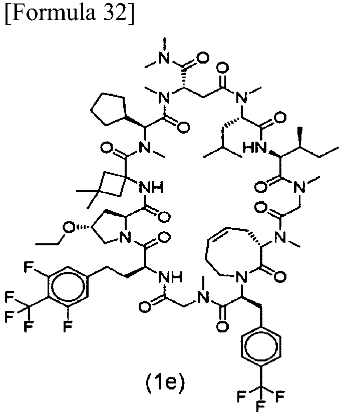
[A97] A composition comprising a compound represented by formula (1f), a pharmaceutically acceptable salt thereof, or a pharmaceutically acceptable solvate thereof, and lauroyl-L-carnitine. 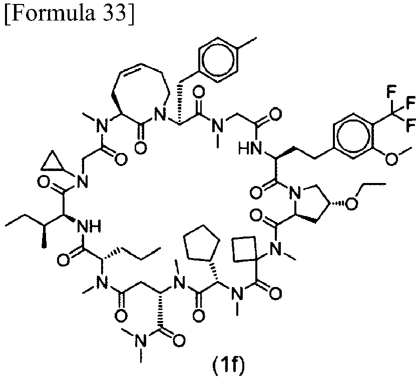
[A98] A composition comprising a compound represented by formula (1f), a pharmaceutically acceptable salt thereof, or a pharmaceutically acceptable solvate thereof, and sodium lauryl sulfate. 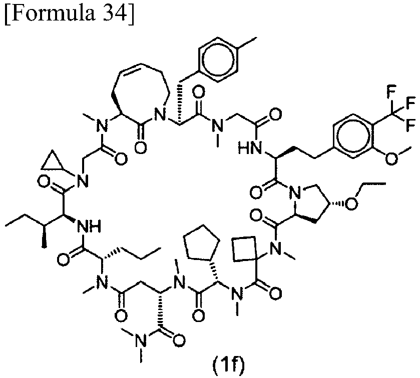
[A99] A composition comprising a compound represented by formula (1f), a pharmaceutically acceptable salt thereof, or a pharmaceutically acceptable solvate thereof, and sodium N-(8-[2-hydroxybenzoyl]amino)caprylate. 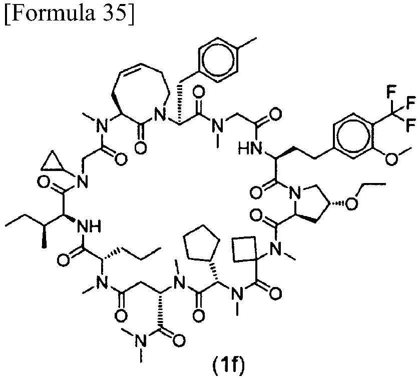
[A100] The composition according to any one of [A82] to [A99], which is a pharmaceutical composition for treating or preventing cancer.
[A101] A use of a surfactant for improving the absorption of a compound represented by general formula (1),
   wherein the surfactant is at least one selected from the group consisting of lauroyl-L-carnitine, sodium caprylate, sodium lauryl sulfate, and sodium N-(8-[2-hydroxybenzoyl]amino)caprylate: 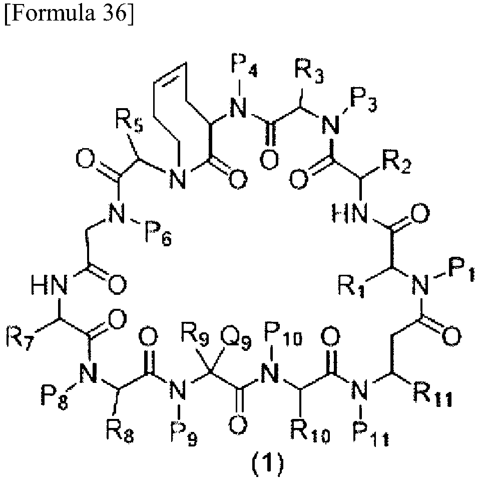 [wherein R₁ is C₁ to C₆ alkyl or C₃ to C₈ cycloalkyl C₁ to C₆ alkyl;
   P₁ is C₁ to C₆ alkyl;
   R₂ is C₁ to C₆ alkyl;
   R₃ is hydrogen, or forms a 4- to 7-membered saturated heterocyclic ring together with a carbon atom bonded to P₃ and R₃ and a nitrogen atom bonded to P₃;
   P₃ is C₁ to C₆ alkyl or C₃ to C₈ cycloalkyl except when R₃ and P₃ form a 4- to 7-membered saturated heterocyclic ring;
   P₄ is C₁ to C₆ alkyl;
   R₅ is benzyl optionally substituted by one or more groups selected from the group consisting of C₁ to C₆ alkyls, C₁ to C₆ haloalkyls, and C₃ to C₈ cycloalkyls;
   P₆ is C₁ to C₆ alkyl;
   R₇ is phenethyl optionally substituted by one or more groups selected from the group consisting of halogens, C₁ to C₆ haloalkyls, and C₁ to C₆ alkoxys;
   R₈ forms a 4- to 7-membered saturated heterocyclic ring together with a carbon atom bonded to P₈ and R₈ and a nitrogen atom bonded to P₈, the 4- to 7-membered saturated heterocyclic ring being optionally substituted by a C₁ to C₆ alkoxy;
   R₉ forms a 3- to 8-membered alicyclic ring together with Q₉ and a carbon atom bonded to R₉ and Q₉, the 3- to 8-membered alicyclic ring being optionally substituted by one or more C₁ to C₆ alkyls;
   P₉ is hydrogen or C₁ to C₆ alkyl;
   R₁₀ is C₁ to C₆ alkyl or C₃ to C₈ cycloalkyl;
   P₁₀ is C₁ to C₆ alkyl;
   R₁₁ is di-C₁ to C₆ alkylaminocarbonyl or 4- to 8-membered cyclic aminocarbonyl; and
   P₁₁ is C₁ to C₆ alkyl].

### Advantageous Effects of Invention

The present invention can provide a composition containing a cyclic peptide compound and a surfactant, having enhanced membrane permeability and/or BA of the cyclic peptide compound. The present invention can also provide a composition containing a cyclic peptide compound for use in combination with a surfactant. The present invention can further provide a method for using a surfactant to improve the membrane permeability and/or BA of the cyclic peptide compound.

### Description of Embodiments

Hereinafter, the embodiments of the present invention will be described in detail. However, the present invention is not limited by the embodiments given below.

The term "one or more" as used herein means the number of 1 or 2 or larger. When the term "one or more" is used in the context associated with a substituent of a group, the term means a number from 1 to the maximum number of substituents acceptable by the group. Specific examples of the term "one or more" include 1, 2, 3, 4, 5, 6, 7, 8, 9, 10, and/or larger numbers.

As used herein, the term "to" that indicates a range includes values of both ends thereof. For example, "A to B" means the range of A or more and B or less.

The term "about" as used herein, when used in combination with a numeric value, means the value range of +10% and -10% of the numeric value.

In the present invention, the meaning of the term "and/or" includes any combination in which "and" and "or" are appropriately combined. Specifically, for example, the term "A, B, and/or C" includes the following seven variations: (i) A, (ii) B, (iii) C, (iv) A and B, (v) A and C, (vi) B and C, and (vii) A, B, and C.

The molecular weight as used herein means the sum of the atomic weights of the atoms constituting the compound molecule (unit: "g/mol"), and is obtained by calculating the sum of the atomic weights of the atoms included in the molecular structure formula (unit "g/mol").

The use of the articles "one (a)", "one (an)", and "the" in both the specification and claims is to be construed as including both the singular and the plural, unless specifically indicated herein or expressly denied by context.

Examples of the "halogen atom" as used herein include F, Cl, Br, and I.

As used herein, the term "alkyl" is a monovalent group induced by removing any one hydrogen atom from aliphatic hydrocarbon, and has a subset of a hydrocarbyl or hydrocarbon group structure containing hydrogen and carbon atoms without containing a heteroatom (which refers to an atom other than carbon and hydrogen atoms) or an unsaturated carbon-carbon bond in the skeleton. The alkyl includes not only a linear form but also a branched form. The alkyl is specifically alkyl having 1 to 20 carbon atoms (C₁ to C₂₀; hereinafter, "Cₚ to C_{q}" means that the number of carbon atoms is p to q), preferably C₁ to C₁₀ alkyl, and more preferably C₁ to C₆ alkyl. Specific examples of the alkyl include methyl, ethyl, n-propyl, i-propyl, n-butyl, s-butyl, t-butyl, isobutyl (2-methylpropyl), n-pentyl, s-pentyl (1-methylbutyl), t-pentyl (1,1-dimethylpropyl), neopentyl (2,2-dimethylpropyl), isopentyl (3-methylbutyl), 3-pentyl (1-ethylpropyl), 1,2-dimethylpropyl, 2-methylbutyl, n-hexyl, 1,1,2-trimethylpropyl, 1,2,2-trimethylpropyl, 1,1,2,2-tetramethylpropyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, and 2-ethylbutyl.

As used herein, the term "alkenyl" is a monovalent group having at least one double bond (two adjacent SP² carbon atoms). Depending on the conformation of the double bond and a substituent (if present), the geometric morphology of the double bond can assume entgegen (E) or zusammen (Z) and cis or trans conformations. The alkenyl includes not only a linear form but also a branched form. The alkenyl is preferably C₂ to C₁₀ alkenyl, more preferably C₂ to C₇ alkenyl or C₂ to C₆ alkenyl. Specific examples thereof include vinyl, allyl, 1-propenyl, 2-propenyl, 1-butenyl, 2-butenyl (which includes cis and trans), 3-butenyl, pentenyl, 3-methyl-2-butenyl, hexenyl, and 6-heptenyl.

As used herein, the term "alkynyl" is a monovalent group having at least one triple bond (two adjacent SP carbon atoms). The alkynyl includes not only a linear form but also a branched form. The alkynyl is preferably C₂ to C₁₀ alkynyl, more preferably C₂ to C₆ alkynyl. Specific examples thereof include ethynyl, 1-propynyl, propargyl, 3-butynyl, pentynyl, hexynyl, 3-phenyl-2-propynyl, 3-(2'-fluorophenyl)-2-propynyl, 2-hydroxy-2-propynyl, 3-(3-fluorophenyl)-2-propynyl, and 3-methyl-(5-phenyl)-4-pentynyl.

The term "cycloalkyl" as used herein means a saturated or partially saturated cyclic monovalent aliphatic hydrocarbon group and includes a monocyclic ring, a bicyclo ring, and a spiro ring. The cycloalkyl is preferably C₃ to C₈ cycloalkyl. Specific examples thereof include cyclopropyl, cyclobutyl, cyclopentyl, cyclohexyl, cycloheptyl, cyclooctyl, bicyclo[2.2.1]heptyl, and spiro[3.3]heptyl.

The term "aryl" as used herein means a monovalent aromatic hydrocarbon ring and is preferably C₆ to C₁₀ aryl. Specific examples of the aryl include phenyl and naphthyl (e.g., 1-naphthyl and 2-naphthyl). As used herein, the aryl includes a bicyclic aryl in which the aromatic hydrocarbon ring is condensed with another saturated or unsaturated ring. For example, the aryl includes an aryl of a condensed ring structure in which the aromatic hydrocarbon ring is a benzene ring and the saturated ring is a 5-membered, 6-membered, or 7-membered saturated hydrocarbon ring or a saturated heterocyclic ring. Specific examples thereof include indanyl, 1,2,3,4-tetrahydronaphthyl, and 2,3-dihydrobenzofuran.

The term "heterocyclyl" as used herein means a nonaromatic cyclic monovalent group containing a carbon atom as well as 1 to 5 heteroatoms. The heterocyclyl may have a double and/or triple bond in the ring. A carbon atom in the ring may form carbonyl through oxidation, and the ring may be a monocyclic ring or a condensed ring. The number of atoms constituting the ring is preferably 3 to 10 (3- to 10-membered heterocyclyl), or 4 to 10 (4- to 10-membered heterocyclyl), more preferably 3 to 7 (3- to 7-membered heterocyclyl), or 4 to 7 (4- to 7-membered heterocyclyl). Specific examples of the heterocyclyl include azetidinyl, oxiranyl, oxetanyl, azetidinyl, dihydrofuryl, tetrahydrofuryl, dihydropyranyl, tetrahydropyranyl, tetrahydropyridyl, tetrahydropyrimidyl, morpholinyl, thiomorpholinyl, pyrrolidinyl, piperidinyl, piperazinyl, pyrazolidinyl, imidazolinyl, imidazolidinyl, oxazolidinyl, isoxazolidinyl, thiazolidinyl, isothiazolidinyl, 1,2-thiazinane, thiadiazolidinyl, azetidinyl, oxazolidone, benzodioxanyl, benzoxazolyl, dioxolanyl, dioxanyl, tetrahydropyrrolo[1,2-c]imidazole, thietanyl, 3,6-diazabicyclo[3.1.1]heptanyl, 2,5-diazabicyclo[2.2.1]heptanyl, 3-oxa-8-azabicyclo[3.2.1]octanyl, sultam, and 2-oxaspiro[3.3]heptyl.

The term "heteroaryl" as used herein means an aromatic cyclic monovalent group containing a carbon atom as well as 1 to 5 heteroatoms. The ring may be a monocyclic ring or a condensed ring with another ring and may be partially saturated. The number of atoms constituting the ring is preferably 5 to 10 (5- to 10-membered heteroaryl), more preferably 5 to 7 (5- to 7-membered heteroaryl). Specific examples of the heteroaryl include furyl, thienyl, pyrrolyl, imidazolyl, pyrazolyl, thiazolyl, isothiazolyl, oxazolyl, isoxazolyl, oxadiazolyl, thiadiazolyl, triazolyl, tetrazolyl, pyridyl, pyrimidyl, pyridazinyl, pyrazinyl, triazinyl, benzofuranyl, benzothienyl, benzothiadiazolyl, benzothiazolyl, benzoxazolyl, benzoxadiazolyl, benzimidazolyl, indolyl, isoindolyl, indazolyl, quinolyl, isoquinolyl, cinnolinyl, quinazolinyl, quinoxalinyl, benzodioxolyl, indolizinyl, and imidazopyridyl.

The term "alkoxy" as used herein means an oxy group bonded to the "alkyl" defined above and is preferably C₁ to C₆ alkoxy. Specific examples of the alkoxy include methoxy, ethoxy, 1-propoxy, 2-propoxy, n-butoxy, i-butoxy, s-butoxy, t-butoxy, pentyloxy, and 3-methylbutoxy.

The term "alkylthio" as used herein means a thiol group bonded to the "alkyl" defined above and is preferably C₁ to C₆ alkylthio. Specific examples of the alkylthio include methylthio, ethylthio, 1-propylthio, 2-propylthio, n-butylthio, i-butylthio, s-butylthio, and t-butylthio.

The term "alkenyloxy" as used herein means an oxy group bonded to the "alkenyl" defined above and is preferably C₂ to C₆ alkenyloxy. Specific examples of the alkenyloxy include vinyloxy, allyloxy, 1-propenyloxy, 2-propenyloxy, 1-butenyloxy, 2-butenyloxy (which includes cis and trans), 3-butenyloxy, pentenyloxy, and hexenyloxy.

The term "cycloalkoxy" as used herein means an oxy group bonded to the "cycloalkyl" defined above and is preferably C₃ to C₈ cycloalkoxy. Specific examples of the cycloalkoxy include cyclopropoxy, cyclobutoxy, and cyclopentyloxy.

The term "aryloxy" as used herein means an oxy group bonded to the "aryl" defined above and is preferably C₆ to C₁₀ aryloxy. Specific examples of the aryloxy include phenoxy, 1-naphthyloxy, and 2-naphthyloxy.

The term "amino" as used herein means -NH₂ in the narrow sense and means -NRR' in the broad sense. In this context, R and R' are each independently selected from hydrogen, alkyl, alkenyl, alkynyl, cycloalkyl, heterocyclyl, aryl, or heteroaryl, or R and R' form a ring together with the nitrogen atom bonded thereto. Examples of the amino preferably include -NH₂, mono-C₁ to C₆ alkylamino, di-C₁ to C₆ alkylamino, and 4- to 8-membered cyclic amino.

The term "monoalkylamino" as used herein means a group of the "amino" defined above in which R is hydrogen, and R' is the "alkyl" defined above, and is preferably mono-C₁ to C₆ alkylamino. Specific examples of the monoalkylamino include methylamino, ethylamino, n-propylamino, i-propylamino, n-butylamino, s-butylamino, and t-butylamino.

The term "dialkylamino" as used herein means a group of the "amino" defined above in which R and R' are each independently the "alkyl" defined above, and is preferably di-C₁ to C₆ alkylamino. Specific examples of the dialkylamino include dimethylamino and diethylamino.

The term "cyclic amino" as used herein means a group of the "amino" defined above in which R and R' form a ring together with the nitrogen atom bonded thereto, and is preferably 4-to 8-membered cyclic amino. Specific examples of the cyclic amino include 1-azetidyl, 1-pyrrolidyl, 1-piperidyl, 1-piperazyl, 4-morpholinyl, 3-oxazolidyl, 1,1-dioxidothiomorpholinyl-4-yl, and 3-oxa-8-azabicyclo[3.2.1]octan-8-yl.

The term "alkylcarbonyl" as used herein means a carbonyl group bonded to the "alkyl" defined above and is preferably C₁ to C₆ alkylcarbonyl. Specific examples of the alkylcarbonyl include acetyl, propionyl, and butyryl. The number of carbon atoms indicated in the above definition indicates the number of carbon atoms in the alkyl moiety. For example, "C₁ to C₆" in "C₁ to C₆ alkylcarbonyl" indicates that the alkyl moiety has 1 to 6 carbon atoms.

The term "aminocarbonyl" as used herein means a carbonyl group bonded to the "amino" defined above and is preferably -CONH₂, mono-C₁ to C₆ alkylaminocarbonyl, di-C₁ to C₆ alkylaminocarbonyl, or 4- to 8-membered cyclic aminocarbonyl. Specific examples of the aminocarbonyl include -CONH₂, dimethylaminocarbonyl, 1-azetidinylcarbonyl, 1-pyrrolidinylcarbonyl, 1-piperidinylcarbonyl, 1-piperazinylcarbonyl, 4-morpholinylcarbonyl, 3-oxazolidinylcarbonyl, 1,1-dioxidothiomorpholinyl-4-ylcarbonyl, and 3-oxa-8-azabicyclo[3.2.1]octane-8-ylcarbonyl.

The term "alkenyloxycarbonyl" as used herein means a carbonyl group bonded to the "alkenyloxy" defined above and is preferably C₂ to C₆ alkenyloxycarbonyl. Specific examples of the alkenyloxycarbonyl include vinyloxycarbonyl, allyloxycarbonyl, 1-propenyloxycarbonyl, 2-propenyloxycarbonyl, 1-butenyloxycarbonyl, 2-butenyloxycarbonyl (which includes cis and trans), 3-butenyloxycarbonyl, pentenyloxycarbonyl, and hexenyloxycarbonyl.

The term "alkylsulfonyl" as used herein means a sulfonyl group bonded to the "alkyl" defined above and is preferably C₁ to C₆ alkylsulfonyl. Specific examples of the alkylsulfonyl include methylsulfonyl.

The term "haloalkyl" as used herein means a group in which one or more hydrogens of the "alkyl" defined above are replaced with halogen, and is preferably C₁ to C₆ haloalkyl, more preferably C₁ to C₆ fluoroalkyl. Specific examples of the haloalkyl include difluoromethyl, trifluoromethyl, 2,2-difluoroethyl, 2,2,2-trifluoroethyl, 3,3-difluoropropyl, 4,4-difluorobutyl, 5,5-difluoropentyl, and 1,1-difluoroethyl.

The term "aminoalkyl" as used herein means a group in which one or more hydrogens of the "alkyl" defined above are replaced with the "amino" defined above, and is preferably C₁ to C₆ aminoalkyl. Specific examples of the aminoalkyl include 1-pyridylmethyl, 2-(1-piperidyl)ethyl, 3-(1-piperidyl)propyl, 4-aminobutyl, and 2-aminoethyl.

The term "cycloalkylalkyl" as used herein means a group in which one or more hydrogens of the "alkyl" defined above are replaced with the "cycloalkyl" defined above, and is preferably C₃ to C₈ cycloalkyl C₁ to C₆ alkyl, more preferably C₃ to C₆ cycloalkyl C₁ to C₂ alkyl. Specific examples of the cycloalkylalkyl include cyclopropylmethyl, cyclobutylmethyl, cyclopentylmethyl, and cyclohexylmethyl.

The term "aralkyl (arylalkyl)" as used herein means a group in which at least one hydrogen atom of the "alkyl" defined above is replaced with the "aryl" defined above, and is preferably C₇ to C₁₄ aralkyl, more preferably C₇ to C₁₀ aralkyl. Specific examples of the aralkyl include benzyl, phenethyl, and 3-phenylpropyl.

The term "alkylene" as used herein means a divalent group induced by further removing any one hydrogen atom from the "alkyl" described above, and is preferably C₄ to C₈ alkylene. Specific examples of the alkylene include -CH₂-, -(CH₂)₂-, -(CH₂)₃-, -CH(CH₃)CH₂-, -C(CH₃)₂-, -(CH₂)₄-, -CH(CH₃)CH₂CH₂-, -C(CH₃)₂CH₂-, -CH₂CH(CH₃)CH₂-, -CH₂C(CH₃)₂-, - CH₂CH₂CH(CH₃)-, -(CH₂)₅-, -(CH₂)₆-, -(CH₂)₇-, and -(CH₂)₈-.

The term "alkenylene" as used herein means a divalent group induced by further removing any one hydrogen atom from the "alkenyl" described above. Depending on the conformation of the double bond and a substituent (if present), the geometric morphology of the double bond can assume entgegen (E) or zusammen (Z) and cis or trans conformations. The alkenylene includes a linear or branched form and is preferably C₂ to C₁₀ alkenylene, more preferably C₂ to C₆ alkenylene.

The term "alkynylene" as used herein means a divalent group induced by further removing any one hydrogen atom from the "alkynyl" described above. The alkynylene includes a linear or branched form and is preferably C₂ to C₁₀ alkynylene, more preferably C₂ to C₆ alkynylene.

The term "alicyclic ring" as used herein means a nonaromatic hydrocarbon ring. The alicyclic ring may have an unsaturated bond in the ring and may be a polycyclic ring having two or more rings. A carbon atom constituting the ring may form carbonyl through oxidation. The alicyclic ring is preferably a 3- to 8-membered alicyclic ring. Specific examples thereof include a cyclopropane ring, a cyclobutane ring, a cyclopentane ring, a cyclohexane ring, a cycloheptane ring, a cyclooctane ring, and a bicyclo[2.2.1]heptane ring.

The term "saturated heterocyclic ring" as used herein means a nonaromatic heterocyclic ring containing a carbon atom as well as 1 to 5 heteroatoms without containing a double bond and/or a triple bond in the ring. The saturated heterocyclic ring may be a monocyclic ring or may form a condensed ring with another ring, for example, an aromatic ring such as a benzene ring. The saturated heterocyclic ring is preferably a 4- to 7-membered saturated heterocyclic ring. Specific examples thereof include an azetidine ring, an oxetane ring, a tetrahydrofuran ring, a tetrahydropyran ring, a morpholine ring, a thiomorpholine ring, a pyrrolidine ring, a 4-oxopyrrolidine ring, a piperidine ring, a 4-oxopiperidine ring, a piperazine ring, a pyrazolidine ring, an imidazolidine ring, an oxazolidine ring, an isoxazolidine ring, a thiazolidine ring, an isothiazolidine ring, a thiadiazolidine ring, an oxazolidone ring, a dioxolane ring, a dioxane ring, a thietane ring, an octahydroindole ring, an indoline ring, and an azepane ring.

The term "optionally substituted" as used herein means that a group may be substituted by any substituent.

Examples of the substituent containing a halogen atom herein include a halogen-derived substituent, an alkyl group, a cycloalkyl group, an alkenyl group, an alkynyl group, an aryl group, a heteroaryl group, and an aralkyl group having halogen as a substituent and more specifically include fluoroalkyl, difluoroalkyl, and trifluoroalkyl.

Examples of halogen-derived substituents include fluoro (-F), chloro (-Cl), bromo (-Br), and iodo (-I).

Examples of the substituent containing an O atom include groups such as hydroxy (-OH), oxy (-OR), carbonyl (-C(=O)-R), carboxy (-CO₂H), oxycarbonyl (-C(=O)-OR), carbonyloxy (-O-C(=O)-R), thiocarbonyl (-C(=O)-SR), carbonylthio (-S-C(=O)-R), aminocarbonyl (-C(=O)-NHR), carbonylamino (-NH-C(=O)-R), oxycarbonylamino (-NH-C(=O)-OR), sulfonylamino (-NH-SO₂-R), aminosulfonyl (-SO₂-NHR), sulfamoylamino (-NH-SO₂-NHR), thiocarboxy (-C(=O)-SH), and carboxycarbonyl (-C(=O)-CO₂H).

Examples of the oxy (-OR) include alkoxy, cycloalkoxy, alkenyloxy, alkynyloxy, aryloxy, heteroaryloxy, and aralkyloxy. The alkoxy is preferably C₁ to C₄ alkoxy or C₁ or C₂ alkoxy, particularly preferably methoxy or ethoxy.

Examples of carbonyl (-C(=O)-R) include formyl (-C(=O)-H), alkylcarbonyl, cycloalkylcarbonyl, alkenylcarbonyl, alkynylcarbonyl, arylcarbonyl, heteroarylcarbonyl, and aralkylcarbonyl.

Examples of oxycarbonyl (-C(=O)-OR) include alkyloxycarbonyl, cycloalkyloxycarbonyl, alkenyloxycarbonyl, alkynyloxycarbonyl, aryloxycarbonyl, heteroaryloxycarbonyl, and aralkyloxycarbonyl.

Examples of carbonyloxy (-O-C(=O)-R) include alkylcarbonyloxy, cycloalkylcarbonyloxy, alkenylcarbonyloxy, alkynylcarbonyloxy, arylcarbonyloxy, heteroarylcarbonyloxy, and aralkylcarbonyloxy.

Examples of thiocarbonyl (-C(=O)-SR) include alkylthiocarbonyl, cycloalkylthiocarbonyl, alkenylthiocarbonyl, alkynylthiocarbonyl, arylthiocarbonyl, heteroarylthiocarbonyl, and aralkylthiocarbonyl.

Examples of carbonylthio (-S-C(=O)-R) include alkylcarbonylthio, cycloalkylcarbonylthio, alkenylcarbonylthio, alkynylcarbonylthio, arylcarbonylthio, heteroarylcarbonylthio, and aralkylcarbonylthio.

Examples of the aminocarbonyl (-C(=O)-NHR) include alkylaminocarbonyl (e.g., C₁ to C₆ or C₁ to C₄ alkylaminocarbonyl, particularly, ethylaminocarbonyl and methylaminocarbonyl), cycloalkylaminocarbonyl, alkenylaminocarbonyl, alkynylaminocarbonyl, arylaminocarbonyl, heteroarylaminocarbonyl, and aralkylaminocarbonyl. Examples thereof additionally include compounds in which the H atom bonded to the N atom in -C(=O)-NHR is further replaced with alkyl, cycloalkyl, alkenyl, alkynyl, aryl, heteroaryl, or aralkyl.

Examples of the carbonylamino (-NH-C(=O)-R) include alkylcarbonylamino, cycloalkylcarbonylamino, alkenylcarbonylamino, alkynylcarbonylamino, arylcarbonylamino, heteroarylcarbonylamino, and aralkylcarbonylamino. Examples thereof additionally include compounds in which the H atom bonded to the N atom in -NH-C(=O)-R is further replaced with alkyl, cycloalkyl, alkenyl, alkynyl, aryl, heteroaryl, or aralkyl.

Examples of the oxycarbonylamino (-NH-C(=O)-OR) include alkoxycarbonylamino, cycloalkoxycarbonylamino, alkenyloxycarbonylamino, alkynyloxycarbonylamino, aryloxycarbonylamino, heteroaryloxycarbonylamino, and aralkyloxycarbonylamino. Examples thereof additionally include compounds in which the H atom bonded to the N atom in -NH-C(=O)-OR is further replaced with alkyl, cycloalkyl, alkenyl, alkynyl, aryl, heteroaryl, or aralkyl.

Examples of the sulfonylamino (-NH-SO₂-R) include alkylsulfonylamino, cycloalkylsulfonylamino, alkenylsulfonylamino, alkynylsulfonylamino, arylsulfonylamino, heteroarylsulfonylamino, and aralkylsulfonylamino. Examples thereof additionally include compounds in which the H atom bonded to the N atom in -NH-SO₂-R is further replaced with alkyl, cycloalkyl, alkenyl, alkynyl, aryl, heteroaryl, or aralkyl.

Examples of the aminosulfonyl (-SO₂-NHR) include alkylaminosulfonyl, cycloalkylaminosulfonyl, alkenylaminosulfonyl, alkynylaminosulfonyl, arylaminosulfonyl, heteroarylaminosulfonyl, and aralkylaminosulfonyl. Examples thereof additionally include compounds in which the H atom bonded to the N atom in -SO₂-NHR is further replaced with alkyl, cycloalkyl, alkenyl, alkynyl, aryl, heteroaryl, or aralkyl.

Examples of the sulfamoylamino (-NH-SO₂-NHR) include alkylsulfamoylamino, cycloalkylsulfamoylamino, alkenylsulfamoylamino, alkynylsulfamoylamino, arylsulfamoylamino, heteroarylsulfamoylamino, and aralkylsulfamoylamino. The two H atoms bonded to the N atoms in -NH-SO₂-NHR may be substituted by substituents each independently selected from the group consisting of alkyl, cycloalkyl, alkenyl, alkynyl, aryl, heteroaryl, and aralkyl, and these two substituents may form a ring.

Examples of the substituent containing a S atom include groups such as thiol (-SH), thio (-S-R), sulfinyl (-S(=O)-R), sulfonyl (-SO₂-R), and sulfo(-SO₃H).

Examples of thio (-S-R) that can be selected include alkylthio, cycloalkylthio, alkenylthio, alkynylthio, arylthio, heteroarylthio, and aralkylthio.

Examples of sulfinyl (-S(=O)-R) include alkylsulfinyl, cycloalkylsulfinyl, alkenylsulfinyl, alkynylsulfinyl, arylsulfinyl, heteroarylsulfinyl, and aralkylsulfinyl.

Examples of the sulfonyl (-SO₂-R) include alkylsulfonyl, cycloalkylsulfonyl, alkenylsulfonyl, alkynylsulfonyl, arylsulfonyl, heteroarylsulfonyl, and aralkylsulfonyl.

Examples of the substituent containing a N atom include groups such as azide (-N₃; also referred to as an "azide group"), cyano (-CN), primary amino (-NH₂), secondary amino (-NH-R; also referred to as mono-substituted amino), tertiary amino (-NR(R'); also referred to as di-substituted amino), amidino (-C(=NH)-NH₂), substituted amidino (-C(=NR)-NR'R"), guanidino (-NH-C(=NH)-NH₂), substituted guanidino (-NR-C(=NR‴)-NR'R"), and aminocarbonylamino (-NR-CO-NR'R").

Examples of the secondary amino (-NH-R: mono-substituted amino) include alkylamino, cycloalkylamino, alkenylamino, alkynylamino, arylamino, heteroarylamino, and aralkylamino.

Examples of the tertiary amino (-NR(R'): di-substituted amino) include an amino group having any two substituents each independently selected from alkyl, cycloalkyl, alkenyl, alkynyl, aryl, heteroaryl, and aralkyl, for example, alkyl(aralkyl)amino. These any two substituents may form a ring. Specific examples thereof include dialkylamino, particularly, C₁-C₆ dialkylamino, C₁-C₄ dialkylamino, dimethylamino, and diethylamino. The term "Cₚ-C_{q} dialkylamino group" as used herein refers to a group in which an amino group is substituted by two Cₚ-C_{q} alkyl groups. Both Cₚ-C_{q} alkyl groups may be the same or different.

Examples of the substituted amidino (-C(=NR)-NR'R") include groups in which three substituents R, R', and R" on the N atoms are each independently selected from alkyl, cycloalkyl, alkenyl, alkynyl, aryl, heteroaryl, and aralkyl, for example, alkyl(aralkyl)(aryl)amidino.

Examples of the substituted guanidino (-NR-C(=NR‴)-NR'R") include groups in which R,R', R", and R‴ are each independently selected from alkyl, cycloalkyl, alkenyl, alkynyl, aryl, heteroaryl, and aralkyl, or groups in which these form a ring.

Examples of the aminocarbonylamino (-NR-CO-NR'R") include groups in which R, R', and R" are each independently selected from a hydrogen atom, alkyl, cycloalkyl, alkenyl, alkynyl, aryl, heteroaryl, and aralkyl, or groups in which these form a ring.

Examples of the substituent containing a B atom include boryl (-BR(R')), and dioxyboryl (-B(OR)(OR')). The two substituents R and R' may be groups each independently selected from alkyl, cycloalkyl, alkenyl, alkynyl, aryl, heteroaryl, aralkyl, or the like, or may be groups in which these form a ring. Specific examples thereof include a cyclic boryl group, and more specifically include a pinacolate boryl group, a neopentanediolate boryl group, and a catecholate boryl group.

### [Composition]

The composition according to the present embodiments includes the following components (1) and/or (2).
(1) A compound represented by general formula (1), a pharmaceutically acceptable salt thereof, or a pharmaceutically acceptable solvate thereof. 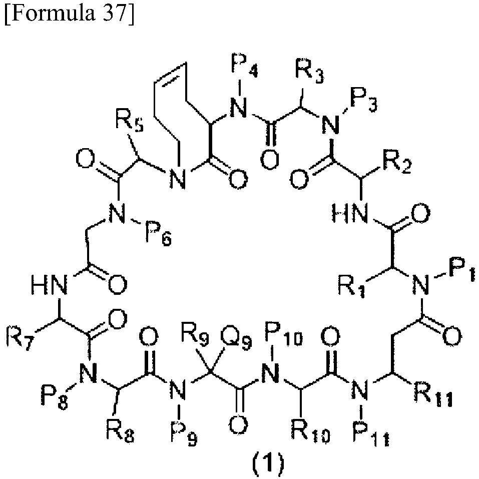 [wherein R₁ is C₁ to C₆ alkyl or C₃ to C₈ cycloalkyl C₁ to C₆ alkyl;
   P₁ is C₁ to C₆ alkyl;
   R₂ is C₁ to C₆ alkyl;
   R₃ is hydrogen, or forms a 4- to 7-membered saturated heterocyclic ring together with a carbon atom bonded to P₃ and R₃ and a nitrogen atom bonded to P₃;
   P₃ is C₁ to C₆ alkyl or C₃ to C₈ cycloalkyl except when R₃ and P₃ form a 4- to 7-membered saturated heterocyclic ring;
   P₄ is C₁ to C₆ alkyl;
   R₅ is benzyl optionally substituted by one or more groups selected from the group consisting of C₁ to C₆ alkyls, C₁ to C₆ haloalkyls, and C₃ to C₈ cycloalkyls;
   P₆ is C₁ to C₆ alkyl;
   R₇ is phenethyl optionally substituted by one or more groups selected from the group consisting of halogens, C₁ to C₆ haloalkyls, and C₁ to C₆ alkoxys;
   R₈ forms a 4- to 7-membered saturated heterocyclic ring together with a carbon atom bonded to P₈ and R₈ and a nitrogen atom bonded to P₈, the 4- to 7-membered saturated heterocyclic ring being optionally substituted by a C₁ to C₆ alkoxy;
   R₉ forms a 3- to 8-membered alicyclic ring together with Q₉ and a carbon atom bonded to R₉ and Q₉, the 3- to 8-membered alicyclic ring being optionally substituted by one or more C₁ to C₆ alkyls;
   P₉ is hydrogen or C₁ to C₆ alkyl;
   R₁₀ is C₁ to C₆ alkyl or C₃ to C₈ cycloalkyl;
   P₁₀ is C₁ to C₆ alkyl;
   R₁₁ is di-C₁ to C₆ alkylaminocarbonyl or 4- to 8-membered cyclic aminocarbonyl; and
   P₁₁ is C₁ to C₆ alkyl].
(2) Surfactant.

### [(1) Compound represented by general formula (1), pharmaceutically acceptable salt thereof, or pharmaceutically acceptable solvate thereof]

As used herein, the compound represented by general formula (1) is also referred to simply as a "compound" or a "cyclic peptide compound."

Examples of the pharmaceutically acceptable salt of the compound according to the present embodiments include: hydrochloride hydrobromide; hydroiodide; phosphate; phosphonate; sulfate; sulfonate such as methanesulfonate and p-toluenesulfonate; carboxylate such as acetate, citrate, malate, tartrate, succinate, and salicylate; or alkali metal salts such as sodium salt, and potassium salt; alkaline earth metal salts such as magnesium salt and calcium salt; and ammonium salts such as ammonium salt, alkylammonium salt, dialkylammonium salt, trialkylammonium salt, and tetraalkylammonium salt. These salts are produced by, for example, bringing the compound into contact with an acid or a base which can be used for production of a medicament. The solvate of the compound according to the present embodiments or a pharmaceutically acceptable salt thereof is one in which the compound or a pharmaceutically acceptable salt thereof and a solvent together form a molecular aggregate, which is called a hydrate when the solvent is water. The solvate of the compound according to the present embodiments or a pharmaceutically acceptable salt thereof is preferably a hydrate, and specific examples of the hydrate include mono- to decahydrate, preferably mono- to pentahydrate, further preferably mono- to trihydrate. The solvate of the compound according to the present embodiments or a pharmaceutically acceptable salt thereof includes not only solvates with a single solvent such as water, alcohol (e.g. methanol, ethanol, 1-propanol or 2-propanol), or dimethylformamide, but also solvates with a plurality of solvents.

In an aspect, R₁ is C₁ to C₆ alkyl or C₃ to C₈ cycloalkyl C₁ to C₆ alkyl in general formula (1). In this aspect, specific examples of R₁ include methyl, ethyl, n-propyl, i-propyl, n-butyl, s-butyl, t-butyl, isobutyl (2-methylpropyl), n-pentyl, s-pentyl (1-methylbutyl), t-pentyl (1,1-dimethylpropyl), neopentyl (2,2-dimethylpropyl), isopentyl (3-methylbutyl), 3-pentyl (1-ethylpropyl), 1,2-dimethylpropyl, 2-methylbutyl, n-hexyl, 1,1,2-trimethylpropyl, 1,2,2-trimethylpropyl, 1,1-dimethylbutyl, 1,2-dimethylbutyl, 1,3-dimethylbutyl, 2,2-dimethylbutyl, 2,3-dimethylbutyl, 3,3-dimethylbutyl, cyclopropylmethyl, cyclobutylmethyl, cyclopentylmethyl, and cyclohexylmethyl.

R₁ is preferably C₁ to C₄ alkyl or C₄ to C₆ cycloalkyl C₁ to C₃ alkyl, more preferably C₂ to C₄ alkyl or C₅ to C₆ cycloalkyl C₁ to C₂ alkyl, and further preferably ethyl, n-propyl, 2-methylpropyl, or cyclopentylmethyl.

In an aspect, P₁ is C₁ to C₆ alkyl in general formula (1). In this aspect, specific examples of P₁ include methyl, ethyl, n-propyl, i-propyl, n-butyl, s-butyl, t-butyl, isobutyl (2-methylpropyl), n-pentyl, s-pentyl (1-methylbutyl), t-pentyl (1,1-dimethylpropyl), neopentyl (2,2-dimethylpropyl), isopentyl (3-methylbutyl), 3-pentyl (1-ethylpropyl), 1,2-dimethylpropyl, 2-methylbutyl, n-hexyl, 1,1,2-trimethylpropyl, 1,2,2-trimethylpropyl, 1,1-dimethylbutyl, 1,2-dimethylbutyl, 1,3-dimethylbutyl, 2,2-dimethylbutyl, 2,3-dimethylbutyl, and 3,3-dimethylbutyl.

P₁ is preferably C₁ to C₃ alkyl, more preferably C₁ to C₂ alkyl, and further preferably methyl.

In an aspect, R₂ is C₁ to C₆ alkyl in general formula (1). In this aspect, specific examples of R₂ include methyl, ethyl, n-propyl, i-propyl, n-butyl, s-butyl, t-butyl, isobutyl (2-methylpropyl), n-pentyl, s-pentyl (1-methylbutyl), t-pentyl (1,1-dimethylpropyl), neopentyl (2,2-dimethylpropyl), isopentyl (3-methylbutyl), 3-pentyl (1-ethylpropyl), 1,2-dimethylpropyl, 2-methylbutyl, n-hexyl, 1,1,2-trimethylpropyl, 1,2,2-trimethylpropyl, 1,1-dimethylbutyl, 1,2-dimethylbutyl, 1,3-dimethylbutyl, 2,2-dimethylbutyl, 2,3-dimethylbutyl, and 3,3-dimethylbutyl.

R₂ is preferably C₃ to C₆ alkyl, more preferably C₄ to C₅ alkyl, and further preferably s-butyl (1-methylpropyl).

In an aspect, R₃ is hydrogen, or forms a 4- to 7-membered saturated heterocyclic ring together with a carbon atom bonded to P₃ and R₃ and a nitrogen atom bonded to P₃ in general formula (1). In this case, the 4- to 7-membered saturated heterocyclic ring is preferably a 4- to 6-membered saturated heterocyclic ring, and more preferably a 5-membered saturated heterocyclic ring. Specific examples of the 4- to 7-membered saturated heterocyclic ring include an azetidine ring, a pyrrolidine ring, a piperidine ring, a piperazine ring, and a morpholine ring, with a pyrrolidine ring being preferred.

In an aspect, P₃ is C₁ to C₆ alkyl or C₃ to C₈ cycloalkyl except when R₃ and P₃ form a 4-to 7-membered saturated heterocyclic ring, in general formula (1). When R₃ and P₃ form a 4- to 7-membered saturated heterocyclic ring, the 4- to 7-membered saturated heterocyclic ring is as described above. When P₃ is C₁ to C₆ alkyl or C₃ to C₈ cycloalkyl, specific examples thereof include methyl, ethyl, n-propyl, i-propyl, n-butyl, s-butyl, t-butyl, isobutyl (2-methylpropyl), n-pentyl, s-pentyl (1-methylbutyl), t-pentyl (1,1-dimethylpropyl), neopentyl (2,2-dimethylpropyl), isopentyl (3-methylbutyl), 3-pentyl (1-ethylpropyl), 1,2-dimethylpropyl, 2-methylbutyl, n-hexyl, 1,1,2-trimethylpropyl, 1,2,2-trimethylpropyl, 1,1-dimethylbutyl, 1,2-dimethylbutyl, 1,3-dimethylbutyl, 2,2-dimethylbutyl, 2,3-dimethylbutyl, 3,3-dimethylbutyl, cyclopropyl, cyclobutyl, cyclopentyl, cyclohexyl, cycloheptyl, cyclooctyl, bicyclo[2.2.1]heptyl, and spiro[3.3]heptyl.

When P₃ is C₁ to C₆ alkyl or C₃ to C₈ cycloalkyl, P₃ is preferably C₁ to C₃ alkyl or C₃ to C₆ cycloalkyl, more preferably C₁ to C₂ alkyl or C₃ to C₄ cycloalkyl, and further preferably methyl or cyclopropyl.

In an aspect, P₄ is C₁ to C₆ alkyl in general formula (1). In this aspect, specific examples of P₄ include methyl, ethyl, n-propyl, i-propyl, n-butyl, s-butyl, t-butyl, isobutyl (2-methylpropyl), n-pentyl, s-pentyl (1-methylbutyl), t-pentyl (1,1-dimethylpropyl), neopentyl (2,2-dimethylpropyl), isopentyl (3-methylbutyl), 3-pentyl (1-ethylpropyl), 1,2-dimethylpropyl, 2-methylbutyl, n-hexyl, 1,1,2-trimethylpropyl, 1,2,2-trimethylpropyl, 1,1-dimethylbutyl, 1,2-dimethylbutyl, 1,3-dimethylbutyl, 2,2-dimethylbutyl, 2,3-dimethylbutyl, and 3,3-dimethylbutyl.

P₄ is preferably C₁ to C₃ alkyl, more preferably C₁ to C₂ alkyl, and further preferably methyl.

In an aspect, R₅ is benzyl optionally substituted by one or more groups selected from the group consisting of C₁ to C₆ alkyls, C₁ to C₆ haloalkyls, and C₃ to C₈ cycloalkyls in general formula (1). When one or more substituents are present, the substituent is preferably one or more groups selected from the group consisting of C₁ to C₃ alkyls, C₁ to C₃ haloalkyls, and C₃ to C₆ cycloalkyls, more preferably one or more groups selected from the group consisting of C₁ to C₃ alkyls and C₁ to C₃ fluoroalkyls, and further preferably one or more groups selected from the group consisting of methyl and trifluoromethyl.

R₅ is preferably benzyl optionally substituted by one or more groups selected from the group consisting of C₁ to C₃ alkyls, C₁ to C₃ haloalkyls, and C₃ to C₆ cycloalkyls, more preferably benzyl optionally substituted by one or more groups selected from the group consisting of C₁ to C₃ alkyls and C₁ to C₃ fluoroalkyls, further preferably benzyl optionally substituted by one or more groups selected from the group consisting of methyl and trifluoromethyl, and particularly preferably 4-methylbenzyl and 4-trifluoromethylbenzyl.

In an aspect, P₆ is C₁ to C₆ alkyl in general formula (1). In this aspect, specific examples of P₆ include methyl, ethyl, n-propyl, i-propyl, n-butyl, s-butyl, t-butyl, isobutyl (2-methylpropyl), n-pentyl, s-pentyl (1-methylbutyl), t-pentyl (1,1-dimethylpropyl), neopentyl (2,2-dimethylpropyl), isopentyl (3-methylbutyl), 3-pentyl (1-ethylpropyl), 1,2-dimethylpropyl, 2-methylbutyl, n-hexyl, 1,1,2-trimethylpropyl, 1,2,2-trimethylpropyl, 1,1-dimethylbutyl, 1,2-dimethylbutyl, 1,3-dimethylbutyl, 2,2-dimethylbutyl, 2,3-dimethylbutyl, and 3,3-dimethylbutyl.

P₆ is preferably C₁ to C₃ alkyl, more preferably C₁ to C₂ alkyl, and further preferably methyl.

In an aspect, R₇ is phenethyl optionally substituted by one or more groups selected from the group consisting of halogens, C₁ to C₆ haloalkyls, and C₁ to C₆ alkoxys in general formula (1). When one or more substituents are present, the substituent is preferably one or more groups selected from the group consisting of halogens, C₁ to C₃ haloalkyls, and C₁ to C₃ alkoxys, more preferably one or more groups selected from the group consisting of F, C₁ to C₃ fluoroalkyls, and C₁ to C₃ alkoxys, and further preferably one or more groups selected from the group consisting of F, trifluoromethyl, and methoxy.

R₇ is preferably phenethyl optionally substituted by one or more groups selected from the group consisting of halogens, C₁ to C₃ haloalkyls, and C₁ to C₃ alkoxys, more preferably phenethyl optionally substituted by one or more groups selected from the group consisting of F, C₁ to C₃ fluoroalkyls, and C₁ to C₃ alkoxys, further preferably phenethyl optionally substituted by one or more groups selected from the group consisting of F, trifluoromethyl, and methoxy, and particularly preferably 3,5-difluoro-4-trifluoromethylphenethyl, and 3-methoxy-4-trifluoromethylphenethyl.

In an aspect, R₈ forms a 4- to 7-membered saturated heterocyclic ring together with a carbon atom bonded to P₈ and R₈ and a nitrogen atom bonded to P₈ in general formula (1). In this case, the 4- to 7-membered saturated heterocyclic ring is preferably a 4- to 6-membered saturated heterocyclic ring, and more preferably a 5-membered saturated heterocyclic ring. Specific examples of the 4- to 7-membered saturated heterocyclic ring include an azetidine ring, a pyrrolidine ring, a piperidine ring, a piperazine ring, and a morpholine ring, with a pyrrolidine ring being preferred. The 4- to 7-membered saturated heterocyclic ring is optionally substituted by a C₁ to C₆ alkoxy. When substituted, the substituent is preferably C₁ to C₃ alkoxy, more preferably C₁ to C₂ alkoxy, and further preferably ethoxy.

In an aspect, R₉ forms a 3- to 8-membered alicyclic ring together with Q₉ and a carbon atom bonded to R₉ and Q₉ in general formula (1). In this case, the 3- to 8-membered alicyclic ring is preferably a 4- to 6-membered alicyclic ring, and more preferably a 4- to 5-membered alicyclic ring. Specific examples of the 3- to 8-membered alicyclic ring include a cyclopropane ring, a cyclobutane ring, a cyclopentane ring, and a cyclohexane ring, with a cyclobutane ring and a cyclopentane ring being preferred. The 3- to 8-membered alicyclic ring is optionally substituted by one or more C₁ to C₆ alkyls. When substituted, the substituent is preferably C₁ to C₃ alkyl, more preferably C₁ to C₂ alkyl, and further preferably methyl.

In an aspect, P₉ is hydrogen or C₁ to C₆ alkyl in general formula (1). In this aspect, specific examples of P₉ include hydrogen, methyl, ethyl, n-propyl, i-propyl, n-butyl, s-butyl, t-butyl, isobutyl (2-methylpropyl), n-pentyl, s-pentyl (1-methylbutyl), t-pentyl (1,1-dimethylpropyl), neopentyl (2,2-dimethylpropyl), isopentyl (3-methylbutyl), 3-pentyl (1-ethylpropyl), 1,2-dimethylpropyl, 2-methylbutyl, n-hexyl, 1,1,2-trimethylpropyl, 1,2,2-trimethylpropyl, 1,1-dimethylbutyl, 1,2-dimethylbutyl, 1,3-dimethylbutyl, 2,2-dimethylbutyl, 2,3-dimethylbutyl, and 3,3-dimethylbutyl.

P₉ is preferably hydrogen or C₁ to C₃ alkyl, more preferably hydrogen or C₁ to C₂ alkyl, and further preferably hydrogen or methyl.

In an aspect, R₁₀ is C₁ to C₆ alkyl or C₃ to C₈ cycloalkyl in general formula (1). In this aspect, specific examples of R₁₀ include methyl, ethyl, n-propyl, i-propyl, n-butyl, s-butyl, t-butyl, isobutyl (2-methylpropyl), n-pentyl, s-pentyl (1-methylbutyl), t-pentyl (1,1-dimethylpropyl), neopentyl (2,2-dimethylpropyl), isopentyl (3-methylbutyl), 3-pentyl (1-ethylpropyl), 1,2-dimethylpropyl, 2-methylbutyl, n-hexyl, 1,1,2-trimethylpropyl, 1,2,2-trimethylpropyl, 1,1-dimethylbutyl, 1,2-dimethylbutyl, 1,3-dimethylbutyl, 2,2-dimethylbutyl, 2,3-dimethylbutyl, 3,3-dimethylbutyl, cyclopropyl, cyclobutyl, cyclopentyl, cyclohexyl, cycloheptyl, cyclooctyl, bicyclo[2.2.1]heptyl, and spiro[3.3]heptyl.

R₁₀ is preferably C₃ to C₆ alkyl or C₄ to C₆ cycloalkyl, more preferably C₄ to C₆ cycloalkyl, and further preferably cyclopentyl.

In an aspect, P₁₀ is C₁ to C₆ alkyl in general formula (1). In this aspect, specific examples of P₁₀ include methyl, ethyl, n-propyl, i-propyl, n-butyl, s-butyl, t-butyl, isobutyl (2-methylpropyl), n-pentyl, s-pentyl (1-methylbutyl), t-pentyl (1,1-dimethylpropyl), neopentyl (2,2-dimethylpropyl), isopentyl (3-methylbutyl), 3-pentyl (1-ethylpropyl), 1,2-dimethylpropyl, 2-methylbutyl, n-hexyl, 1,1,2-trimethylpropyl, 1,2,2-trimethylpropyl, 1,1-dimethylbutyl, 1,2-dimethylbutyl, 1,3-dimethylbutyl, 2,2-dimethylbutyl, 2,3-dimethylbutyl, and 3,3-dimethylbutyl.

P₁₀ is preferably C₁ to C₃ alkyl, more preferably C₁ to C₂ alkyl, and further preferably methyl.

In an aspect, R₁₁ is di-C₁ to C₆ alkylaminocarbonyl or 4- to 8-membered cyclic aminocarbonyl in general formula (1). In this aspect, specific examples of R₁₁ include dimethylaminocarbonyl, 1-azetidinylcarbonyl, 1-pyrrolidinylcarbonyl, 1-piperidinylcarbonyl, 1-piperazinylcarbonyl, 4-morpholinylcarbonyl, 3-oxazolidinylcarbonyl, 1,1-dioxidothiomorpholinyl-4-ylcarbonyl, and 3-oxa-8-azabicyclo[3.2.1]octane-8-ylcarbonyl.

R₁₁ is preferably di-C₁ to C₃ alkylaminocarbonyl or 4- to 6-membered cyclic aminocarbonyl, more preferably di-C₁ to C₃ alkylaminocarbonyl, and further preferably dimethylaminocarbonyl.

In an aspect, P₁₁ is C₁ to C₆ alkyl in general formula (1). In this aspect, specific examples of P₁₁ include methyl, ethyl, n-propyl, i-propyl, n-butyl, s-butyl, t-butyl, isobutyl (2-methylpropyl), n-pentyl, s-pentyl (1-methylbutyl), t-pentyl (1,1-dimethylpropyl), neopentyl (2,2-dimethylpropyl), isopentyl (3-methylbutyl), 3-pentyl (1-ethylpropyl), 1,2-dimethylpropyl, 2-methylbutyl, n-hexyl, 1,1,2-trimethylpropyl, 1,2,2-trimethylpropyl, 1,1-dimethylbutyl, 1,2-dimethylbutyl, 1,3-dimethylbutyl, 2,2-dimethylbutyl, 2,3-dimethylbutyl, and 3,3-dimethylbutyl.

P₁₁ is preferably C₁ to C₃ alkyl, more preferably C₁ to C₂ alkyl, and further preferably methyl.

Specific examples of the compound according to the present embodiments include those represented by formula (1a), formula (1b), formula (1c), formula (1d), formula (1e), and formula (1f). The compounds represented by formula (1a), formula (1b), formula (1c), formula (1d), formula (1e), and formula (1f) correspond to the compounds CP05, CP01, CP02, CP03, CP04 and CP06 in the Examples, respectively.

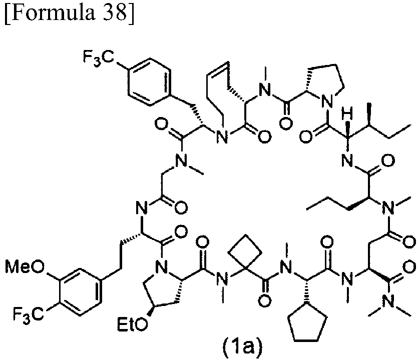

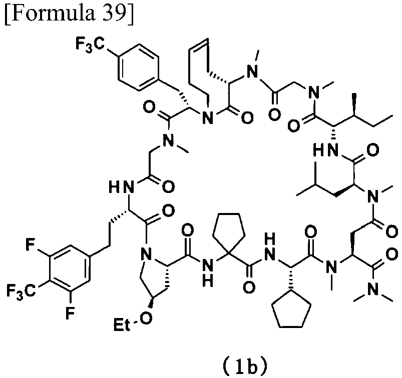

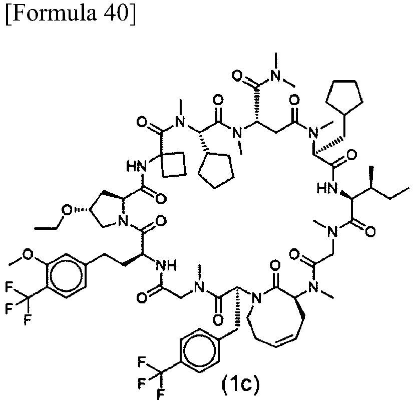

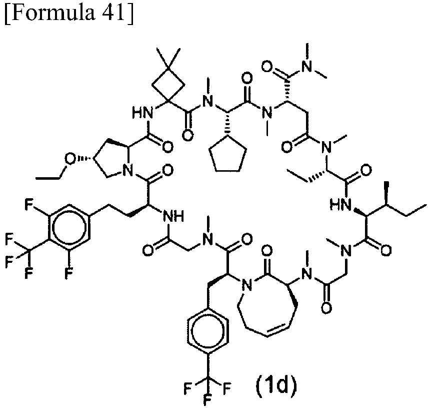

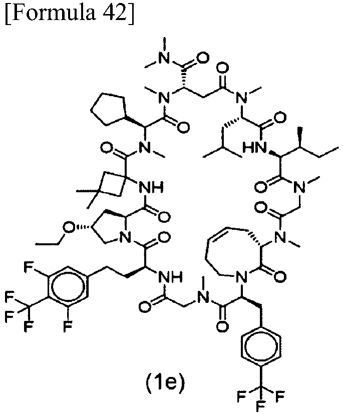

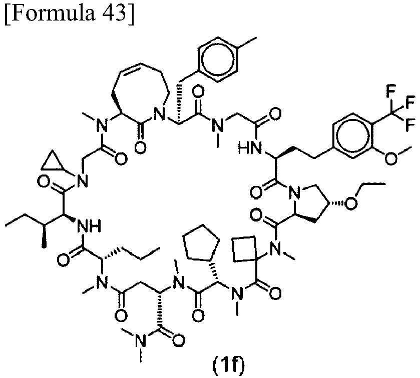

The cyclic peptide compound according to the present embodiments can be synthesized, for example, according to the method described in Patent Literature 3.

The value of Caco-2 Papp (cm/sec) of the cyclic peptide compound according to the present embodiments, as measured in a system in which the surfactant according to the present embodiments is not present, is preferably 1.0E-5 or less, 9.0E-6 or less, 8.0E-6 or less, 7.0E-6 or less, 6.0E-6 or less, 5.0E-6 or less, 4.0E-6 or less, 3.0E-6 or less, and more preferably 2.0E-6 or less, 1.8E-6 or less, 1.6E-6 or less, 1.4E-6 or less, 1.2E-6 or less, 1.0E-6 or less, 9.8E-7 or less, 9.6E-7 or less, 9.4E-7 or less, 9.2E-7 or less, 9.0E-7 or less, 8.8E-7 or less, 8.6E-7 or less, 8.4E-7 or less, 8.2E-7 or less, 8.0E-7 or less, 7.8E-7 or less, 7.6E-7 or less, 7.4E-7 or less, 7.2E-7 or less, 7.0E-7 or less, 6.8E-7 or less, 6.6E-7 or less, 6.4E-7 or less, 6.2E-7 or less, 6.0E-7 or less, 5.8E-7 or less, 5.6E-7 or less, 5.4E-7 or less, 5.2E-7 or less, 5.0E-7 or less, 4.8E-7 or less, 4.6E-7 or less, 4.4E-7 or less, 4.2E-7 or less, 4.0E-7 or less, 3.8E-7 or less, 3.6E-7 or less, 3.4E-7 or less, 3.2E-7 or less, 3.0E-7 or less, 2.8E-7 or less, 2.6E-7 or less, 2.4E-7 or less, 2.2E-7 or less, 2.0E-7 or less, 1.8E-7 or less, 1.6E-7 or less, 1.4E-7 or less, 1.2E-7 or less, or 1.0E-7 or less. It should be noted that E-n (n is a natural number) means 10⁻ⁿ (for example, 1.0E-5 = 1.0 × 10⁻⁵).

The value of Caco-2 Papp (cm/sec) of the cyclic peptide compound according to the present embodiments, as measured in the system in which the surfactant according to the present embodiments is present, is preferably 1.0E-9 or more, 1.0E-8 or more, 2.0E-8 or more, 3.0E-8 or more, 4.0E-8 or more, 5.0E-8 or more, 6.0E-8 or more, 7.0E-8 or more, 8.0E-8 or more, 9.0E-8 or more, 1.0E-7 or more, 1.1E-7 or more, 1.2E-7 or more, 1.3E-7 or more, 1.4E-7 or more, 1.5E-7 or more, 1.6E-7 or more, 1.7E-7 or more, 1.8E-7 or more, 1.9E-7 or more, or 2.0E-7 or more.

The value of Caco-2 Papp (cm/sec) of the cyclic peptide compound according to the present embodiments, as measured in the system in which the surfactant according to the present embodiments is present, is preferably 1.1-fold or more, more preferably 1.2-fold or more, further preferably 1.5-fold or more, further preferably 2-fold or more, further preferably 3-fold or more, further preferably 5-fold or more the value as measured in the system in which the surfactant is not present.

The value of Caco-2 Papp (cm/sec) is a value that serves as an indicator of the membrane permeability in the cell membrane, and can be measured by the method below.
(i) Caco-2 cells are cultured on a plate (e.g., 96-well Transwell and Falcon(R)96) for 3 weeks, and then the composition to be evaluated and a FaSSIF/HBSS buffer (pH 6.5) are added to the apical side, and an HBSS buffer (pH 7.4) containing 4% BSA is added to the basal side (the start of the permeability study).
(ii) Each well is shaken at 80 rpm in 5%CO₂ at 37°C, and 180 minutes after the start, a sample on the basal side is taken and the permeation amount of the cyclic peptide compound is measured by liquid chromatography-mass spectrometry (LC/MS/MS).
(iii) From the measured permeation amount, the permeability coefficient (Caco-2 Papp (cm/sec)) is calculated.

In the above measurements, a Pgp inhibitor, such as Zosuquidar, can be added to the FaSSIF/HBSS buffer and the HBSS buffer, respectively.

After the above step (i) and before step (ii), pre-incubation can be performed by leaving each well standing at 5% CO₂, 37°C, and 80 rpm for 20 to 24 hours.

After pre-incubation, the solution on the basal side can be removed and washed, and a new solution of the same composition can be added. A Pgp inhibitor can also be added to the solution. When the pre-incubation is performed, it is preferable that a DMEM solution (pH 7.4) containing 4% BSA is used instead of the HBSS buffer (pH 7.4).

In addition, as the concentration of the substance to be evaluated on the donor side used in the calculation of the permeability coefficient in the above step (iii), the concentration when initially added can be used, or the concentration measured by collecting the solution at the apical side before the start of pre-incubation or the start of shaking in the above step (ii) can be also used. In particular, when the preincubation is performed, it is preferable to use the concentration of the solution at the apical side collected before pre-incubation.

Specifically, the concentration can be measured by the method described in Examples.

In this manner, when measuring Caco-2 Papp (cm/sec) of the composition according to the present embodiments, the substance to be measured is a cyclic peptide compound contained in the composition.

The value of the bioavailability (BA) of the cyclic peptide compound according to the present embodiments, as measured in the system in which the surfactant according to the present embodiments is present, is preferably 1.1-fold or more, more preferably 1.2-fold or more, further preferably 1.4-fold or more, further preferably 1.6-fold or more, further preferably 1.8-fold or more, further preferably 2.0-fold or more the value as measured in the system in which the surfactant is not present.

The value of the bioavailability (BA) can be measured, for example, by the following method. The composition to be evaluated is administered to test animals, and blood is collected over time from the jugular vein using a syringe up to 24 hours after administration. The blood is dispensed into tubes treated with heparin as an anticoagulant, and centrifuged to separate plasma. After deproteinization with acetonitrile, the plasma concentration of the cyclic peptide compound is measured with an LC/MS/MS device (e.g., XEVO TQ-XS, manufactured by Waters Corporation). From the resulting changes in plasma concentration, the pharmacokinetic parameters are calculated by non-compartmental analysis using analysis software Phoenix WinNonlin 8.2 (manufactured by Certara L.P.).

### [(2) Surfactant]

The surfactant according to the present embodiments is preferably at least one or more selected from the group consisting of (A) a surfactant having a linear alkylene structure, a linear alkenylene structure, or a linear alkynylene structure, and having 5 or more and 13 or less carbon atoms in the structure, and (B) a surfactant having a carnitine residue. The surfactant may be used singly or in combination of two or more. The specific examples of the surfactant described below may be used in the form of a salt (e.g., hydrochloride salt and sodium salt).

The surfactant according to one embodiment may be a component that promotes the emulsification and dispersion of the cyclic peptide compound, a component that promotes absorption via the transcellular pathway, or a component that promotes absorption via the paracellular pathway.

The surfactant according to one embodiment has a linear alkylene structure, a linear alkenylene structure, or a linear alkynylene structure, and the number of carbon atoms contained in the structure is 5 or more and 13 or less. The number of carbon atoms is preferably 6 or more, more preferably 8 or more, further preferably 10 or more, and particularly preferably 11. The number of carbon atoms contained in the linear alkylene structure may be 6 or more and 13 or less, preferably 8 or more and 12 or less, more preferably 10 or more and 12 or less, and particularly preferably 11.

Specific examples of the surfactant are shown below (the number within the parentheses represents the number of carbon atoms in the linear alkylene structure).
(a) Caproic acid (5)
(b) Caprylic acid (7)
(c) Capric acid (9)
(d) Lauric acid (11)
(e) Lauroylcarnitine (11)
(f) Lauroyl-L-carnitine (11)
(g) Carnitine palmitate (15)

The surfactant according to one embodiment is preferably a compound represented by any one of the following general formulae (a1) to (a3):

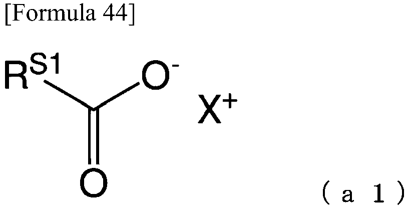

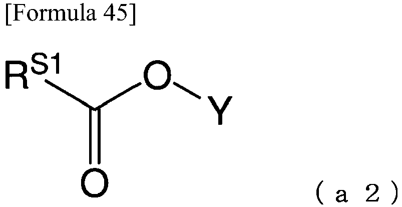

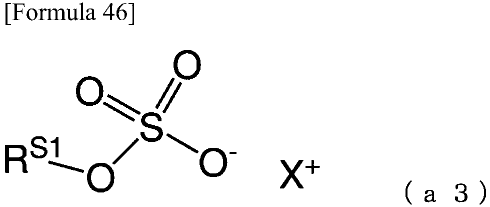

In general formulae (a1) to (a3), R^{S1} represents a saturated or unsaturated, linear hydrocarbon group having 5 or more and 13 or less carbon atoms which may have a substituent, X represents sodium or potassium, and Y represents a group represented by the following formula (a4) or a stereoisomer thereof.

The "hydrocarbon group" refers to "alkyl," "alkenyl," and "alkynyl." The saturated hydrocarbon group is alkyl and the unsaturated hydrocarbon group is alkenyl or alkynyl.

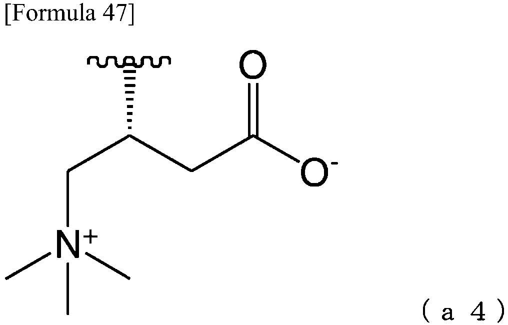

In the general formulae (a1) to (a3), R^{S1} is preferably an alkyl group having 5 or more and 13 or less carbon atoms, more preferably an alkyl group having 7 or more and 12 or less carbon atoms, further preferably an alkyl group having 8 or more and 12 or less carbon atoms, further more preferably an alkyl group having 10 or more and 12 or less carbon atoms, and particularly preferably an alkyl group having 11 carbon atoms. R^{S1} is also preferably a linear alkyl group. And R^{S1} is also preferably an unsubstituted alkyl group. In formula (a4), 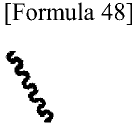 represents a bond.

The surfactant according to one embodiment also contains a medium-chain fatty acid structure. The medium-chain fatty acid refers to a fatty acid having 6 or more and 12 or less carbon atoms. The number of carbon atoms in the medium-chain fatty acid structure is more preferably 8 or more, further preferably 10 or more, and particularly preferably 12.

The surfactant according to one embodiment may be a medium-chain fatty acid ester, a sodium salt of medium-chain fatty acid, or a potassium salt of medium-chain fatty acid. The medium-chain fatty acid ester is a compound in which an ester bond is formed between a carboxy group of a medium-chain fatty acid and a hydroxy group of a hydroxy group-containing compound. Examples of the medium-chain fatty acid include, but are not limited to, caproic acid, enanthic acid, caprylic acid, pelargonic acid, capric acid, undecylic acid, and lauric acid, and among them, more preferably caprylic acid, capric acid, and lauric acid, and further preferably lauric acid. Examples of the hydroxy group-containing compound include, but are not limited to, aliphatic alcohol, polyhydric alcohol, and hydroxy group-containing betaine, such as carnitine, trimethylglycine, and proline betaine.

The surfactant according to one embodiment is preferably acylcarnitine, further preferably lauroylcarnitine or carnitine palmitate, further preferably lauroylcarnitine, and particularly preferably lauroyl-L-camitine.

The surfactant according to one embodiment has a carnitine residue. The surfactant having a carnitine residue may preferably be acylcamitine. Acylcarnitine is a compound in which an ester bond is formed between a hydroxy group of carnitine and a carboxy group of a carboxy group-containing compound. The carnitine may be in a D-form or an L-form. The carboxy group-containing compound may be an organic acid, preferably a medium-chain fatty acid such as a saturated fatty acid or an unsaturated fatty acid, more preferably a saturated fatty acid. The number of carbon atoms of the medium-chain fatty acid is preferably 6 or more, more preferably 8 or more, further preferably 10 or more, and particularly preferably 12. The number of carbon atoms of the saturated fatty acid is preferably 6 or more, more preferably 8 or more, further preferably 10 or more, and particularly preferably 12. Examples of the saturated fatty acid according to the present invention include caproic acid, caprylic acid, capric acid, and lauric acid. The acylcamitine according to the present invention is more preferably lauroylcarnitine or carnitine palmitate, further preferably lauroylcarnitine, and particularly preferably lauroyl-L-carnitine.

The surfactant according to one embodiment may be an alkyl carboxylate or an alkyl sulfate. The alkyl carboxylate is preferably a caprylate, and more preferably sodium caprylate. The alkyl sulfate is preferably a lauryl sulfate, and more preferably sodium lauryl sulfate.

Examples of the surfactant according to the present embodiments preferably include a surfactant selected from the group consisting of lauroyl-L-carnitine, sodium caprylate, sodium lauryl sulfate, and sodium N-(8-[2-hydroxybenzoyl]amino)caprylate.

The surfactant according to one embodiment is added to the composition according to the present embodiments as an isolated component.

### [Solubility improver]

The composition according to the present embodiments may further include a solubility improver that improves the solubility of a cyclic peptide compound. Examples of the component that improves the solubility of a cyclic peptide compound include various oily components, a polymer that forms a solid dispersion (Amorphous Solid Dispersion, hereinafter also referred to as "ASD") with a cyclic peptide compound, and a component that adjusts pH. The solubility improver may be used singly or in combination of two or more.

Preferred specific examples of the oily component can include fatty acids such as oleic acid, stearic acid, linoleic acid, palmitic acid, linolenic acid, and myristic acid, olive oil, almond oil, coconut oil, cocoa butter, macadamia nut oil, avocado oil, safflower oil, soybean oil, flaxseed oil, rapeseed oil, castor oil, palm oil, high oleic sunflower oil, high oleic safflower oil, sunflower oil, cottonseed oil, corn oil, sesame oil, peanut oil, apricot kernel oil, candlenut oil, grape seed oil, pistachio seed oil, sunflower oil, hazelnut oil, jojoba oil, meadowfoam oil, rosehip oil, tricaproin, tricaprylin, tricaprin, tripalmitolein, triolein, trilinolein, trilinolenin, trieicosenoin, and trierucin. Examples of the oily component include, in addition to those listed above, vegetable oils collected from plants, those partially decomposed by a hydrolysis treatment thereof, and those separated and purified. The oily component may also be obtained by synthesizing using a synthesis method.

Regarding the oily component, it is further preferable to use a compound having a polyoxyethylene structure added to the oily component as a solubility improver. The polyoxyethylene structure is represented by -(CH₂-CH₂-O)ₙ-. In this compound, the average number of moles of ethylene oxide added is preferably 2 or more and 100 or less, more preferably 3 or more and 80 or less, further preferably 3 or more and 60 or less, and further more preferably 3 or more and 50 or less. The average number of moles of ethylene oxide added is also preferably 5 or more and 40 or less, more preferably 10 or more and 40 or less, further preferably 20 or more and 40 or less, and further more preferably 30 or more and 40 or less.

Specific examples of the compound having the polyoxyethylene structure added include polyoxyethylene castor oil, polyoxyethylene hydrogenated castor oil, and a polyoxyethylene sorbitan fatty acid ester. Among these, polyoxyethylene castor oil, polyoxyethylene hydrogenated castor oil, and polyoxyethylene sorbitan fatty acid esters are preferred, polyoxyethylene castor oil and polyoxyethylene sorbitan fatty acid esters are more preferred, polyoxyethylene castor oil with an average number of moles of ethylene oxide added of 30 or more and 40 or less and polyoxyethylene sorbitan fatty acid esters with an average number of moles of ethylene oxide added of 10 or more and 40 or less are further more preferred, and polyoxyethylene castor oil 35 (e.g., Cremophor EL) and polyoxyethylene (20) sorbitan monooleate (Tween 80) are still more preferred.

Examples of the polymer that forms ASD with a cyclic peptide compound include polyethylene glycol, polyvinylpyrrolidone, copovidone, polyvinyl alcohol, a cellulose-based polymer, and a methacrylic acid methacrylic acid copolymer. Specific examples thereof include a vinyl polymer and copolymer having at least one substituent selected from hydroxyl, alkylacyloxy, and a group containing a cyclic amide; a vinyl copolymer of at least one hydrophilic hydroxyl-containing repeating unit and at least one hydrophobic alkyl- or aryl-containing repeating unit; polyvinyl alcohol; polyvinyl alcohol having at least a portion of repeating units of a non-hydrolyzed (vinyl acetate) form; a polyvinyl alcohol polyvinyl acetate copolymer; polyvinyl pyrrolidone; copovidone; a copolymer of acrylate and methacrylic acid; a polyethylene polyvinyl alcohol copolymer; a polyoxyethylene-polyoxypropylene block copolymer (also called as poloxamer), polyethylene glycol, hydroxypropyl methyl cellulose acetate (HPMCA), hydroxypropyl methyl cellulose (HPMC), hydroxypropyl cellulose (HPC), methylcellulose, hydroxyethyl methyl cellulose, hydroxyethyl cellulose, hydroxyethyl cellulose acetate, hydroxyethyl ethyl cellulose, cellulose acetate phthalate, cellulose acetate trimellitate, cellulose acetate succinate, methyl cellulose phthalate, hydroxymethyl cellulose ethyl phthalate, hydroxypropyl methyl cellulose phthalate, hydroxypropyl methyl cellulose acetate succinate

(HPMCAS), hydroxypropyl methyl acetate maleate, hydroxypropyl methyl trimellitate, carboxymethyl ethyl cellulose, polyvinyl butylate phthalate, polyvinyl alcohol acetate phthalate, a methacrylic acid/ethyl acrylate copolymer (preferably in a mass ratio of 1 : 99 to 99 : 1), a methacrylic acid/methyl methacrylate copolymer (preferably in a mass ratio of 1 : 99 to 99 : 1), a methacrylic acid copolymer, aminoalkyl methacrylic acid copolymer E, and polyvinyl acetal diethyl aminoacetate.

Specific examples of the pH adjusting agent include lactic acid, succinic acid, gluconic acid, citric acid, citric acid hydrate, trisodium citrate, phosphoric acid, potassium carbonate, sodium hydrogen carbonate, tartaric acid, malic acid, ascorbic acid, fumaric acid, aspartic acid, glutamic acid, glutamic acid hydrochloride, malonic acid, maleic acid, meglumine, arginine, lysine, glycine, sodium carbonate, and sodium hydrogen phosphate.

### [Composition]

Regarding the contents of (1) a compound represented by general formula (1), a pharmaceutically acceptable salt thereof, or a pharmaceutically acceptable solvate thereof (hereinafter also referred to as "component (1)"), and (2) a surfactant (hereinafter also referred to as "component (2)") in the composition according to the present embodiments, the component (2) may be 0.05 parts by mass or more, 0.075 parts by mass or more, 0.1 parts by mass or more, 0.2 parts by mass or more, or 0.3 parts by mass or more based on 1 part by mass of the component (1). In addition, the component (2) may be 300 parts by mass or less, 200 parts by mass or less, 150 parts by mass or less, 100 parts by mass or less, 80 parts by mass or less, 60 parts by mass or less, 40 parts by mass or less, or 30 parts by mass or less based on 1 part by mass of the component (1). Furthermore, the content of the component (2) may be 0.05 parts by mass or more and 300 parts by mass or less, 0.05 parts by mass or more and 200 parts by mass or less, 0.05 parts by mass or more and 150 parts by mass or less, 0.05 parts by mass or more and 100 parts by mass or less, 0.075 parts by mass or more and 80 parts by mass or less, 0.1 parts by mass or more and 60 parts by mass or less, 0.2 parts by mass or more and 40 parts by mass or less, or 0.3 parts by mass or more and 30 parts by mass or less based on 1 part by mass of the component (1). When the component (1) contains two or more cyclic peptide compounds or the like, the above range is to the total amount thereof. The same holds for the component (2). The contents of the component (1) and the component (2) in the composition can be measured by liquid chromatography-mass spectrometry (LC-MS), liquid chromatography with charged aerosol detection, or a nuclear magnetic resonance device (NMR).

In addition, when the component (2) is a liquid at 25°C, the content of the component (2) based on 100% by volume of the liquid component contained in the composition including the component (2) itself is preferably 0.05% by volume or more, more preferably 0.075% by volume or more, further preferably 0.1% by volume or more, further preferably 0.2% by volume or more, further preferably 0.3% by volume or more, further preferably 0.5% by volume or more, further preferably 0.8% by volume or more, and further preferably 1.0% by volume or more. The content of the component (2) is also preferably 100% by volume or less, more preferably 85% by volume or less, further preferably 50% by volume or less, further preferably 40% by volume or less, further preferably 30% by volume or less, further preferably 20% by volume or less, further preferably 15% by volume or less, and further preferably 10% by volume or less. The content of the component (2) is also preferably 0.05% by volume or more and 50% by volume or less, more preferably 0.3% by volume or more and 30% by volume or less, further preferably 0.5% by volume or more and 15% by volume or less, and further preferably 0.8% by volume or more and 10% by volume or less.

When the composition according to the present embodiments contains (3) the solubility improver (hereinafter also referred to as "component (3)"), regarding the contents of the component (1) and the component (3) in the composition, the component (3) may be 0.1 parts by mass or more, 0.2 parts by mass or more, 0.3 parts by mass or more, 0.4 parts by mass or more, 0.5 parts by mass or more, 1 part by mass or more, 3 parts by mass or more, 4 parts by mass or more, 5 parts by mass or more, 6 parts by mass or more, or 7 parts by mass or more based on 1 part by mass of the component (1). Also, the component (3) may be 100 parts by mass or less, 80 parts by mass or less, 60 parts by mass or less, 40 parts by mass or less, and 20 parts by mass or less. In addition, the content of the component (3) may be 0.1 parts by mass or more and 100 parts by mass or less, 5 parts by mass or more and 60 parts by mass or less, 6 parts by mass or more and 40 parts by mass or less, or 7 parts by mass or more and 20 parts by mass or less based on 1 part by mass of the component (1). When the component (1) contains two or more cyclic peptide compounds or the like, the above range is to the total amount thereof. The same holds for the component (3).

In addition, when the component (3) is a liquid at 25°C, the content of the component (3) based on 100% by volume of the liquid component contained in the composition including the component (3) itself is preferably 0.05% by volume or more, more preferably 0.075% by volume or more, further preferably 0.1% by volume or more, further preferably 0.2% by volume or more, further preferably 0.3% by volume or more, further preferably 0.5% by volume or more, and further preferably 1.0% by volume or more. The content of the component (3) is also preferably 100% by volume or less, more preferably 85% by volume or less, further preferably 50% by volume or less, further preferably 40% by volume or less, further preferably 30% by volume or less, further preferably 20% by volume or less, further preferably 15% by volume or less, and further preferably 10% by volume or less. The content of the component (3) is also preferably 0.05% by volume or more and 50% by volume or less, more preferably 0.3% by volume or more and 30% by volume or less, further preferably 0.5% by volume or more and 15% by volume or less, and further preferably 0.8% by volume or more and 10% by volume or less.

The content of the component (1) in the composition according to the present embodiments may be appropriately set according to the type of the cyclic peptide compound, the application of the composition, and the like. Examples of the content of the component (1) in the composition according to the present embodiments are, but are not limited to, 0.01 mg/ml or more and 300 mg/ml or less, 0.03 mg/ml or more and 200 mg/ml or less, 0.1 mg/ml or more and 100 mg/ml or less, 0.3 mg/ml or more and 50 mg/ml or less, 1 mg/ml or more and 25 mg/ml or less, and 3 mg/ml or more and 10 mg/ml or less per 1 ml of the liquid component contained in the composition according to the present embodiments.

The composition according to the present embodiments can also include a component in a pre-mix state of (2) a surfactant and (3) a solubility improver, such as a Self Emulsifying Drug Delivery System (hereinafter referred to as "SEDDS").

The composition according to the present embodiments may contain a pharmaceutically acceptable carrier. Examples of the carrier include saline, buffered saline, water, an isotonic aqueous buffer, and combinations of these.

The composition according to the present embodiments may contain other pharmaceutically acceptable components to the extent that the effect according to the present invention is not impaired. Examples of the other components include an excipient, a disintegrant, a fluidizer/lubricant, a flavor modifier, a stabilizer, a preservative, an antioxidant, and a binder.

Examples of the excipient include lactose, corn starch, white sugar, glucose, D-mannitol, sorbit, starch, crystalline cellulose, silicon dioxide, and magnesium aluminometasilicate.

Examples of the disintegrant include starch, pregelatinized starch, agar, gelatin powder, crystalline cellulose, calcium carbonate, sodium chloride, sodium hydrogen carbonate, calcium citrate, anhydrous silicic acid, dextrin, pectin, carmellose, calcium carmellose, sodium croscarmellose, crospopidone, low-substituted hydroxypropyl cellulose, and sodium starch glycolate.

Examples of the fluidizer/lubricant include light anhydrous silicic acid, aqueous silicic acid dioxide, magnesium stearate, and talc.

Examples of the flavor modifier include cocoa powder, menthol, aromatic powder, mint oil, borneol, and cinnamon powder.

Examples of the stabilizer include phosphatidic acid, ascorbic acid, glycerin, and cetanol.

Examples of the preservative include ethyl paraoxybenzoate and propyl paraoxybenzoate.

Examples of the antioxidant include butylated hydroxytoluene, butylated hydroxyanisole, propyl gallate, and gallic acid propyl.

Examples of the binder include sucrose, gelatin, gum arabic powder, and methylcellulose.

### [Use]

The composition according to the present embodiments can be used as a composition for promoting absorption of a cyclic peptide compound, since it promotes oral absorption of a cyclic peptide compound having low membrane permeability.

The composition according to the present embodiments may also be used as a pharmaceutical composition for tough targets such as protein-protein interaction inhibition, agonists, molecular chaperones, or the like, depending on the type of cyclic peptide compound used.

Furthermore, the composition according to the present embodiments may be used as a composition for administration, especially as a composition for oral administration, to a living organism. Examples of the subject to be administered include a mammal, specifically mouse, rat, rabbit, dog, monkey, and human. The composition according to the present embodiments may be used particularly for administration to human. Thus, the composition according to the present invention can be used as a pharmaceutical composition. Furthermore, the present invention provides a method for treatment and/or prevention, which includes administering an effective amount of the composition according to the present invention to a subject in need thereof.

### [Production method of composition]

The composition according to the present embodiments can be produced by a method comprising the following steps (a) and (b):
(a) providing a compound represented by general formula (1), a pharmaceutically acceptable salt thereof, or a pharmaceutically acceptable solvate thereof, and
(b) mixing a surfactant as an isolated component with the compound represented by general formula (1), a pharmaceutically acceptable salt thereof, or a pharmaceutically acceptable solvate thereof.

The production method of the composition according to the present embodiments can further comprise the following step (c):
(c) mixing a solubility improver with the compound represented by general formula (1), a pharmaceutically acceptable salt thereof, or a pharmaceutically acceptable solvate thereof. The compound represented by general formula (1), a pharmaceutically acceptable salt thereof, or a pharmaceutically acceptable solvate thereof, the surfactant, and the solubility improver are each as disclosed herein.

### [Other aspects of the present invention]

In one non-limiting aspect, the composition according to the present embodiments contains (1) a compound represented by general formula (1), a pharmaceutically acceptable salt thereof, or a pharmaceutically acceptable solvate thereof. In this aspect, the composition can be used in combination with a surfactant to improve the absorption of the compound according to the present embodiments. Except for being free of surfactant, i.e. component (2), the composition can be applied without limitation to each of the aspects mentioned above. When used in combination with a surfactant, the amount of surfactant used may be 0.05 parts by mass or more, 0.075 parts by mass or more, 0.1 parts by mass or more, 0.2 parts by mass or more, or 0.3 parts by mass or more based on 1 part by mass of the component (1) in the composition according to the present embodiments. In addition, the surfactant may be 300 parts by mass or less, 200 parts by mass or less, 150 parts by mass or less, 100 parts by mass or less, 80 parts by mass or less, 60 parts by mass or less, 40 parts by mass or less, or 30 parts by mass or less based on 1 part by mass of the component (1). Furthermore, the amount of surfactant used may be 0.05 parts by mass or more and 300 parts by mass or less, 0.05 parts by mass or more and 200 parts by mass or less, 0.05 parts by mass or more and 150 parts by mass or less, 0.05 parts by mass or more and 100 parts by mass or less, 0.075 parts by mass or more and 80 parts by mass or less, 0.1 parts by mass or more and 60 parts by mass or less, 0.2 parts by mass or more and 40 parts by mass or less, or 0.3 parts by mass or more and 30 parts by mass or less based on 1 part by mass of the component (1). When the component (1) contains two or more cyclic peptide compounds or the like, the above range is to the total amount thereof. The same holds for the surfactant.

In one non-limiting aspect, the present invention can also be regarded as a method for improving the absorption of a compound represented by general formula (1), the method comprising incorporating, in a same composition, the compound represented by general formula (1), a pharmaceutically acceptable salt thereof, or a pharmaceutically acceptable solvate thereof, and a surfactant. As a specific aspect in the method according to the present embodiments, the aspect described in the composition according to the present invention can be applied without any particular limitations. In addition, in the method according to the present embodiments, the surfactant is preferably at least one selected from the group consisting of lauroyl-L-carnitine, sodium caprylate, sodium lauryl sulfate, and sodium N-(8-[2-hydroxybenzoyl]amino)caprylate.

In one non-limiting aspect, the present invention can also be regarded as a method for improving the absorption of a compound represented by general formula (1), the method including using the compound represented by general formula (1), a pharmaceutically acceptable salt thereof, or a pharmaceutically acceptable solvate thereof, in combination with a surfactant. As a specific aspect in the method according to the present embodiments, the aspect described in the composition according to the present invention can be applied without any particular limitations. In addition, in the method according to the present embodiments, the surfactant is preferably at least one selected from the group consisting of lauroyl-L-carnitine, sodium caprylate, sodium lauryl sulfate, and sodium N-(8-[2-hydroxybenzoyl]amino)caprylate.

In one non-limiting aspect, the present invention can also be regarded as a method for improving the absorption of a compound represented by general formula (1), the method including using a composition containing the compound represented by general formula (1), a pharmaceutically acceptable salt thereof, or a pharmaceutically acceptable solvate thereof, in combination with a composition containing a surfactant. As a specific aspect in the method according to the present embodiments, the aspect described in the composition according to the present invention can be applied without any particular limitations. In addition, in the method according to the present embodiments, the surfactant is preferably at least one selected from the group consisting of lauroyl-L-carnitine, sodium caprylate, sodium lauryl sulfate, and sodium N-(8-[2-hydroxybenzoyl]amino)caprylate.

### Examples

Hereinafter, preferred specific aspects of the present invention are described by way of Examples, but the present invention is not limited thereto.

### [Synthesis Example] Synthesis of cyclic peptide compounds

Cyclic peptide compounds CP01 to CP06 (they are also referred to simply as compounds CP01 to CP06.) shown in Tables 1 and 2 were synthesized by the same method as described in International Publication No. WO 2022/234853, and the final products were obtained as dry products. Specifically, PP1105 in International Publication No. WO 2022/234853 corresponds to compound CP01, PP1248 corresponds to compound CP02, compound PP1275 corresponds to compound CP03, PP1650 corresponds to compound CP04, compound PP2320 corresponds to compound CP05, and PP2328 corresponds to compound CP06.

Tables 1 and 2 show the structural formulae of compounds CP01 to CP06, and Table 3 shows the chemical names of compounds CP01 to CP06.

**[Table 1]**

| Compound No. | Structure |
|---|---|
| CP01 | 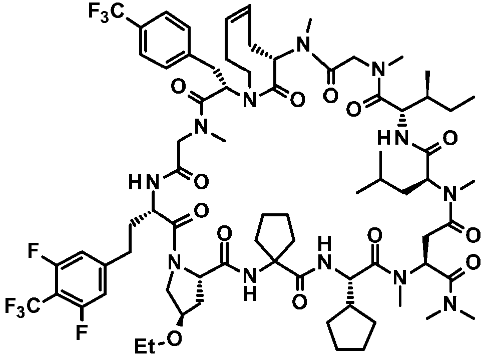 |
| CP02 | 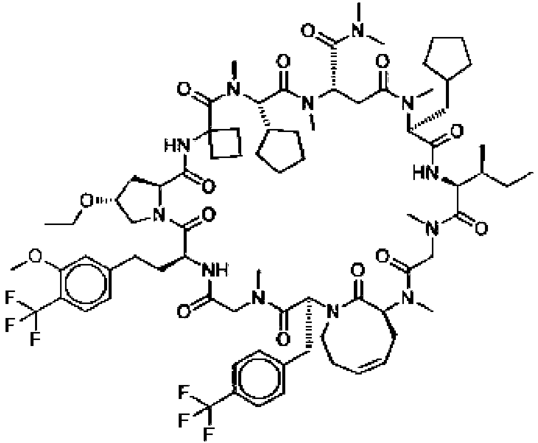 |
| CP03 | 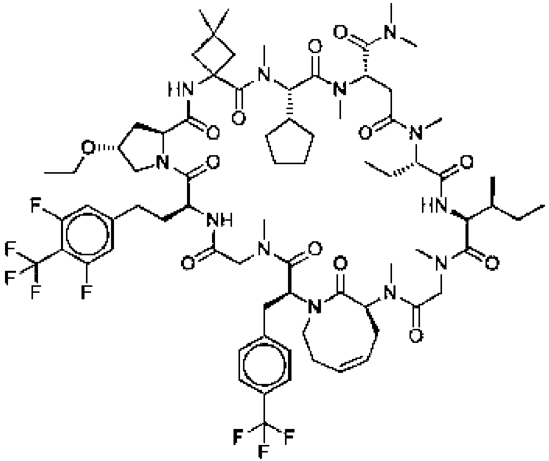 |

**[Table 2]**

| Compound No. | Structure |
|---|---|
| CP04 | 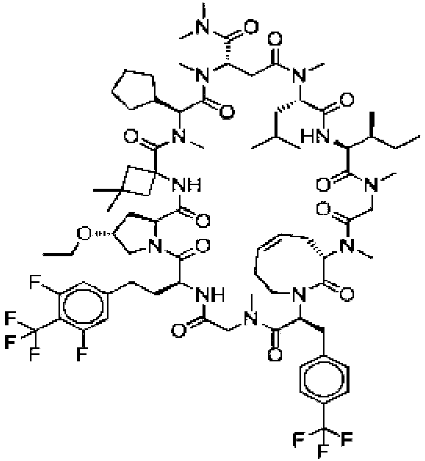 |
| CP05 | 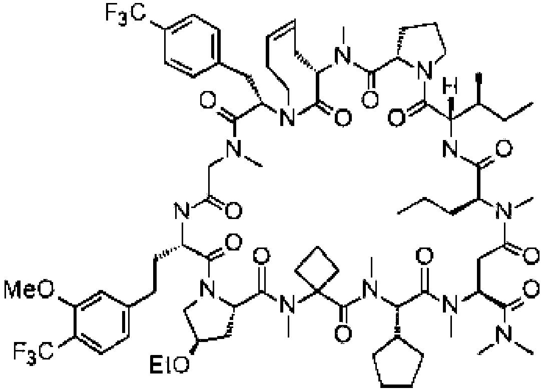 |
| CP06 | 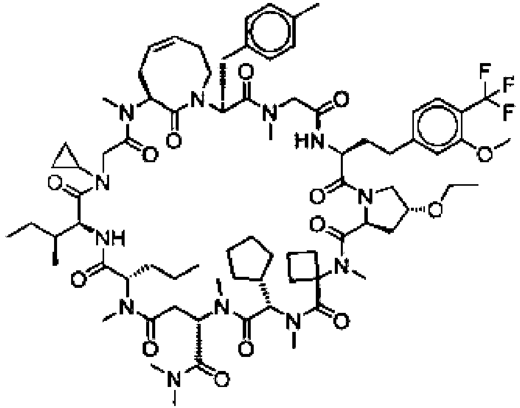 |

**[Table 3]**

| Compound No. | *Chemical Name* |
|---|---|
| CP01 | *(2S,8S,12R,14S,20S,23S,27S,30S,36S,38Z)-20-cyclopentyl-8-[2-[3,5-difluoro-4-(trifluoromethyl)phenyl]ethyl]-12-ethoxy-27-isobutyl-N,N,4,22,26,32,35-heptamethyl-30-[(1S)-1-methylpropyl]-3,6,9,15,18,21,25,28,31,34,42-undecaoxo-2-[(4-(trifluoromethyl)phenyl]methyl]spiro[1,4,7,10,16,19,22,26,29,32,35-undecazatricyclo[34.5.1.0^{10,14}]dotetraconta-38-en-17,1'-cyclopentane]-23-carboxamide* |
| CP02 | *(2S,8S,12R,14S,20S,23S,27S,30S,36S,38Z)-20-cyclopentyl-27-(cyclopentylmethyl)-12-ethoxy-8-[2-[3-methoxy-4-(trifluoromethyl)phenyl]ethyl]-N,N,4,19,22,26,32,35-octamethyl-30-[(1S)-1-methylpropyl]-3,6,9,15,18,21,25,28,31,34,42-undecaoxo-2-[[4-(trifluoromethyl)phenyl]methyl]spiro[1,4,7,10,16,19,22,26,29,32,35-undecazatricyclo[34.5.1.0^{10,14}]dotetraconta-38-en-17,1'-cyclobutane]-23-carboxamide* |
| CP03 | *(2S,8S,12R,14S,20S,23S,27S,30S,36S,38Z)-20-cyclopentyl-8-[2-[3,5-difluoro-4-(trifluoromethyl)phenyl]ethyl]-12-ethoxy-27-ethyl-N,N,3',3',4,19,22,26,32,35-decamethyl-30-[(1S)-1-methylpropyl]-3,6,9,15,18,21,25,28,31,34,42-undecaoxo-2-[[4-(trifluoromethyl)phenyl]methyl]spiro[1,4,7,10,16,19,22,26,29,32,35-undecazatricyclo[34.5.1.0^{10,14}]dotetraconta-38-en-17,1'-cyclobutane]-23-carboxamide* |
| CP04 | *(2S,8S,12R,14S,20S,23S,27S,30S,36S,38Z)-20-cyclopentyl-8-[2-[3,5-difluoro-4-(trifluoromethyl)phenyl]ethyl]-12-ethoxy-27-isobutyl-N,N,3',3',4,19,22,26,32,35-decamethyl-30-[(1S)-1-methylpropyl]-3,6,9,15,18,21,25,28,31,34,42-undecaoxo-2-[[4-(trifluoromethyl)phenyl]methyljspiro[1,4,7,10,16,19,22,26,29,32,35-undecazatricyclo[34.5.1.0^{10,14}]dotetraconta-38-en-17,1'-cyclobutane]-23-carboxamide* |
| CP05 | *(1S,4S,10S,13S,17S,20S,26S,28R,32S,38S,42Z)-20-cyclopentyl-28-ethoxy-32-[2-[3-methoxy-4-(trifluoromethyl)phenyl]ethyl]-N,N,2,14,18,21,24,36-octamethyl-10-[(1S)-1-methylpropyl]-3,9,12,15,19,22,25,31,34,37,45-undecaoxo-13-propyl-38-[[4-(trifluoromethyl)phenyl]methyl]spiro[2,8,11,14,18,21,24,30,33,36,39-undecazatetracyclo[37.5.1.0^{4,8}.0^{26,30}]pentatetraconta-42-en-23,1'-cyclobutane]-17-carboxamide* |
| CP06 | *(2S,8S,12R,14S,20S,23S,27S,30S,36S,38Z)-20-cyclopentyl-32-cyclopropyl-12-ethoxy-8-[2-[3-methoxy-4-(trifluoromethyl)phenyl]ethyl]-N,N,4,16,19,22,26,35-octamethyl-30-[(1S)-1-methylpropyl]-3,6,9,15,18,21,25,28,31,34,42-undecaoxo-27-propyl-2-(p-tolylmethyl)spiro[1,4,7,10,16,19,22,26,29,32,35-undecazatricyclo[34.5.1.0^{10,14}]dotetraconta-38-en-17,1'-cyclobutane]-23-carboxamide* |

### (Evaluation Example 1) Evaluation of Caco-2 membrane permeability

Caco-2 cells were cultured on 96-well Transwell for 3 weeks. The permeability study was then started by adding 10 µM of compound CP01 and a FaSSIF/HBSS buffer (pH 6.5) containing lauroyl-L-carnitine hydrochloride (manufactured by Sinochem Japan Co., Ltd.), sodium caprylate (manufactured by Sigma-Aldrich Co., Ltd.,) or salcaprozate sodium (manufactured by Fluorochem) to the apical side and adding an HBSS buffer (pH 7.4) containing 4% BSA to the basal side. Each well was shaken at 80 rpm in 5%CO₂ at 37°C, and 180 minutes after the start, a sample on the basal side was taken and the permeation amount of the compound was measured by liquid chromatography-mass spectrometry (LC/MS/MS). From the permeation amount, the permeability coefficient (Caco-2 Papp (cm/sec)) was calculated. The results are shown in Table 4.

As comparative examples, the permeation amount was measured by the same procedure as described above except that a FaSSIF/HBSS buffer (pH 6.5) without surfactant was used. In other words, on the apical side, the compound CP01 and a FaSSIF/HBSS buffer (pH 6.5) were added. From the measured permeation amount, the permeability coefficient was calculated. The results are shown in Table 4.

**[Table 4]**

| Surfactant | | Permeability Coefficient (Caco-2 Papp) (cm/sec) |
|---|---|---|
| Type | (mM) | |
| - | - | 5.25E-08 |
| Lauroyl-L-carnitine Hydrochloride | 1 | 9.94E-08 |
| | 3 | 2.14E-07 |
| | 5 | 2.37E-07 |
| Sodium Caprylate | 1 | 1.10E-07 |
| | 5 | 6.43E-08 |
| Salcaprozate Sodium | 10 | 1.19E-07 |
| | 30 | 2.91E-07 |

The above results, it was confirmed that membrane permeability was improved under the condition where lauroyl-L-carnitine hydrochloride, sodium caprylate, or salcaprozate sodium was added, compared with the condition where these surfactants were not added. In particular, a concentration-dependent improvement of the permeability coefficient was observed with lauroyl-L-carnitine hydrochloride, which exhibited a high permeability coefficient even at low concentrations.

### [Examples 1 to 32, Comparative Examples 1 to 27, and Production Examples 1 to 14]

### Preparations of absorption-promoting formulations (1) to (32), solution formulations (1) to (27), and iv formulations (1) to (14)

Compounds CP01 to CP06, lauroyl-L-carnitine hydrochloride (manufactured by Sinochem Japan Co., Ltd.), water for injection (manufactured by Otsuka Pharmaceutical Factory Co., Ltd.), dimethyl sulfoxide (manufactured by Wako Pure Chemical Co., Ltd.), Cremophor EL (manufactured by Sigma-Aldrich Co., Ltd., Kolliphor EL, generic name: polyoxyethylene castor oil, the average number of moles of ethylene oxide added is 35), Tween 80 (manufactured by Nacalai Tesque, Inc., polyoxyethylene sorbitan monooleate), and monkey serum were mixed to be the compositions of Tables 5, 6, and 7, to prepare absorption-promoting formulations (1) to (32), solution formulations (1) to (27), and iv formulations (1) to (14). It should be noted that compounds CP01 to CP06 were mixed while dissolved in dimethyl sulfoxide. In addition, lauroyl-L-carnitine hydrochloride, Cremophor EL and Tween 80 were added in the form of powder or undiluted solution and mixed.

In Tables 5, 6 and 7, (*1) indicates the content in 1 mL of solvent and (*2) indicates the content based on 100% by volume of the solvent.

**[Table 5]**

| | | Solute | | | Solvent | | |
|---|---|---|---|---|---|---|---|
| | | Compound | | Surfactant | Solubility Improver | Water for Injection | DMSO |
| | | | | Lauroyl-L-carnitine Hydrochloride | Cremophor EL | | |
| | | Type | mg/mL ^{(*1)} | mg/mL ^{(*1)} | % by volume ^{(*2)} | % by volume ^{(*2)} | % by volume ^{(*2)} |
| Comparative Example 1 | Solution formulation (1) | CP01 | 0.15 | 0 | 1 | 89 | 10 |
| Example 1 | Absorption-promoting agent (1) | CP01 | 0.15 | 20 | 1 | 89 | 10 |
| Comparative Example 2 | Solution formulation (2) | CP01 | 5 | 0 | 10 | 80 | 10 |
| Example 2 | Absorption-promoting agent (2) | CP01 | 5 | 20 | 10 | 80 | 10 |
| Comparative Example 3 | Solution formulation (3) | CP02 | 0.15 | 0 | 1 | 89 | 10 |
| Example 3 | Absorption-promoting agent (3) | CP02 | 0.15 | 20 | 1 | 89 | 10 |
| Comparative Example 4 | Solution formulation (4) | CP02 | 5 | 0 | 10 | 80 | 10 |
| Example 4 | Absorption-promoting agent (4) | CP02 | 5 | 20 | 10 | 80 | 10 |
| Comparative Example 5 | Solution formulation (5) | CP03 | 0.15 | 0 | 1 | 89 | 10 |
| Example 5 | Absorption-promoting agent (5) | CP03 | 0.15 | 20 | 1 | 89 | 10 |
| Comparative Example 6 | Solution formulation (6) | CP03 | 5 | 0 | 10 | 80 | 10 |
| Example 6 | Absorption-promoting agent (6) | CP03 | 5 | 20 | 10 | 80 | 10 |
| Comparative Example 7 | Solution formulation (7) | CP04 | 0.3 | 0 | 1 | 89 | 10 |
| Example 7 | Absorption-promoting agent (7) | CP04 | 0.3 | 10 | 1 | 89 | 10 |
| Comparative Example 8 | Solution formulation (8) | CP04 | 3 | 0 | 7.5 | 82.5 | 10 |
| Example 8 | Absorption-promoting agent (8) | CP04 | 3 | 10 | 7.5 | 82.5 | 10 |
| Comparative Example 9 | Solution formulation (9) | CP05 | 0.3 | 0 | 1 | 89 | 10 |
| Example 9 | Absorption-promoting agent (9) | CP05 | 0.3 | 10 | 1 | 89 | 10 |
| Comparative Example 10 | Solution formulation (10) | CP05 | 3 | 0 | 10 | 80 | 10 |
| Example 10 | Absorption-promoting agent (10) | CP05 | 3 | 10 | 10 | 80 | 10 |
| Comparative Example 11 | Solution formulation (11) | CP06 | 0.3 | 0 | 1 | 89 | 10 |
| Example 11 | Absorption-promoting agent (11) | CP06 | 0.3 | 10 | 1 | 89 | 10 |
| Comparative Example 12 | Solution formulation (12) | CP06 | 3 | 0 | 7.5 | 82.5 | 10 |
| Example 12 | Absorption-promoting agent (12) | CP06 | 3 | 10 | 7.5 | 82.5 | 10 |
| Comparative Example 13 | Solution formulation (13) | CP01 | 0.3 | 0 | 1 | 89 | 10 |
| Example 13 | Absorption-promoting agent (13) | CP01 | 0.3 | 10 | 1 | 89 | 10 |
| Comparative Example 14 | Solution formulation (14) | CP01 | 3 | 0 | 5 | 85 | 10 |
| Example 14 | Absorption-promoting agent (14) | CP01 | 3 | 10 | 5 | 85 | 10 |
| Comparative Example 15 | Solution formulation (15) | CP02 | 0.3 | 0 | 1 | 89 | 10 |
| Example 15 | Absorption-promoting agent (15) | CP02 | 0.3 | 10 | 1 | 89 | 10 |
| Comparative Example 16 | Solution formulation (16) | CP02 | 3 | 0 | 5 | 85 | 10 |
| Example 16 | Absorption-promoting agent (16) | CP02 | 3 | 10 | 5 | 85 | 10 |

**[Table 6]**

| | | Solute | | | Solvent | | |
|---|---|---|---|---|---|---|---|
| | | Compound | | Surfactant | Solubility Improver | Water for Injection | DMSO |
| | | | | Lauroyl-L-carnitine Hydrochloride | Cremophor EL | | |
| | | Type | mg/mL ^{(*1)} | mg/mL ^{(*1)} | % by volume ^{(*2)} | % by volume ^{(*2)} | % by volume ^{(*2)} |
| Comparative Example 17 | Solution formulation (17) | CP03 | 0.3 | 0 | 1 | 89 | 10 |
| Example 17 | Absorption-promoting agent (17) | CP03 | 0.3 | 10 | 1 | 89 | 10 |
| Comparative Example 18 | Solution formulation (18) | CP03 | 3 | 0 | 5 | 85 | 10 |
| Example 18 | Absorption-promoting agent (18) | CP03 | 3 | 10 | 5 | 85 | 10 |
| Comparative Example 19 | Solution formulation (19) | CP04 | 0.3 | 0 | 1 | 89 | 10 |
| Example 19 | Absorption-promoting agent (19) | CP04 | 0.3 | 10 | 1 | 89 | 10 |
| Comparative Example 20 | Solution formulation (20) | CP04 | 3 | 0 | 7.5 | 82.5 | 10 |
| Example 20 | Absorption-promoting agent (20) | CP04 | 3 | 10 | 7.5 | 82.5 | 10 |
| Comparative Example 21 | Solution formulation (21) | CP05 | 0.3 | 0 | 1 | 89 | 10 |
| Example 21 | Absorption-promoting agent (21) | CP05 | 0.3 | 10 | 1 | 89 | 10 |
| Comparative Example 22 | Solution formulation (22) | CP05 | 3 | 0 | 5 | 85 | 10 |
| Example 22 | Absorption-promoting agent (22) | CP05 | 3 | 10 | 5 | 85 | 10 |
| Comparative Example 23 | Solution formulation (23) | CP06 | 0.3 | 0 | 1 | 89 | 10 |
| Example 23 | Absorption-promoting agent (23) | CP06 | 0.3 | 10 | 1 | 89 | 10 |
| Comparative Example 24 | Solution formulation (24) | CP06 | 3 | 0 | 5 | 85 | 10 |
| Example 24 | Absorption-promoting agent (24) | CP06 | 3 | 10 | 5 | 85 | 10 |
| Comparative Example 25 | Solution formulation (25) | CP01 | 0.2 | 0 | 0.4 | 89.6 | 10 |
| Example 25 | Absorption-promoting agent (25) | CP01 | 0.2 | 1 | 0.4 | 89.6 | 10 |
| Example 26 | Absorption-promoting agent (26) | CP01 | 0.2 | 2 | 0.4 | 89.6 | 10 |
| Example 27 | Absorption-promoting agent (27) | CP01 | 0.2 | 5 | 0.4 | 89.6 | 10 |
| Comparative Example 26 | Solution formulation (26) | CP01 | 0.6 | 0 | 1 | 89 | 10 |
| Example 28 | Absorption-promoting agent (28) | CP01 | 0.6 | 1 | 1 | 89 | 10 |
| Example 29 | Absorption-promoting agent (29) | CP01 | 0.6 | 2 | 1 | 89 | 10 |
| Example 30 | Absorption-promoting agent (30) | CP01 | 0.6 | 5 | 1 | 89 | 10 |
| Comparative Example 27 | Solution formulation (27) | CP05 | 0.6 | 0 | 1 | 89 | 10 |
| Example 31 | Absorption-promoting agent (31) | CP05 | 0.6 | 2 | 1 | 89 | 10 |
| Example 32 | Absorption-promoting agent (32) | CP05 | 0.6 | 5 | 1 | 89 | 10 |

**[Table 7]**

| | | Solute | | Solvent | | | |
|---|---|---|---|---|---|---|---|
| | | Compound | | Solubility Improver | Water for Injection | Monkey Serum | DMSO |
| | | | | Tween 80 | | | |
| | | Type | mg/mL ^{(*1)} | % by volume ^{(*2)} | % by volume ^{(*2)} | % by volume ^{(*2)} | % by volume ^{(*2)} |
| Production Example 1 | iv formulation (1) | CP01 | 0.2 | 0.4 | 89.6 | - | 10 |
| Production Example 2 | iv formulation (2) | CP02 | 0.2 | 0.7 | 89.3 | - | 10 |
| Production Example 3 | iv formulation (3) | CP03 | 0.2 | 0.4 | 89.6 | - | 10 |
| Production Example 4 | iv formulation (4) | CP04 | 0.2 | 1 | 89 | - | 10 |
| Production Example 5 | iv formulation (5) | CP05 | 0.2 | 1 | 89 | - | 10 |
| Production Example 6 | iv formulation (6) | CP06 | 0.2 | 1 | 89 | - | 10 |
| Production Example 7 | iv formulation (7) | CP01 | 0.15 | 0.25 | 89.75 | - | 10 |
| Production Example 8 | iv formulation (8) | CP02 | 0.15 | 0.5 | 89.5 | - | 10 |
| Production Example 9 | iv formulation (9) | CP03 | 0.15 | 0.25 | 89.75 | - | 10 |
| Production Example 10 | iv formulation (10) | CP04 | 0.15 | 0.5 | 89.5 | - | 10 |
| Production Example 11 | iv formulation (11) | CP05 | 0.15 | 0.5 | 89.5 | - | 10 |
| Production Example 12 | iv formulation (12) | CP06 | 0.15 | 0.5 | 89.5 | - | 10 |
| Production Example 13 | iv formulation (13) | CP01 | 0.1 | - | - | 99 | 1 |
| Production Example 14 | iv formulation (14) | CP05 | 0.1 | 0.3 | 89.7 | - | 10 |

### (Evaluation Example 2) Mouse PK study (3 mg/kg, 100 mg/kg)

Pharmacokinetic after oral administration of solution formulations (1) and (2) prepared in Comparative Examples 1 and 2 and absorption-promoting formulations (1) and (2) prepared in Examples 1 and 2 in mice were evaluated. To female mice (Balb/c nu-nu, 7 weeks of age, manufactured by Charles River Laboratories Japan, Inc.: 3 mice per group), either solution formulation (1) prepared in Comparative Example 1 or absorption-promoting formulation (1) prepared in Example 1 was orally administered at a dose of 3 mg/kg of compound CP01, and blood was collected over time from the jugular vein using a syringe up to 24 hours after administration. In addition, either solution formulation (2) prepared in Comparative Example 2 or absorption-promoting formulation (2) prepared in Example 2 was orally administered at a dose of 100 mg/kg of compound CP01, and blood was collected over time from the jugular vein using a syringe up to 24 hours after administration. Furthermore, iv formulation (1) prepared in Production Example 1 was administered intravenously at a dose of 1 mg/kg of compound CP01, and blood was collected over time from the jugular vein using a syringe up to 24 hours after administration. The blood was dispensed into tubes treated with heparin as an anticoagulant, and centrifuged to separate plasma. After deproteinization with acetonitrile, the plasma concentration of compound CP01 was measured with an LC/MS/MS device (XEVO TQ-XS, manufactured by Waters Corporation). From the obtained changes in plasma concentration, the pharmacokinetic parameters were calculated by non-compartmental analysis using analysis software Phoenix WinNonlin 8.2 (manufactured by Certara L.P.). The results are shown in Table 8.

As the pharmacokinetic parameters, area under the plasma concentration-time curve (AUC; ng-h/mL), the highest plasma concentration after oral administration (Cmax; ng/mL), and bioavailability (BA) were calculated. When a plasma concentration was equal to or below the lower limit of quantitation, the concentration was treated as 0 ng/mL. BA was calculated as a ratio of AUC of solution formulation (AUCsol) or AUC of absorption-promoting formulation (AUClc) to AUC of iv formulation (AUCiv), i.e., AUCsol/AUCiv or AUClc/AUCiv. In compound CP01, it was confirmed that any AUC in the administration groups of absorption-promoting formulations (1) or (2) of Examples 1 or 2 was higher than AUC in the administration group of solution formulations (1) or (2) of Comparative Examples 1 or 2, and an increase in Cmax was also observed (Table 8). From this, it was confirmed that, with the use of lauroyl-L-carnitine hydrochloride, the compound with low membrane-permeability showed a high BA compared to without lauroyl-L-carnitine hydrochloride.

**[Table 8]**

| Pharmacokinetic parameters of each formulation of compound CP01 (mice, 3 mg/kg or 100 mg/kg) | | | | |
|---|---|---|---|---|
| Dose | Formulation | AUC (ng*h/mL) | Cmax (ng/mL) | BA (%) |
| 3 mg/kg | Solution formulation (1) | 180 | 60.5 | 2.50 |
| | Absorption-promoting formulation (1) | 984 | 241 | 13.7 |
| 100 mg/kg | Solution formulation (2) | 19700 | 3650 | 8.23 |
| | Absorption-promoting formulation (2) | 79300 | 11100 | 33.1 |
| 1 mg/kg | iv formulation (1) | 2400 | - | - |

### (Evaluation Example 3) Mouse PK study (3 mg/kg, 100 mg/kg)

Pharmacokinetic after oral administration of solution formulations (3) and (4) prepared in Comparative Examples 3 and 4 and absorption-promoting formulations (3) and (4) prepared in Examples 3 and 4 and after intravenous administration of iv formulation (2) prepared in Production Example 2 in female mice (Balb/c nu-nu, 7 weeks of age) were evaluated in the same manner as in Evaluation Example 2. From the resulting changes in plasma concentration, the pharmacokinetic parameters were calculated by the same analysis as in Evaluation Example 2. The results are shown in Table 9. As a result, it was confirmed that any AUC in the administration groups of absorption-promoting formulations (3) or (4) of Examples 3 or 4 was higher than AUC in the administration group of solution formulations (3) or (4) of Comparative Examples 3 or 4, and an increase in Cmax was also observed (Table 9). From this, it was confirmed that, with the use of lauroyl-L-carnitine hydrochloride, a high BA was exhibited compared to without lauroyl-L-carnitine hydrochloride.

**[Table 9]**

| Pharmacokinetic parameters of each formulation of compound CP02 (mice, 3 mg/kg or 100 mg/kg) | | | | |
|---|---|---|---|---|
| Dose | Formulation | AUC (ng*h/mL) | Cmax (ng/mL) | BA (%) |
| 3 mg/kg | Solution formulation (3) | 60.5 | 21.3 | 1.45 |
| | Absorption-promoting formulation (3) | 616 | 110 | 14.7 |
| 100 mg/kg | Solution formulation (4) | 12600 | 2110 | 9.05 |
| | Absorption-promoting formulation (4) | 35000 | 7400 | 38.3 |
| 1 mg/kg | iv formulation (2) | 1390 | - | - |

### (Evaluation Example 4) Mouse PK study (3 mg/kg, 100 mg/kg)

Pharmacokinetic after oral administration of solution formulations (5) and (6) prepared in Comparative Examples 5 and 6 and absorption-promoting formulations (5) and (6) prepared in Examples 5 and 6 and after intravenous administration of iv formulation (3) prepared in Production Example 3 in female mice (Balb/c nu-nu, 7 weeks of age) were evaluated in the same manner as in Evaluation Example 2. From the resulting changes in plasma concentration, the pharmacokinetic parameters were calculated by the same analysis as in Evaluation Example 2. The results are shown in Table 10. As a result, it was confirmed that any AUC in the administration groups of absorption-promoting formulations (5) or (6) of Examples 5 or 6 was higher than AUC in the administration group of solution formulations (5) or (6) of Comparative Examples 5 or 6, and an increase in Cmax was also observed (Table 10). From this, it was confirmed that, with the use of lauroyl-L-carnitine hydrochloride, a high BA was exhibited compared to without lauroyl-L-carnitine hydrochloride.

**[Table 10]**

| Pharmacokinetic parameters of each formulation of compound CP03 (mice, 3 mg/kg or 100 mg/kg) | | | | |
|---|---|---|---|---|
| Dose | Formulation | AUC (ng*h/mL) | Cmax (ng/mL) | BA (%) |
| 3 mg/kg | Solution formulation (5) | 209 | 68.9 | 5.15 |
| | Absorption-promoting formulation (5) | 789 | 156 | 19.5 |
| 100 mg/kg | Solution formulation (6) | 35600 | 6030 | 26.4 |
| | Absorption-promoting formulation (6) | 110000 | 15700 | 81.6 |
| 1 mg/kg | iv formulation (3) | 1350 | - | - |

### (Evaluation Example 5) Mouse PK study (3 mg/kg, 30 mg/kg)

Pharmacokinetic after oral administration of solution formulations (7) and (8) prepared in Comparative Examples 7 and 8 and absorption-promoting formulations (7) and (8) prepared in Examples 7 and 8 and after intravenous administration of iv formulation (4) prepared in Production Example 4 in male mice (C57BL6J, 10 weeks of age, manufactured by The Jackson Laboratory Japan, Inc.: 3 mice per group) were evaluated in the same manner as in Evaluation Example 2. From the resulting changes in plasma concentration, the pharmacokinetic parameters were calculated by the same analysis as in Evaluation Example 2. The results are shown in Table 11. As a result, it was confirmed that any AUC in the administration groups of absorption-promoting formulations (7) or (8) of Examples 7 or 8 was higher than AUC in the administration group of solution formulations (7) or (8) of Comparative Examples 7 or 8, and an increase in Cmax was also observed (Table 11). From this, it was confirmed that, with the use of lauroyl-L-carnitine hydrochloride, a high BA was exhibited compared to without lauroyl-L-carnitine hydrochloride.

**[Table 11]**

| Pharmacokinetic parameters of each formulation of compound CP04 (mice, 3 mg/kg or 30 mg/kg) | | | | |
|---|---|---|---|---|
| Dose | Formulation | AUC (ng*h/mL) | Cmax (ng/mL) | BA (%) |
| 3 mg/kg | Solution formulation (7) | 1270 | 184 | 3.48 |
| | Absorption-promoting formulation (7) | 8490 | 1190 | 23.3 |
| 30 mg/kg | Solution formulation (8) | 56900 | 6330 | 15.6 |
| | Absorption-promoting formulation (8) | 164000 | 18200 | 45.0 |
| 1 mg/kg | iv formulation (4) | 12200 | - | - |

### (Evaluation Example 6) Mouse PK study (3 mg/kg, 30 mg/kg)

Pharmacokinetic after oral administration of solution formulations (9) and (10) prepared in Comparative Examples 9 and 10 and absorption-promoting formulations (9) and (10) prepared in Examples 9 and 10 and after intravenous administration of iv formulation (5) prepared in Production Example 5 in male mice (C57BL6J, 10 weeks of age) were evaluated in the same manner as in Evaluation Example 2. From the resulting changes in plasma concentration, the pharmacokinetic parameters were calculated by the same analysis as in Evaluation Example 2. The results are shown in Table 12. As a result, it was confirmed that any AUC in the administration groups of absorption-promoting formulations (9) or (10) of Examples 9 or 10 was higher than AUC in the administration group of solution formulations (9) or (10) of Comparative Examples 9 or 10, and an increase in Cmax was also observed (Table 12). From this, it was confirmed that, with the use of lauroyl-L-carnitine hydrochloride, a high BA was exhibited compared to without lauroyl-L-carnitine hydrochloride.

**[Table 12]**

| Pharmacokinetic parameters of each formulation of compound CP05 (mice, 3 mg/kg or 30 mg/kg) | | | | |
|---|---|---|---|---|
| Dose | Formulation | AUC (ng*h/mL) | Cmax (ng/mL) | BA (%) |
| 3 mg/kg | Solution formulation (9) | 1930 | 222 | 22.4 |
| | Absorption-promoting formulation (9) | 4140 | 530 | 48.1 |
| 30 mg/kg | Solution formulation (10) | 41600 | 4830 | 48.3 |
| | Absorption-promoting formulation (10) | 80100 | 7710 | 93.1 |
| 1 mg/kg | iv formulation (5) | 2870 | - | - |

### (Evaluation Example 7) Mouse PK study (3 mg/kg, 30 mg/kg)

Pharmacokinetic after oral administration of solution formulations (11) and (12) prepared in Comparative Examples 11 and 12 and absorption-promoting formulations (11) and (12) prepared in Examples 11 and 12 and after intravenous administration of iv formulation (6) prepared in Production Example 6 in male mice (C57BL6J, 10 weeks of age) were evaluated in the same manner as in Evaluation Example 2. From the resulting changes in plasma concentration, the pharmacokinetic parameters were calculated by the same analysis as in Evaluation Example 2. The results are shown in Table 13. As a result, it was confirmed that any AUC in the administration groups of absorption-promoting formulations (11) or (12) of Examples 11 or 12 was higher than AUC in the administration group of solution formulations (11) or (12) of Comparative Examples 11 or 12, and an increase in Cmax was also observed (Table 13). From this, it was confirmed that, with the use of lauroyl-L-carnitine hydrochloride, a high BA was exhibited compared to without lauroyl-L-carnitine hydrochloride.

**[Table 13]**

| Pharmacokinetic parameters of each formulation of compound CP06 (mice, 3 mg/kg or 30 mg/kg) | | | | |
|---|---|---|---|---|
| Dose | Formulation | AUC (ng*h/mL) | Cmax (ng/mL) | BA (%) |
| 3 mg/kg | Solution formulation (11) | 956 | 153 | 10.1 |
| | Absorption-promoting formulation (11) | 3970 | 474 | 42.0 |
| 30 mg/kg | Solution formulation (12) | 75200 | 5020 | 58.8 |
| | Absorption-promoting formulation (12) | 70800 | 6810 | 75.7 |
| 1 mg/kg | iv formulation (6) | 3150 | - | - |

### (Evaluation Example 8) Rat PK study (3 mg/kg, 30 mg/kg)

Pharmacokinetic after oral administration of solution formulations (13) and (14) prepared in Comparative Examples 13 and 14 and absorption-promoting formulations (13) and (14) prepared in Examples 13 and 14 and after intravenous administration of iv formulation (7) prepared in Production Example 7 in male rats (SD, 6 weeks of age, manufactured by The Jackson Laboratory Japan, Inc.: 3 rats per group) were evaluated in the same manner as in Evaluation Example 2. From the resulting changes in plasma concentration, the pharmacokinetic parameters were calculated by the same analysis as in Evaluation Example 2. The results are shown in Table 14. As a result, it was confirmed that any AUC in the administration groups of absorption-promoting formulations (13) or (14) of Examples 13 or 14 was higher than AUC in the administration group of solution formulations (13) or (14) of Comparative Examples 13 or 14, and an increase in Cmax was also observed (Table 14). From this, it was confirmed that, with the use of lauroyl-L-carnitine hydrochloride, a high BA was exhibited compared to without lauroyl-L-carnitine hydrochloride.

**[Table 14]**

| Pharmacokinetic parameters of each formulation of compound CP01 (rats, 3 mg/kg or 30 mg/kg) | | | | |
|---|---|---|---|---|
| Dose | Formulation | AUC (ng*h/mL) | Cmax (ng/mL) | BA (%) |
| 3 mg/kg | Solution formulation (13) | 115 | 74.5 | 0.928 |
| | Absorption-promoting formulation (13) | 1620 | 536 | 13.1 |
| 30 mg/kg | Solution formulation (14) | 3510 | 1350 | 2.83 |
| | Absorption-promoting formulation (14) | 16200 | 4440 | 13.0 |
| 0.3 mg/kg | iv formulation (7) | 1240 | - | - |

### (Evaluation Example 9) Rat PK study (3 mg/kg, 30 mg/kg)

Pharmacokinetic after oral administration of solution formulations (15) and (16) prepared in Comparative Examples 15 and 16 and absorption-promoting formulations (15) and (16) prepared in Examples 15 and 16 and after intravenous administration of iv formulation (8) prepared in Production Example 8 in male rats (SD, 6 weeks of age) were evaluated in the same manner as in Evaluation Example 2. From the resulting changes in plasma concentration, the pharmacokinetic parameters were calculated by the same analysis as in Evaluation Example 2. The results are shown in Table 15. As a result, it was confirmed that any AUC in the administration groups of absorption-promoting formulations (15) or (16) of Examples 15 or 16 was higher than AUC in the administration group of solution formulations (15) or (16) of Comparative Examples 15 or 16, and an increase in Cmax was also observed (Table 15). From this, it was confirmed that, with the use of lauroyl-L-carnitine hydrochloride, a high BA was exhibited compared to without lauroyl-L-carnitine hydrochloride.

**[Table 15]**

| Pharmacokinetic parameters of each formulation of compound CP02 (rats, 3 mg/kg or 30 mg/kg) | | | | |
|---|---|---|---|---|
| Dose | Formulation | AUC (ng*h/mL) | Cmax (ng/mL) | BA (%) |
| 3 mg/kg | Solution formulation (15) | 123 | 101 | 1.51 |
| | Absorption-promoting formulation (15) | 2760 | 1750 | 34.0 |
| 30 mg/kg | Solution formulation (16) | 939 | 358 | 1.16 |
| | Absorption-promoting formulation (16) | 20700 | 5630 | 25.5 |
| 0.3 mg/kg | iv formulation (8) | 812 | - | - |

### (Evaluation Example 10) Rat PK study (3 mg/kg, 30 mg/kg)

Pharmacokinetic after oral administration of solution formulations (17) and (18) prepared in Comparative Examples 17 and 18 and absorption-promoting formulations (17) and (18) prepared in Examples 17 and 18 and after intravenous administration of iv formulation (9) prepared in Production Example 9 in male rats (SD, 6 weeks of age) were evaluated in the same manner as in Evaluation Example 2. From the resulting changes in plasma concentration, the pharmacokinetic parameters were calculated by the same analysis as in Evaluation Example 2. The results are shown in Table 16. As a result, it was confirmed that any AUC in the administration groups of absorption-promoting formulations (17) or (18) of Examples 17 or 18 was higher than AUC in the administration group of solution formulations (17) or (18) of Comparative Examples 17 or 18, and an increase in Cmax was also observed (Table 16). From this, it was confirmed that, with the use of lauroyl-L-carnitine hydrochloride, a high BA was exhibited compared to without lauroyl-L-carnitine hydrochloride.

**[Table 16]**

| Pharmacokinetic parameters of each formulation of compound CP03 (rats, 3 mg/kg or 30 mg/kg) | | | | |
|---|---|---|---|---|
| Dose | Formulation | AUC (ng*h/mL) | Cmax (ng/mL) | BA (%) |
| 3 mg/kg | Solution formulation (17) | 331 | 124 | 5.31 |
| | Absorption-promoting formulation (17) | 1030 | 422 | 16.5 |
| 30 mg/kg | Solution formulation (18) | 7790 | 1850 | 12.5 |
| | Absorption-promoting formulation (18) | 20900 | 4550 | 33.6 |
| 0.3 mg/kg | iv formulation (9) | 624 | - | - |

### (Evaluation Example 11) Rat PK study (3 mg/kg, 30 mg/kg)

Pharmacokinetic after oral administration of solution formulations (19) and (20) prepared in Comparative Examples 19 and 20 and absorption-promoting formulations (19) and (20) prepared in Examples 19 and 20 and after intravenous administration of iv formulation (10) prepared in Production Example 10 in male rats (SD, 7 weeks of age, The Jackson Laboratory Japan, Inc.: 3 rats per group) were evaluated in the same manner as in Evaluation Example 2. From the resulting changes in plasma concentration, the pharmacokinetic parameters were calculated by the same analysis as in Evaluation Example 2. The results are shown in Table 17. As a result, it was confirmed that any AUC in the administration groups of absorption-promoting formulations (19) or (20) of Examples 19 or 20 was higher than AUC in the administration group of solution formulations (19) or (20) of Comparative Examples 19 or 20, and an increase in Cmax was also observed (Table 17). From this, it was confirmed that, with the use of lauroyl-L-carnitine hydrochloride, a high BA was exhibited compared to without lauroyl-L-carnitine hydrochloride.

**[Table 17]**

| Pharmacokinetic parameters of each formulation of compound CP04 (rats, 3 mg/kg or 30 mg/kg) | | | | |
|---|---|---|---|---|
| Dose | Formulation | AUC (ng*h/mL) | Cmax (ng/mL) | BA (%) |
| 3 mg/kg | Solution formulation (19) | 1030 | 437 | 6.99 |
| | Absorption-promoting formulation (19) | 4880 | 1160 | 33.2 |
| 30 mg/kg | Solution formulation (20) | 6790 | 1860 | 4.62 |
| | Absorption-promoting formulation (20) | 65700 | 11800 | 44.7 |
| 0.3 mg/kg | iv formulation (10) | 1470 | - | - |

### (Evaluation Example 12) Rat PK study (3 mg/kg, 30 mg/kg)

Pharmacokinetic after oral administration of solution formulations (21) and (22) prepared in Comparative Examples 21 and 22 and absorption-promoting formulations (21) and (22) prepared in Examples 21 and 22 and after intravenous administration of iv formulation (11) prepared in Production Example 11 in male rats (SD, 7 weeks of age) were evaluated in the same manner as in Evaluation Example 2. From the resulting changes in plasma concentration, the pharmacokinetic parameters were calculated by the same analysis as in Evaluation Example 2. The results are shown in Table 18. As a result, it was confirmed that any AUC in the administration groups of absorption-promoting formulations (21) or (22) of Examples 21 or 22 was higher than AUC in the administration group of solution formulations (21) or (22) of Comparative Examples 21 or 22, and an increase in Cmax was also observed (Table 18). From this, it was confirmed that, with the use of lauroyl-L-carnitine hydrochloride, a high BA was exhibited compared to without lauroyl-L-carnitine hydrochloride.

**[Table 18]**

| Pharmacokinetic parameters of each formulation of compound CP05 (rats, 3 mg/kg or 30 mg/kg) | | | | |
|---|---|---|---|---|
| Dose | Formulation | AUC (ng*h/mL) | Cmax (ng/mL) | BA (%) |
| 3 mg/kg | Solution formulation (21) | 5650 | 1080 | 86.1 |
| | Absorption-promoting formulation (21) | 7050 | 1080 | 107 |
| 30 mg/kg | Solution formulation (22) | 24700 | 3740 | 37.7 |
| | Absorption-promoting formulation (22) | 52800 | 7400 | 80.4 |
| 0.3 mg/kg | iv formulation (11) | 656 | - | - |

### (Evaluation Example 13) Rat PK study (3 mg/kg, 30 mg/kg)

Pharmacokinetic after oral administration of solution formulations (23) and (24) prepared in Comparative Examples 23 and 24 and absorption-promoting formulations (23) and (24) prepared in Examples 23 and 24 and after intravenous administration of iv formulation (12) prepared in Production Example 12 in male rats (SD, 7 weeks of age) were evaluated in the same manner as in Evaluation Example 2. From the resulting changes in plasma concentration, the pharmacokinetic parameters were calculated by the same analysis as in Evaluation Example 2. The results are shown in Table 19. As a result, it was confirmed that any AUC in the administration groups of absorption-promoting formulations (23) or (24) of Examples 23 or 24 was higher than AUC in the administration group of solution formulations (23) or (24) of Comparative Examples 23 or 24, and an increase in Cmax was also observed (Table 19). From this, it was confirmed that, with the use of lauroyl-L-carnitine hydrochloride, a high BA was exhibited compared to without lauroyl-L-carnitine hydrochloride.

**[Table 19]**

| Pharmacokinetic parameters of each formulation of compound CP06 (rats, 3 mg/kg or 30 mg/kg) | | | | |
|---|---|---|---|---|
| Dose | Formulation | AUC (ng*h/mL) | Cmax (ng/mL) | BA (%) |
| 3 mg/kg | Solution formulation (23) | 4040 | 642 | 65.7 |
| | Absorption-promoting formulation (23) | 8550 | 1500 | 139 |
| 30 mg/kg | Solution formulation (24) | 54600 | 7660 | 88.7 |
| | Absorption-promoting formulation (24) | 88100 | 11700 | 143 |
| 0.3 mg/kg | iv formulation (12) | 615 | - | - |

### (Evaluation Example 14) Monkey PK study (1 mg/kg, 3 mg/kg)

Pharmacokinetic after oral administration of solution formulations (25) and (26) prepared in Comparative Examples 25 and 26 and absorption-promoting formulations (25) to (30) prepared in Examples 25 to 30 and after intravenous administration of iv formulation (13) prepared in Production Example 13 in male cynomolgus monkeys (manufactured by Tian Hu Cambodia Animal Breeding Research Center: 4 monkeys per group) were evaluated in the same manner as in Evaluation Example 2 except that blood was collected over time from the femoral vein using a syringe treated with heparin as an anticoagulant. From the resulting changes in plasma concentration, the pharmacokinetic parameters were calculated by the same analysis as in Evaluation Example 2. The results are shown in Table 20. As a result, it was confirmed that any AUC in the administration groups of absorption-promoting formulations (25) to (27) or (28) to (30) of Examples 25 to 27 or 28 to 30 was higher than AUC in the administration group of solution formulations (25) or (26) of Comparative Examples 25 or 26, and an increase in Cmax was also observed (Table 20). From this, it was confirmed that, with the use of lauroyl-L-carnitine hydrochloride, a high BA was exhibited compared to without lauroyl-L-carnitine hydrochloride.

**[Table 20]**

| Pharmacokinetic parameters of each formulation of compound CP01 (monkeys, 1 mg/kg or 3 mg/kg) | | | | |
|---|---|---|---|---|
| Dose | Formulation | AUC (ng*h/mL) | Cmax (ng/mL) | BA (%) |
| 1 mg/kg | Solution formulation (25) | 314 | 61.4 | 4.45 |
| | Absorption-promoting formulation (25) | 1110 | 298 | 17.5 |
| | Absorption-promoting formulation (26) | 1570 | 295 | 24.0 |
| | Absorption-promoting formulation (27) | 1830 | 438 | 29.6 |
| 3 mg/kg | Solution formulation (26) | 1340 | 251 | 6.54 |
| | Absorption-promoting formulation (28) | 1670 | 290 | 8.36 |
| | Absorption-promoting formulation (29) | 2710 | 610 | 13.8 |
| | Absorption-promoting formulation (30) | 12300 | 2570 | 63.8 |
| 0.2 mg/kg | iv formulation (13) | 1230 | - | - |

### (Evaluation Example 15) Monkey PK study (3 mg/kg)

Pharmacokinetic after oral administration of solution formulation (27) prepared in Comparative Example 27 and absorption-promoting formulations (31) and (32) prepared in Examples 31 and 32 and after intravenous administration of iv formulation (14) prepared in Production Example 14 in male cynomolgus monkeys were evaluated in the same manner as in Evaluation Example 2. From the resulting changes in plasma concentration, the pharmacokinetic parameters were calculated by the same analysis as in Evaluation Example 2. The results are shown in Table 21. As a result, it was confirmed that any AUC in the administration group of absorption-promoting formulations (31) and (32) of Examples 31 and 32 was higher than AUC in the administration group of solution formulation (27) of Comparative Example 27, and an increase in Cmax was also observed (Table 21). From this, it was confirmed that, with the use of lauroyl-L-carnitine hydrochloride, a high BA was exhibited compared to without lauroyl-L-carnitine hydrochloride.

**[Table 21]**

| Pharmacokinetic parameters of each formulation of compound CP05 (monkeys, 3 mg/kg) | | | | |
|---|---|---|---|---|
| Dose | Formulation | AUC (ng*h/mL) | Cmax (ng/mL) | BA (%) |
| 3 mg/kg | Solution formulation (27) | 4880 | 505 | 43.0 |
| | Absorption-promoting formulation (31) | 9250 | 765 | 80.3 |
| | Absorption-promoting formulation (32) | 9400 | 811 | 82.3 |
| 0.2 mg/kg | iv formulation (14) | 745 | - | - |

### [Example 33] Preparation of solid dispersion-containing formulation (1)

Compound CP01 and HPMCAS (manufactured by Shin-Etsu Chemical Co., Ltd.) were added to acetone such that the quantitative ratio of the compound CP01 and HPMCAS was 1 : 2 and the solid concentration was 12 wt/vol%, thereby preparing a suspension. The suspension was spray-dried to obtain a solid dispersion. This solid dispersion was mixed with sodium lauryl sulfate (manufactured by BASF) in a quantitative ratio of 9:5 to prepare a solid dispersion-containing formulation (1).

### (Evaluation Example 16) Monkey PK study (3 mg/kg)

Pharmacokinetic after oral administration of solution formulation (26) prepared in Comparative Example 26, followed by oral administration of capsules filled with solid dispersion-containing formulation (1) prepared in Example 33 in combination with capsules filled with lauroyl-L-carnitine hydrochloride (10 mg/kg), and after intravenous administration of iv formulation (13) prepared in Production Example 13 in male cynomolgus monkeys were evaluated in the same manner as in Evaluation Example 2. From the resulting changes in plasma concentration, the pharmacokinetic parameters were calculated by the same analysis as in Evaluation Example 2. The results are shown in Table 22. As a result, it was confirmed that the AUC in the combined administration group of capsules filled with solid dispersion-containing formulation (1) prepared in Example 33 and capsules filled with lauroyl-L-carnitine hydrochloride (10 mg/kg) was higher than AUC in the administration group of solution formulation (26) of Comparative Example 26, and an increase in Cmax was also observed (Table 22). From this, it was confirmed that a high BA was exhibited when mixed with sodium lauryl sulfate and further used in combination with lauroyl-L-carnitine hydrochloride.

**[Table 22]**

| Pharmacokinetic parameters of each formulation of compound CP01 (monkeys, 3 mg/kg) | | | | |
|---|---|---|---|---|
| Dose | Formulation | AUC (ng*h/mL) | Cmax (ng/mL) | BA (%) |
| 3 mg/kg | Solution formulation (26) | 1340 | 251 | 6.54 |
| | Solid dispersion-containing formulation (1) + Combined use of lauroyl-L-carnitine hydrochloride | 4120 | 575 | 21.6 |
| 0.2 mg/kg | iv formulation (13) | 1230 | - | - |

## Claims

1. A composition comprising a compound represented by general formula (1), a pharmaceutically acceptable salt thereof, or a pharmaceutically acceptable solvate thereof, and a surfactant,
wherein the surfactant is represented by any one of the following general formulae (a1) to (a3): 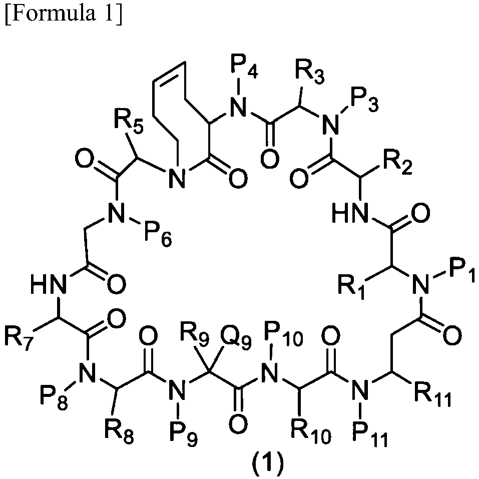 [wherein R₁ is C₁ to C₆ alkyl or C₃ to C₈ cycloalkyl C₁ to C₆ alkyl;
P₁ is C₁ to C₆ alkyl;
R₂ is C₁ to C₆ alkyl;
R₃ is hydrogen, or forms a 4- to 7-membered saturated heterocyclic ring together with a carbon atom bonded to P₃ and R₃ and a nitrogen atom bonded to P₃;
P₃ is C₁ to C₆ alkyl or C₃ to C₈ cycloalkyl except when R₃ and P₃ form a 4- to 7-membered saturated heterocyclic ring;
P₄ is C₁ to C₆ alkyl;
R₅ is benzyl optionally substituted by one or more groups selected from the group consisting of C₁ to C₆ alkyls, C₁ to C₆ haloalkyls, and C₃ to C₈ cycloalkyls;
P₆ is C₁ to C₆ alkyl;
R₇ is phenethyl optionally substituted by one or more groups selected from the group consisting of halogens, C₁ to C₆ haloalkyls, and C₁ to C₆ alkoxys;
R₈ forms a 4- to 7-membered saturated heterocyclic ring together with a carbon atom bonded to P₈ and R₈ and a nitrogen atom bonded to P₈, the 4- to 7-membered saturated heterocyclic ring being optionally substituted by a C₁ to C₆ alkoxy;
R₉ forms a 3- to 8-membered alicyclic ring together with Q₉ and a carbon atom bonded to R₉ and Q₉, the 3- to 8-membered alicyclic ring being optionally substituted by one or more C₁ to C₆ alkyls;
P₉ is hydrogen or C₁ to C₆ alkyl;
R₁₀ is C₁ to C₆ alkyl or C₃ to C₈ cycloalkyl;
P₁₀ is C₁ to C₆ alkyl;
R₁₁ is di-C₁ to C₆ alkylaminocarbonyl or 4- to 8-membered cyclic aminocarbonyl; and
P₁₁ is C₁ to C₆ alkyl], 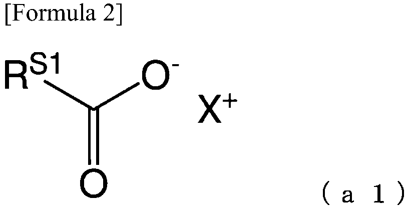 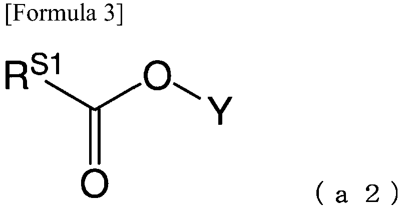 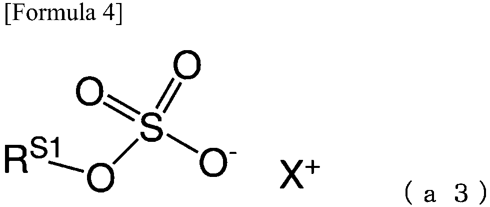
[wherein R^{S1} represents a saturated or unsaturated, linear hydrocarbon group having 5 or more and 13 or less carbon atoms which may have a substituent, X represents sodium or potassium, and Y represents a group represented by the following formula (a4) or a stereoisomer thereof.] 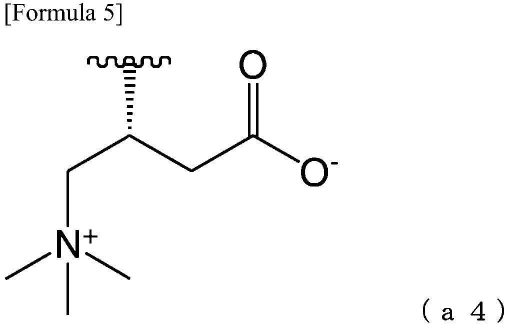 [wherein 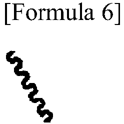 represents a bond].

2. A composition to be used in combination with a surfactant,
wherein the surfactant is represented by any one of the following general formulae (a1) to (a3),
the composition comprising a compound represented by general formula (1), a pharmaceutically acceptable salt thereof, or a pharmaceutically acceptable solvate thereof: 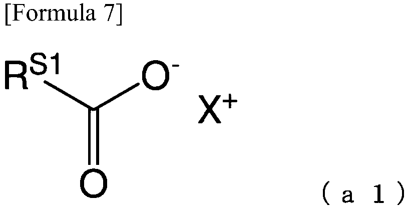 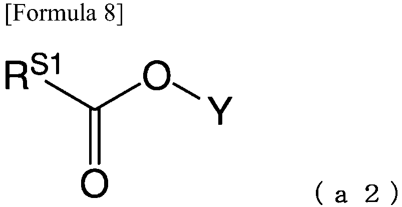 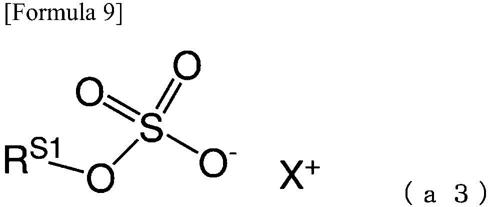 [wherein R^{S1} represents a saturated or unsaturated, linear hydrocarbon group having 5 or more and 13 or less carbon atoms which may have a substituent, X represents sodium or potassium, and Y represents a group represented by the following formula (a4) or a stereoisomer thereof.] 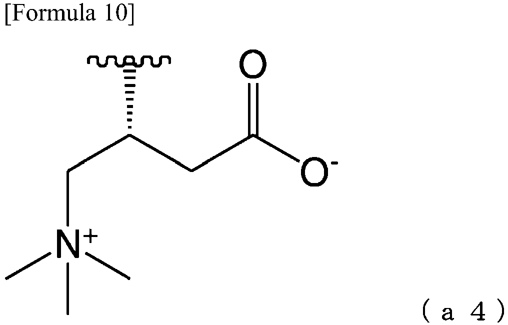 [wherein 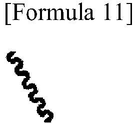 represents a bond], 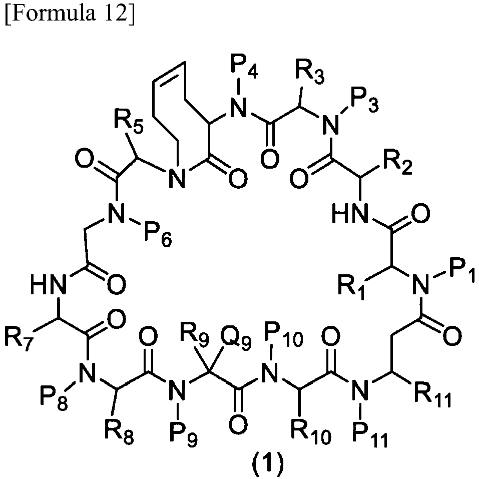 [wherein R₁ is C₁ to C₆ alkyl or C₃ to C₈ cycloalkyl C₁ to C₆ alkyl;
P₁ is C₁ to C₆ alkyl;
R₂ is C₁ to C₆ alkyl;
R₃ is hydrogen, or forms a 4- to 7-membered saturated heterocyclic ring together with a carbon atom bonded to P₃ and R₃ and a nitrogen atom bonded to P₃;
P₃ is C₁ to C₆ alkyl or C₃ to C₈ cycloalkyl except when R₃ and P₃ form a 4- to 7-membered saturated heterocyclic ring;
P₄ is C₁ to C₆ alkyl;
R₅ is benzyl optionally substituted by one or more groups selected from the group consisting of C₁ to C₆ alkyls, C₁ to C₆ haloalkyls, and C₃ to C₈ cycloalkyls;
P₆ is C₁ to C₆ alkyl;
R₇ is phenethyl optionally substituted by one or more groups selected from the group consisting of halogens, C₁ to C₆ haloalkyls, and C₁ to C₆ alkoxys;
R₈ forms a 4- to 7-membered saturated heterocyclic ring together with a carbon atom bonded to P₈ and R₈ and a nitrogen atom bonded to P₈, the 4- to 7-membered saturated heterocyclic ring being optionally substituted by a C₁ to C₆ alkoxy;
R₉ forms a 3- to 8-membered alicyclic ring together with Q₉ and a carbon atom bonded to R₉ and Q₉, the 3- to 8-membered alicyclic ring being optionally substituted by one or more C₁ to C₆ alkyls;
P₉ is hydrogen or C₁ to C₆ alkyl;
R₁₀ is C₁ to C₆ alkyl or C₃ to C₈ cycloalkyl;
P₁₀ is C₁ to C₆ alkyl;
R₁₁ is di-C₁ to C₆ alkylaminocarbonyl or 4- to 8-membered cyclic aminocarbonyl; and
P₁₁ is C₁ to C₆ alkyl].

3. The composition according to claim 1 or 2, wherein the surfactant is acylcarnitine.

4. The composition according to claim 1 or 2, wherein the surfactant is an alkyl carboxylate.

5. The composition according to claim 1 or 2, wherein the surfactant is an N-(8-[2-hydroxybenzoyl]amino)caprylate.

6. The composition according to any one of claims 1 to 5, wherein the compound represented by general formula (1) is a compound represented by formula (1a).

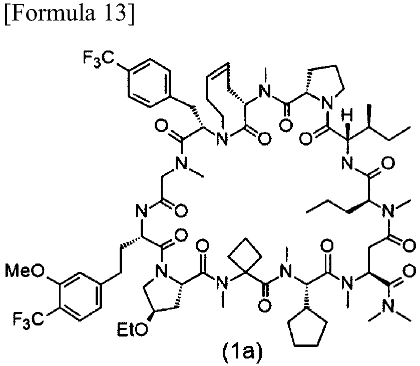

7. The composition according to any one of claims 1 to 5, wherein the compound represented by general formula (1) is a compound represented by formula (1b).

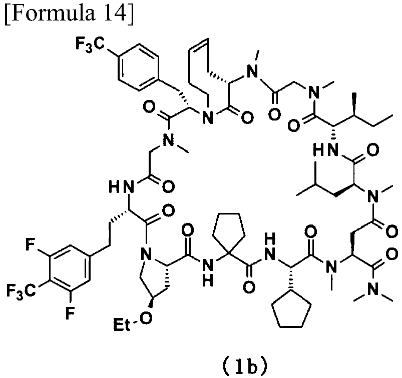

8. The composition according to any one of claims 1 to 5, wherein the compound represented by general formula (1) is a compound represented by formula (1c).

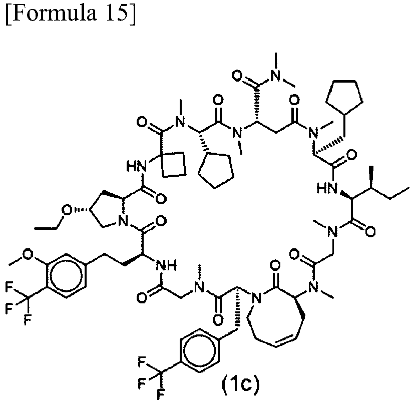

9. The composition according to any one of claims 1 to 5, wherein the compound represented by general formula (1) is a compound represented by formula (1d).

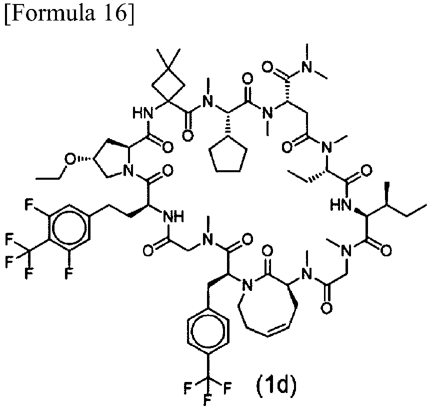

10. The composition according to any one of claims 1 to 5, wherein the compound represented by general formula (1) is a compound represented by formula (1e).

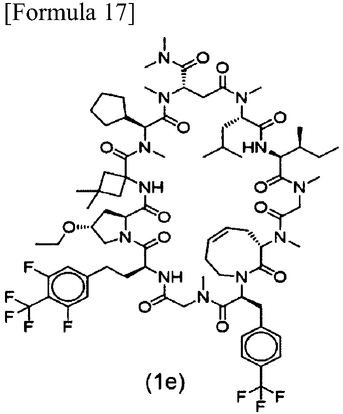

11. The composition according to any one of claims 1 to 5, wherein the compound represented by general formula (1) is a compound represented by formula (1f).

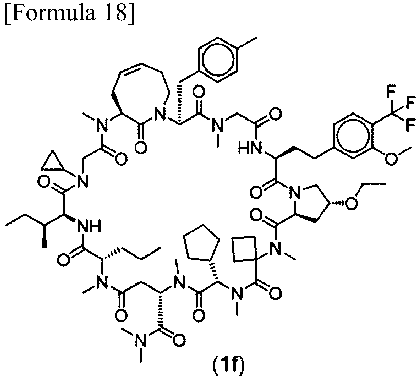

12. The composition according to any one of claims 1 to 11, further comprising a solubility improver.

13. The composition according to claim 12, wherein the solubility improver comprises a polymer that forms a solid dispersion with the compound.

14. The composition according to any one of claims 1 to 13, wherein a value of bioavailability (BA) of the compound as measured in a system in which the surfactant is present is 1.1-fold or more the value as measured in a system in which the surfactant is not present.

15. A method for improving the absorption of a compound represented by general formula (1),
the method comprising incorporating, in a same composition, a compound represented by general formula (1), a pharmaceutically acceptable salt thereof, or a pharmaceutically acceptable solvate thereof, and a surfactant selected from the group consisting of lauroyl-L-carnitine, sodium caprylate, sodium lauryl sulfate, and sodium N-(8-[2-hydroxybenzoyl]amino)caprylate: 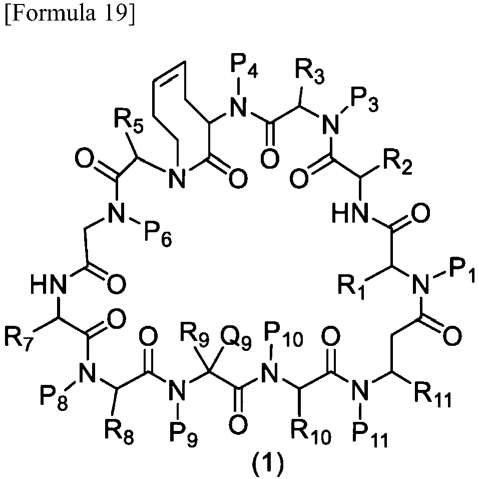 [wherein R₁ is C₁ to C₆ alkyl or C₃ to C₈ cycloalkyl C₁ to C₆ alkyl;
P₁ is C₁ to C₆ alkyl;
R₂ is C₁ to C₆ alkyl;
R₃ is hydrogen, or forms a 4- to 7-membered saturated heterocyclic ring together with a carbon atom bonded to P₃ and R₃ and a nitrogen atom bonded to P₃;
P₃ is C₁ to C₆ alkyl or C₃ to C₈ cycloalkyl except when R₃ and P₃ form a 4- to 7-membered saturated heterocyclic ring;
P₄ is C₁ to C₆ alkyl;
R₅ is benzyl optionally substituted by one or more groups selected from the group consisting of C₁ to C₆ alkyls, C₁ to C₆ haloalkyls, and C₃ to C₈ cycloalkyls;
P₆ is C₁ to C₆ alkyl;
R₇ is phenethyl optionally substituted by one or more groups selected from the group consisting of halogens, C₁ to C₆ haloalkyls, and C₁ to C₆ alkoxys;
R₈ forms a 4- to 7-membered saturated heterocyclic ring together with a carbon atom bonded to P₈ and R₈ and a nitrogen atom bonded to P₈, the 4- to 7-membered saturated heterocyclic ring being optionally substituted by a C₁ to C₆ alkoxy;
R₉ forms a 3- to 8-membered alicyclic ring together with Q₉ and a carbon atom bonded to R₉ and Q₉, the 3- to 8-membered alicyclic ring being optionally substituted by one or more C₁ to C₆ alkyls;
P₉ is hydrogen or C₁ to C₆ alkyl;
R₁₀ is C₁ to C₆ alkyl or C₃ to C₈ cycloalkyl;
P₁₀ is C₁ to C₆ alkyl;
R₁₁ is di-C₁ to C₆ alkylaminocarbonyl or 4- to 8-membered cyclic aminocarbonyl; and
P₁₁ is C₁ to C₆ alkyl].
